# EUROPEAN PATENT SPECIFICATION

(11) **EP 4 676 311 B1**
(45) Date of publication and mention of the grant of the patent: **21.10.2026**
(21) Application number: 24719011.9
(22) Date of filing: 22.03.2024
(51) Int. Cl.: A61B 5/00, A61B 5/02

(54) **SYSTEMS AND METHODS FOR DETERMINING FILTERED CARDIAC OUTPUT**
SYSTEME UND VERFAHREN ZUR BESTIMMUNG DER GEFILTERTEN HERZLEISTUNG
SYSTÈMES ET PROCÉDÉS POUR DÉTERMINER UN DÉBIT CARDIAQUE FILTRÉ

(30) Priority: 24.03.2023 US 202363492159 P; 24.03.2023 US 202363492173 P; 24.03.2023 US 202363492176 P
(43) Date of publication of application: 14.01.2026
(73) Proprietor: Becton, Dickinson and Company, Franklin Lakes, NJ 07417 (US)
(72) Inventor: POTES BLANDON, Cristhian, M., Irvine, California 92614 (US); GUPTA, Piyush, Irvine, California 92614 (US); MOSES, Kevin, James, Irvine, California 92614 (US)
(74) Representative: Venner, Julia Ann
(86) International application number: PCT/US2024/021217
(87) International publication number: WO 2024/206178

(56) References cited:
- US-A- 5 687 733
- US-A1- 2020 121 851
- US-B1- 7 925 347
- DANIEL RÜSCHEN ET AL: "Online cardiac output estimation during transvalvular left ventricular assistance", COMPUTER METHODS AND PROGRAMS IN BIOMEDICINE, vol. 171, 1 April 2019 (2019-04-01), NL, pages 87 - 97, XP055614478, ISSN: 0169-2607, DOI: 10.1016/j.cmpb.2016.08.020

## Description

### CROSS-REFERENCE TO RELATED APPLICATION(S)

This application claims the benefit of U.S. Provisional Application No. 63/492,173, filed March 24, 2023, and entitled "SYSTEMS AND METHODS FOR DETERMINING FILTERED CARDIAC OUTPUT." This application also claims the benefit of U.S. Provisional Application No. 63/492,176, filed March 24, 2023, and entitled "SYSTEMS AND METHODS FOR DETERMINING FLOW AND CARDIAC OUTPUT." This application also claims the benefit of U.S. Provisional Application No. 63/492,159, filed March 24, 2023, and entitled "SYSTEMS AND METHODS FOR VALIDATING HEMODYNAMIC DATA."

### BACKGROUND

The present disclosure relates generally to hemodynamic monitoring, and in particular, to determining blood flow and cardiac output in a patient using monitored hemodynamic data.

Monitoring hemodynamic variables of a patient allows for improved patient care. Cardiac output, the amount of blood pumped by the heart in one minute, is a key hemodynamic variable. Cardiac output is monitored by clinicians to make diagnoses and provide interventions in critically ill patients. Often, however, the accuracy of cardiac output monitoring diminishes at high cardiac output levels. Further, the delay in cardiac output measurements can affect the accuracy of the cardiac output information. Equipment necessary to monitor cardiac output is often expensive, difficult to acquire, or too invasive.

### SUMMARY

Aspects of the presently claimed invention are set out in the independent claims. Particular embodiments of these aspects are set out in the dependent claims. Any subject matter contained herein that does not fall within the scope of the appended claims is considered as being useful for understanding the invention.

In one example, a system for determining a hemodynamic condition of a patient includes a first hemodynamic sensor and a display. The first hemodynamic sensor produces, on an ongoing basis, a first hemodynamic sensor signal representative of a right ventricular pressure waveform of the patient. The system further includes one or more processors and computer-readable memory encoded with instructions that, when executed by the one or more processors, cause the system to receive the first hemodynamic sensor signal representative of the right ventricular pressure waveform of the patient. The instructions further cause the system to estimate a filtered cardiac output by filtering one or more cardiac output estimates using a Kalman filter algorithm and output the filtered cardiac output to the display. At least one of the one or more cardiac output estimates is determined using the right ventricular pressure waveform of the patient or features extracted from the right ventricular pressure waveform of the patient.

In another example, a system for determining a hemodynamic condition of a patient includes a first hemodynamic sensor and a display. The first hemodynamic sensor produces, on an ongoing basis, a first hemodynamic sensor signal representative of a right ventricular pressure waveform of the patient. The system further includes one or more processors and computer-readable memory encoded with instructions that, when executed by the one or more processors, cause the system to receive the first hemodynamic sensor signal representative of the right ventricular pressure waveform of the patient. The instructions further cause the system to estimate a filtered change in cardiac output by filtering one or more change in cardiac output estimates using a Kalman filter algorithm and output the filtered change in cardiac output to the display. At least one of the one or more change in cardiac output estimates is determined using the right ventricular pressure waveform of the patient or features extracted from the right ventricular pressure waveform of the patient.

In another example, a system for determining a hemodynamic condition of a patient includes a first hemodynamic sensor and a display. The first hemodynamic sensor produces, on an ongoing basis, a first hemodynamic sensor signal representative of a right ventricular pressure waveform of the patient. The system further includes one or more processors and computer-readable memory encoded with instructions that, when executed by the one or more processors, cause the system to receive the first hemodynamic sensor signal representative of the right ventricular pressure waveform of the patient. The instructions further cause the system to estimate a filtered cardiac output by filtering one or more cardiac output estimates using a first Kalman filter algorithm, estimate a filtered change in cardiac output by filtering one or more change in cardiac output estimates using a second Kalman filter algorithm, and output the filtered cardiac output and the filtered change in cardiac output to the display. At least one of the one or more cardiac output estimates is determined using the right ventricular pressure waveform of the patient or features extracted from the right ventricular pressure waveform of the patient. At least one of the one or more change in cardiac output estimates is determined using the right ventricular pressure waveform of the patient or the features extracted from the right ventricular pressure waveform of the patient.

In another example, a method of determining a hemodynamic condition of a patient includes receiving, by a hemodynamic monitoring system, sensed hemodynamic data representative of a right ventricular pressure waveform of the patient. The method further includes estimating, by the hemodynamic monitoring system, a filtered cardiac output by filtering one or more cardiac output estimates using a first Kalman filter algorithm; estimating, by the hemodynamic monitoring system, a filtered change in cardiac output by filtering one or more change in cardiac output estimates using a second Kalman filter algorithm; and outputting, by the hemodynamic monitoring system, the filtered cardiac output and the filtered change in cardiac output to a display. At least one of the one or more cardiac output estimates is determined using the right ventricular pressure waveform of the patient or features extracted from the right ventricular pressure waveform of the patient. At least one of the one or more change in cardiac output estimates is determined using the right ventricular pressure waveform of the patient or the features extracted from the right ventricular pressure waveform of the patient.

### BRIEF DESCRIPTION OF THE DRAWINGS

FIG. 1 is a schematic block diagram illustrating an example hemodynamic monitoring system that determines cardiac output and blood flow for a patient based on hemodynamic data.
FIG. 2 is a perspective view of an example hemodynamic monitor that analyzes a right ventricular pressure waveform and a pulmonary artery pressure waveform to provide cardiac output and blood flow for the patient.
FIG. 3 is a perspective view of an example catheter that can be inserted in the patient and connected to one or more hemodynamic sensors for providing hemodynamic data to the hemodynamic monitor.
FIG. 4 is a perspective view of an example minimally invasive pressure sensor that can be attached to the patient for sensing hemodynamic data representative of a right ventricular pressure or a pulmonary artery pressure of the patient.
FIG. 5 is a perspective view of an example oximetry module for receiving oximetry data from a catheter inserted within a patient.
FIG. 6 is a schematic block diagram illustrating inputs and outputs for modules of the hemodynamic monitoring system.
FIG. 7 is a graph illustrating an example right ventricular pressure waveform trace including example indicia indicative of blood flow and cardiac output.
FIG. 8 is a graph illustrating an example pulmonary artery pressure waveform trace including example indicia indicative of blood flow and cardiac output.
FIG. 9 is a schematic block diagram illustrating a validation module, as shown in FIG. 1.
FIG. 10 is a graph illustrating an example right ventricular pressure waveform trace including indicia indicative of catheter placement and data quality.
FIG. 11 is a graph illustrating an example right ventricular pressure waveform trace and an example pulmonary artery pressure waveform trace including example indicia indicative of catheter placement and data quality.
FIG. 12 is a schematic block diagram illustrating a flow module, as shown in FIG. 1.
FIG. 13 is a schematic block diagram illustrating a right ventricular pressure waveform transforming into a processed flow waveform via the flow module.
FIG. 14 is a schematic block diagram illustrating an autoencoder model of the flow module.
FIG. 15 is a schematic diagram illustrating filters of the autoencoder model.
FIG. 16 is a flow diagram illustrating an example process for training the autoencoder model to predict human blood flow based on the right ventricular pressure waveform.
FIG. 17 is a schematic block diagram illustrating a CO_{AE} module, as shown in FIG. 1.
FIG. 18 is a schematic block diagram illustrating a CO_{LR} module, as shown in FIG. 1.
FIG. 19 is a schematic block diagram illustrating reference and current features used as inputs to a regression model of the CO_{LR} module.
FIG. 20A is a schematic block diagram illustrating a CO_{filtered} module, as shown in FIG. 1, including a first example of a filter sub-module.
FIG. 20B is a schematic block diagram illustrating the CO_{filtered} module, as shown in FIG. 1, including a second example of a filter sub-module.
FIG. 21A is a graph illustrating an example predicted value of the CO_{filtered} module.
FIG. 21B is a graph illustrating an example measured value of the CO_{filtered} module over time.
FIG. 21C is a graph illustrating an example filtered value from the CO_{filtered} module over time.
FIG. 22 is a graph comparing example cardiac output values from the CO_{AE} module, the CO_{LR} module, and the CO_{filtered} module and example intermittent cardiac output values over time for a patient in a pre-bypass state, a post-bypass state, and an ICU state.
FIG. 23 is a schematic block diagram illustrating inputs and outputs for each module of an example implementation of the hemodynamic monitoring system of FIG. 1.

### DETAILED DESCRIPTION

As described herein, a hemodynamic monitoring system implements a series of modules that produce continuous blood flow and cardiac output measurements for a patient. The hemodynamic monitoring system utilizes a catheter and hemodynamic sensors to generate a right ventricular pressure ("RVP") waveform and, optionally, a pulmonary artery pressure ("PAP") waveform for input into modules of the hemodynamic monitoring system to produce the blood flow and cardiac output of the patient during operation in, e.g., an operating room (OR), an intensive care unit (ICU), or other patient care environment. Medical workers can use the blood flow and cardiac output information to improve patient care.

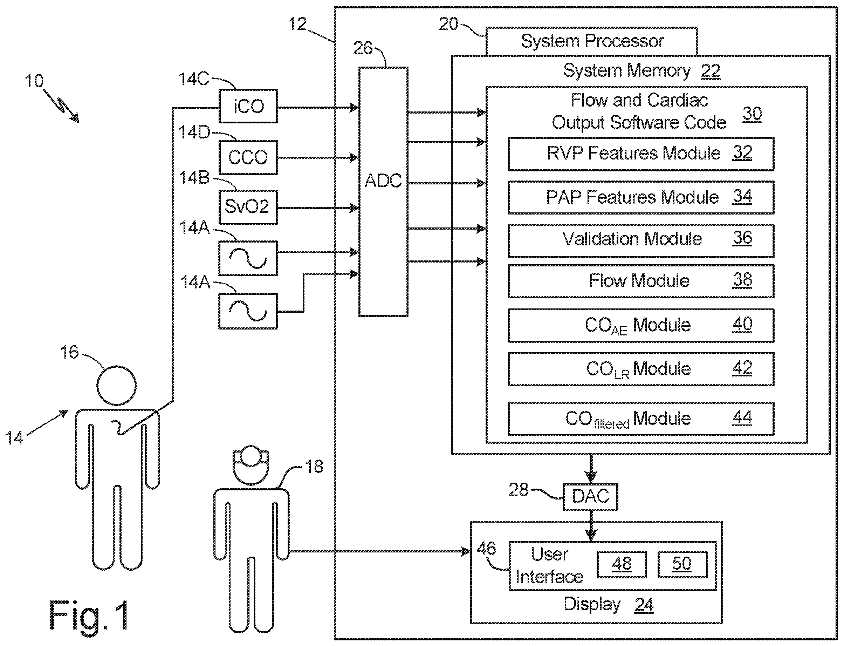

### Hemodynamic Monitoring System (FIGS. 1-8)

FIG. 1 is a schematic block diagram of hemodynamic monitoring system 10 that determines cardiac output and blood flow for a patient based on hemodynamic data. FIG. 1 shows hemodynamic monitoring system 10, including hemodynamic monitor 12 and hemodynamic sensors 14 (including hemodynamic sensors 14A, 14B, 14C, and 14D). Hemodynamic monitor 12 includes system processor 20, system memory 22, display 24, analog-to-digital converter (ADC) 26, and digital-to-analog converter (DAC) 28. System memory 22 includes flow and cardiac output software code 30, which includes RVP features module 32, PAP features module 34, validation module 36, flow module 38, CO_{AE} module 40, CO_{LR} module 42, and CO_{filtered} module 44. Display 24 includes user interface 46, which includes control elements 48 and sensory alarm 50. FIG. 1 also shows patient 16 and healthcare worker 18.

As illustrated in FIG. 1, hemodynamic monitoring system 10 includes hemodynamic monitor 12 and hemodynamic sensors 14 (including hemodynamic sensors 14A, 14B, 14C, and 14D). Hemodynamic monitoring system 10 can be implemented within a patient care environment, such as an ICU, an OR, or other patient care environment, for monitoring a hemodynamic condition of a patient. As illustrated in FIG. 1, the patient care environment can include patient 16 and healthcare worker 18 trained to utilize hemodynamic monitoring system 10.

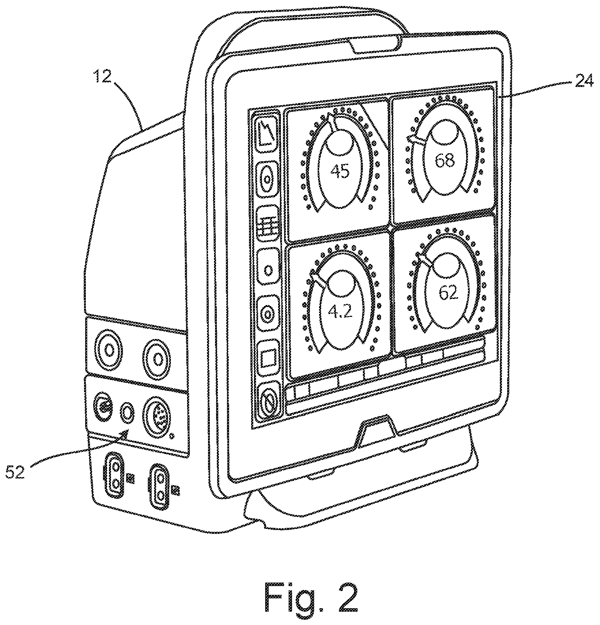

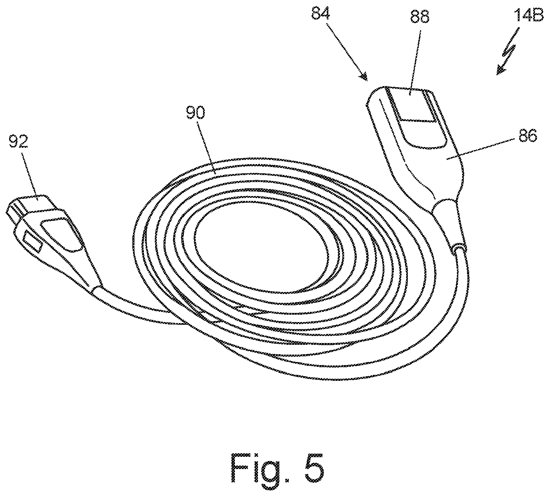

Hemodynamic monitor 12, as described below with respect to FIG. 2, can be an integrated hardware unit including system processor 20, system memory 22, display 24, ADC 26, and DAC 28. In other examples, any one or more components and/or described functionality of hemodynamic monitor 12 can be distributed among multiple hardware units. For instance, in some examples, display 24 can be a separate display device that is remote from and operatively coupled with hemodynamic monitor 12. Likewise, at least a portion of data processing within hemodynamic monitoring system 10 can occur via a smart cable that is connected between a catheter (e.g., catheter 54, as shown in FIG. 5) or sensor and hemodynamic monitor 12. In general, though illustrated and described in the example of FIG. 1 as an integrated hardware unit, it should be understood that hemodynamic monitor 12 can include any combination of devices and components that are electrically, communicatively, or otherwise operatively connected to perform functionality attributed herein to hemodynamic monitor 12.

As illustrated in FIG. 1, system memory 22 stores flow and cardiac output software code 30. Flow and cardiac output software code 30 includes RVP features module 32, PAP features module 34, validation module 36, flow module 38, CO_{AE} module 40, CO_{LR} module 42, and CO_{filtered} module 44. Display 24 provides user interface 46, which includes control elements 48 that enable user interaction with hemodynamic monitor 12 and/or other components of hemodynamic monitoring system 10. User interface 46, as illustrated in FIG. 1, also provides sensory alarm 50 to provide warning to medical personnel based on flow and/or cardiac output of patient 16, as is further described below.

Hemodynamic sensors 14 can be attached to patient 16 to sense hemodynamic data representative of an RVP waveform, a PAP waveform, blood oxygen saturation ("SvO2"), or cardiac output of patient 16 or any combination of these hemodynamic data. Hemodynamic sensors 14 are operatively connected to hemodynamic monitor 12 (e.g., electrically and/or communicatively connected via wired or wireless connection, or both) to provide the sensed hemodynamic data to hemodynamic monitor 12. In some examples, hemodynamic sensors 14 provide the hemodynamic data of patient 16 to hemodynamic monitor 12 as an analog signal, which is converted by ADC 26 to digital hemodynamic data representative of the RVP waveform and/or the PAP waveform. In other examples, hemodynamic sensors 14 can provide the sensed hemodynamic data to hemodynamic monitor 12 in digital form, in which case hemodynamic monitor 12 may not include or utilize ADC 26. In yet other examples, hemodynamic sensors 14 can provide the hemodynamic data of patient 16 to hemodynamic monitor 12 as an analog signal, which is analyzed in its analog form by hemodynamic monitor 12.

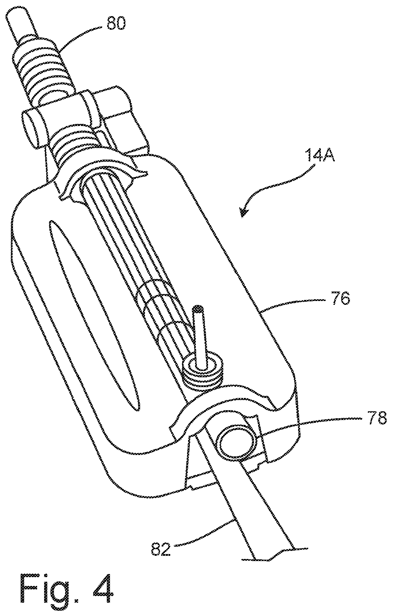

Hemodynamic sensors 14 can include one or more non-invasive, minimally invasive, or invasive sensors attached to patient 16. For instance, hemodynamic sensors 14 can take the form of invasive hemodynamic sensor 14A, such as second pressure transducer 14A that provides RVP waveform data sensed at right ventricle port 68C located within the right ventricle of the patient's heart (shown in FIG. 4). Hemodynamic sensors 14 can take the form of invasive hemodynamic sensor 14B, such as oximetry module 14B that provides blood oxygen saturation data within the pulmonary artery based on light pulses emitted from module 14B into the pulmonary artery and reflected, returned, and received by module 14B via optical connector 62 of catheter 54 (shown in FIG. 5). Further, hemodynamic sensors 14 can take the form of non-invasive hemodynamic sensors. In some examples, hemodynamic sensors 14 can be attached non-invasively at an extremity of patient 16, such as a forehead, a wrist, an arm, a finger, an ankle, a toe, or other extremity of patient 16. Hemodynamic sensors 14 can also take the form of other invasive, minimally invasive, or non-invasive hemodynamic sensors.

In certain examples, hemodynamic sensors 14 can be configured to sense RVP, PAP, or both right ventricular and pulmonary artery pressures of patient 16. In some instances, hemodynamic sensors 14 may also be used to sense cardiac output of the patient, blood oxygen saturation within the pulmonary artery, or both cardiac output and blood oxygen saturations in addition to right ventricular and pulmonary artery pressure waveforms. For instance, one or more hemodynamic sensor 14 can be attached to patient 16 via a radial arterial catheter inserted into an arm of patient 16. In other examples, hemodynamic sensor 14 can be attached to patient 16 via a femoral arterial catheter inserted into a leg of patient 16. Such techniques can similarly enable multiple hemodynamic sensors 14 to provide substantially continuous beat-to-beat monitoring of the RVP and PAP as well as monitoring of cardiac output and blood oxygen saturation of patient 16, or any combination of these hemodynamic data, over an extended period of time, such as minutes or hours.

System processor 20 executes flow and cardiac output software code 30, which implements RVP features module 32, PAP features module 34, validation module 36, flow module 38, CO_{AE} module 40, CO_{LR} module 42, and CO_{filtered} module 44, which utilize the RVP waveform and, optionally, the PAP waveform to produce blood flow and/or cardiac output measurements. Examples of system processor 20 can include any one or more of a microprocessor, a controller, a digital signal processor (DSP), an application specific integrated circuit (ASIC), a field-programmable gate array (FPGA), or other equivalent discrete or integrated logic circuitry.

System memory 22 can be configured to store information within hemodynamic monitor 12 during operation. System memory 22, in some examples, is described as computer-readable storage media. In some examples, a computer-readable storage medium can include a non-transitory medium. The term "non-transitory" can indicate that the storage medium is not embodied in a carrier wave or a propagated signal. In certain examples, a non-transitory storage medium can store data that can, over time, change (e.g., in RAM or cache). System memory 22 can include volatile and non-volatile computer-readable memories. Examples of volatile memories can include random access memories (RAM), dynamic random-access memories (DRAM), static random-access memories (SRAM), and other forms of volatile memories. Examples of non-volatile memories can include, e.g., magnetic hard discs, optical discs, flash memories, or forms of electrically programmable memories (EPROM) or electrically erasable and programmable (EEPROM) memories.

Display 24 can be a liquid crystal display (LCD), a light-emitting diode (LED) display, an organic light-emitting diode (OLED) display, or other display device suitable for providing information to users in graphical form. User interface 46 can include graphical and/or physical control elements that enable user input to interact with hemodynamic monitor 12 and/or other components of hemodynamic monitoring system 10. In some examples, user interface 46 can take the form of a graphical user interface (GUI) that presents graphical control elements presented at, e.g., a touch-sensitive and/or presence sensitive display screen of display 24. In such examples, user input can be received in the form of gesture input, such as touch gestures, scroll gestures, zoom gestures, or other gesture input. In certain examples, user interface 46 can take the form of and/or include physical control elements, such as a physical buttons, keys, knobs, or other physical control elements configured to receive user input to interact with components of hemodynamic monitoring system 10.

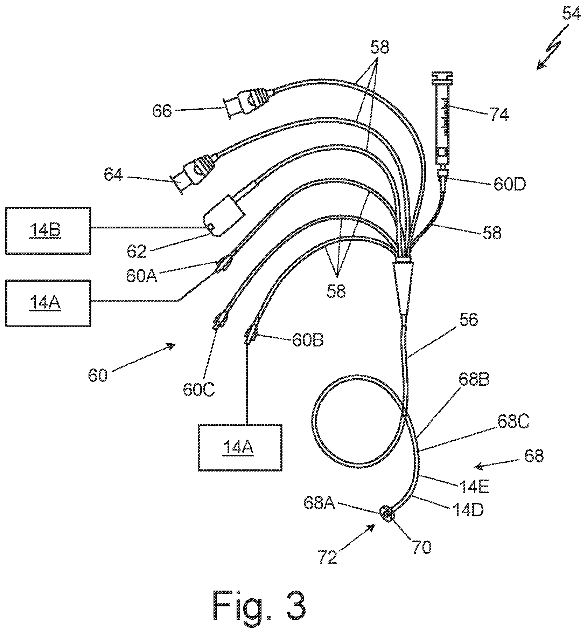

An example of hemodynamic monitor 12 is shown in FIG. 2. An example of a catheter is shown in FIG. 3. An example of a minimally invasive pressure sensor is shown in FIG. 4. An example of an oximetry module is shown in FIG. 5.

FIG. 2 is a perspective view of hemodynamic monitor 12 that analyzes the RVP waveform and the PAP waveform to provide cardiac output and blood flow for patient 16. As illustrated in FIG. 2, hemodynamic monitor 12 includes display 24 that, in the example of FIG. 1, presents a graphical user interface 46 including control elements 48 (e.g., graphical control elements) that enable user interaction with hemodynamic monitor 12. Hemodynamic monitor 12 can also include a plurality of input and/or output (I/O) connectors 52 configured for wired connection (e.g., electrical and/or communicative connection) with one or more peripheral components, such as one or more hemodynamic sensors 14. While the example of FIG. 2 illustrates five separate I/O connectors 52, it should be understood that in other examples, hemodynamic monitor 12 can include fewer than five I/O connectors 52 or greater than five I/O connectors 52. In yet other examples, hemodynamic monitor 12 may not include I/O connectors 52, but rather may communicate wirelessly with various peripheral devices.

As described with respect to FIG. 1, hemodynamic monitor 12 includes one or more system processors 20 and computer-readable system memory 22 that stores flow and cardiac output software code 30, which is executable to produce continuous blood flow and cardiac output measurements. Hemodynamic monitor 12 can receive sensed hemodynamic data representative of an RVP waveform and a PAP waveform, such as via one or more hemodynamic sensors 14 connected to hemodynamic monitor 12 via I/O connectors 52. Hemodynamic monitor 12 executes the flow and cardiac output software code 30 to obtain, using the received hemodynamic data and multiple profiling parameters (e.g., input features), blood flow and cardiac output measurements, as is further described below.

As illustrated in FIG. 1, hemodynamic monitor 12 can present a graphical user interface at display 24. Display 24 can be an LCD, an LED display, an OLED display, or other display device suitable for providing information to users in graphical form. In some examples, such as the example of FIG. 2, display 24 can be a touch-sensitive and/or presence-sensitive display device configured to receive user input in the form of gestures, such as touch gestures, scroll gestures, zoom gestures, swipe gestures, or other gesture input.

Hemodynamic monitor 12 receives hemodynamic data from patient 16 via one or more hemodynamic sensors 14A, 14B, 14C, and 14D (collectively hemodynamic sensors 14). In response to receiving hemodynamic data of patient 16, hemodynamic monitor 12 executes flow and cardiac output software code 30 to determine the blood flow and/or cardiac output and display the blood flow and/or cardiac output on display 24. Additionally, hemodynamic monitor 12 can invoke a sensory alarm, such as an audible alarm, a haptic alarm, or other sensory alarm in response to the blood flow and/or cardiac output measurements (for example, sensory alarm 50 as shown in FIG. 1). Accordingly, hemodynamic monitor 12 can provide a warning to alert medical personnel of concerning blood flow or cardiac output measurements.

FIG. 3 is a perspective view of catheter 54 that can be inserted into patient 16 and connected to one or more hemodynamic sensors 14 for providing hemodynamic data to hemodynamic monitor 12. For example, catheter 54 may be connected to one or more pressure-sensing hemodynamic sensors 14A for detecting RVP, PAP, or both right ventricular and pulmonary artery pressures of patient 16. Additionally, catheter 54 may interface with oximetry module 14B for sensing mixed venous oxygen saturation of the patient. Protected by sheath 56, catheter 54 includes multiple lumens 58 that place fluid connectors 60, optical connector 62, thermistor connector 64, and thermal filament connector 66 in communication with one of ports 68, an embedded hemodynamic sensor 14C (e.g., a thermistor), or an embedded hemodynamic sensor 14D (e.g., a thermal filament). To facilitate insertion of catheter 54 within patient 16, or for certain hemodynamic measurements, catheter 54 includes balloon 70 located at tip 72 of catheter 54.

As shown in FIG. 3, catheter 54 includes distal port connector 60A communicating with port 68A at tip 72. Proximal injectate connector 60B communicates with proximal port 68B disposed approximately 30 cm from tip 72 and can be used for dispensing fluids and drugs into the patient's heart. Right ventricular pacing connector 60C communicates with right ventricle port 68C, which may be spaced approximately 19 cm from tip 72 or approximately 12 to 13 cm from tip 72. Connector 60C can be used for sensing RVP. Thermistor connector 64 electrically connects to hemodynamic sensor 14C (e.g., the thermistor) installed near tip 72 of catheter 54 for measuring core blood temperature within the pulmonary artery. In some examples of catheter 54, thermal filament connector 66 electrically connects to hemodynamic sensor 14D (e.g., the thermal filament) embedded within catheter 54 located within the patient's right ventricle. In some examples, catheter 54 does not include a thermal filament or corresponding thermal filament connector 66. Balloon connector 60D communicates with balloon 70 and with the use of syringe 74 can be used to inflate and deflate balloon 70.

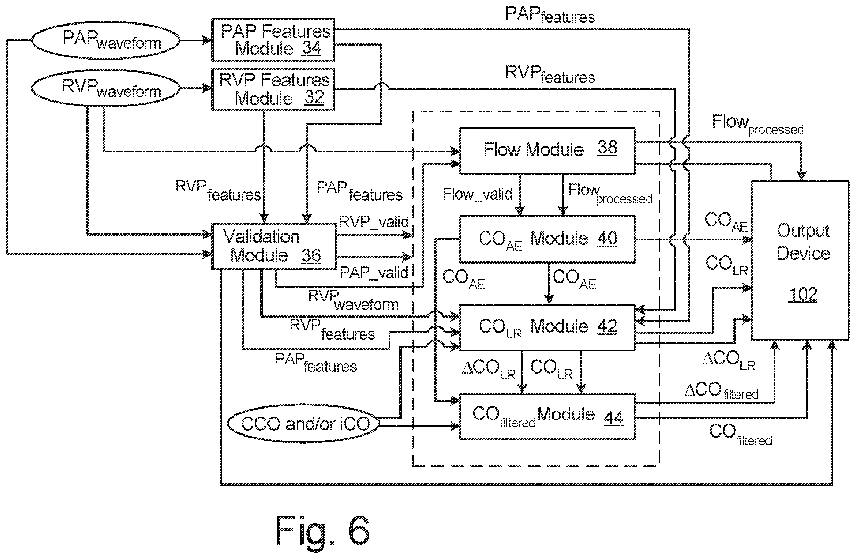

After insertion into patient 16, e.g., via an introducer, distal port connector 60A and right ventricular pacing connector 60C can be connected to separate pressure transducer sensors 14A. A first pressure transducer 14A provides PAP waveform data sensed at distal port 68A located within the pulmonary artery of the patient's heart to hemodynamic monitor 12, while a second pressure transducer 14A provides RVP waveform data sensed at right ventricle port 68C located within the right ventricle of the patient's heart to hemodynamic monitor 12. Blood oxygen saturation data within the pulmonary artery can be provided by oximetry module 14B based on light pulses emitted from oximetry module 14B into the pulmonary artery and reflected light returns received by oximetry module 14B via optical connector 62 of catheter 54. Additionally, utilizing thermal filament connector 66 and thermistor connector 64 and associated cabling, hemodynamic monitor 12 can receive cardiac output data of patient 16 using, for example, a thermal dilution technique. The cardiac output measured via the thermal filament and corresponding thermal filament connector 66 can be considered a continuous cardiac output ("CCO"). If catheter 54 does not include a thermal filament, cardiac output can be determined using a thermistor after injecting a fluid bolus (or a set of boluses) of known volume and temperature via proximal injectate port 68B using the thermal dilution technique. The cardiac output measured via the thermistor and corresponding thermistor connector 64 after injection of the fluid bolus can be considered an intermittent cardiac output ("iCO"). Intermittent cardiac output iCO measurements can be obtained at a frequency that is on the order of, e.g., minutes, hours, several hours, or even longer intervals, depending on the level of monitoring a patient requires. For example, a clinician may administer a bolus set of 3-4 fluid boluses, where one fluid bolus of the set is administered approximately every minute such that the complete bolus set lasts around three minutes. In one example, fluid boluses can be administered very frequently, such as every minute or every few minutes, when a clinician is assessing a patient's responsiveness to medication or another medical intervention. In another example, fluid boluses can be administered less frequently, such as every hour, every six hours, etc., if a patient is relatively stable in the ICU. Accordingly, catheter 54 can be used to provide CCO and/or iCO, as described below with reference to FIGS. 6, 16, and 18-20A.

Catheter 54 is one example of a catheter that can be used to measure RVP, PAP, CCO, and/or iCO. In other examples, any catheter configured to measure RVP, PAP, CCO, and/or iCO can be used.

FIG. 4 is a perspective view of hemodynamic sensor 14A that can be attached to patient 16 for sensing hemodynamic data representative of RVP or PAP of patient 16. As illustrated in FIG. 4, hemodynamic sensor 14A includes housing 76, fluid input port 78, catheter-side fluid port 80, and I/O cable 82. Fluid input port 78 is configured to be connected via tubing or other hydraulic connection to a fluid source, such as a saline bag or other fluid input source. Catheter-side fluid port 80 is configured to be connected via tubing or other hydraulic connection to a catheter (e.g., a radial arterial catheter or a femoral arterial catheter) that is inserted into an arm of the patient (i.e., a radial arterial catheter) or a leg of the patient (i.e., a femoral arterial catheter). I/O cable 82 is configured to connect to hemodynamic monitor 12 via, e.g., one or more of I/O connectors 52 (shown in FIG. 2). Housing 76 of hemodynamic sensor 14A encloses one or more pressure transducers, communication circuitry, processing circuity, and corresponding electronic components to sense fluid pressure corresponding to the RVP or PAP of patient 16 that is transmitted to hemodynamic monitor 12 (shown in FIG. 2) via I/O cable 82.

In operation, a column of fluid (e.g., saline solution) is introduced from a fluid source (e.g., a saline bag) through hemodynamic sensor 14A via fluid input port 78 to catheter-side fluid port 80 toward the catheter inserted into patient 16. RVP or PAP is communicated through the fluid column to pressure sensors located within housing 76 which sense the pressure of the fluid column. Hemodynamic sensor 14A translates the sensed pressure of the fluid column to an electrical signal via the pressure transducers and outputs the corresponding electrical signal to hemodynamic monitor 12 (shown in FIG. 1) via I/O cable 82. Hemodynamic sensor 14 therefore transmits analog sensor data (or a digital representation of the analog sensor data) to hemodynamic monitor 12 (shown in FIG. 1) that is representative of substantially continuous beat-to-beat monitoring of the RVP or PAP of patient 16.

FIG. 5 is a perspective view of oximetry module 14B for receiving oximetry data from a catheter inserted within patient 16. As depicted in FIG. 5, hemodynamic sensor 14B includes an optical transmitter and an optical receiver arranged to communicate to a catheter via I/O connector 84 installed within housing 86 and accessible via protective door 88. Within housing 86, hemodynamic sensor 14B, as depicted by FIG. 5, includes communication circuitry, processing circuity, and corresponding electronic components to sense blood oxygen saturation data derived from optical light emissions transmitted via a catheter into a patient and corresponding light returns received from patient 16 via the catheter. An electrical signal indicative of the patient blood oxygen saturation levels is transmitted to hemodynamic monitor 12 via cable 90 and connector 92, which interfaces with one of I/O connectors 52 (shown in FIG. 2).

FIG. 6 is a schematic block diagram illustrating inputs and outputs for each module of hemodynamic monitoring system 10. Although hemodynamic monitoring system 10 has been described with respect to catheter 54, any suitable catheter may be used with hemodynamic monitoring system 10. FIG. 6 shows RVP features module 32, PAP features module 34, validation module 36, flow module 38, CO_{AE} module 40 (a module that estimates cardiac output based on an autoencoder model), CO_{LR} module 42 (a module that estimates cardiac output using a linear regression model), CO_{filtered} module 44 (a module that filters one or more estimates of cardiac output), and output device 102. Each of RVP features module 32, PAP features module 34, validation module 36, flow module 38, CO_{AE} module 40, CO_{LR} module 42, and CO_{filtered} module 44 is a functional module of flow and cardiac output software code 30, as shown in FIG. 1. Although flow and cardiac output software code 30 is described herein as being divided into seven modules, in other examples, the functionality of flow and cardiac output software code 30 could also be described as more or fewer modules, which could depend, in some examples, on how the code is written or organized. Additionally, any modules and/or sub-modules could also be entirely separate collections of code. The modules of flow and cardiac output software code 30 will be described sequentially; however, these modules can also include overlapping or interspersed functionality.

RVP features module 32 is a first module of flow and cardiac output software code 30 in hemodynamic monitoring system 10. RVP features module 32 includes methods in code for extracting RVP features ("RVP_{features}") from an RVP waveform ("RVP_{waveform}"). RVP features module 32 receives RVP_{waveform} as an input. RVP_{waveform} corresponds to hemodynamic data sensed by one of hemodynamic sensors 14A and received by hemodynamic monitor 12. The sensed hemodynamic data to which RVP_{waveform} corresponds is passed to RVP features module 32. RVP features module 32 extracts RVP_{features} from RVP_{waveform}. RVP features module 32 outputs RVP_{features} to validation module 36. RVP features module 32 also outputs RVP_{features} to CO_{LR} module 42.

PAP features module 34 is a second module of flow and cardiac output software code 30 in hemodynamic monitoring system 10. PAP features module 34 includes methods in code for extracting PAP features ("PAP_{features}") from a PAP waveform ("PAP_{waveform}"). PAP features module 34 receives PAP_{waveform} as an input. PAP_{waveform} corresponds to hemodynamic data sensed by one of hemodynamic sensors 14A and received by hemodynamic monitor 12. The hemodynamic data to which PAP_{waveform} corresponds is passed to PAP features module 34. PAP features module 34 extracts PAP_{features} from PAP_{waveform}. PAP features module 34 outputs PAP_{features} to validation module 36. PAP features module 34 also outputs PAP_{features} to CO_{LR} module 42.

Validation module 36 is a third module of flow and cardiac output software code 30 in hemodynamic monitoring system 10. Validation module 36 includes methods in code for cleaning data and ensuring RVP_{waveform} and PAP_{waveform} are valid and reliable. Validation module 36 receives RVP_{waveform}, PAPw_{aveform}, RVP_{features}, and PAP_{features} as inputs. The sensed hemodynamic data to which RVP_{waveform} and PAP_{waveform} correspond is passed to validation module 36. RVP_{features} are fed to validation module 36 from RVP features module 32. Likewise, PAP_{features} are fed to validation module 36 from PAP features module 34. Validation module 36 outputs instructions indicating whether valid RVP ("RVP_valid") and valid PAP ("PAP_valid") are true or false (i.e., whether RVP_{waveform} and PAP_{waveform} are valid or not valid) to the modules contained in the dashed line box shown in FIG. 6 (i.e., flow module 38, CO_{AE} module 40, CO_{LR} module 42, and CO_{filtered} module 44) and can output the determination indicating whether RVP_{waveform} and PAP_{waveform} are valid to output device 102, such as display 24 (shown in FIG. 1). An instruction indicating that RVP_valid and PAP_valid are true (e.g., "RVP_valid=true" and "PAP_valid=true") means that hemodynamic data corresponding to RVP_{waveform} and PAP_{waveform} is valid for further processing by the modules contained in the dashed line box. An instruction indicating that RVP_valid and PAP_valid are false (e.g., "RVP_valid=false" and "PAP_valid=false") means that hemodynamic data corresponding to RVP_{waveform} and PAP_{waveform} is not valid for further processing. Validation module 36 also outputs a smoothed RVP_{waveform} to flow module 38 and outputs RVP_{features} and PAP_{features} to COLR module 42.

Flow module 38 is a fourth module of flow and cardiac output software code 30 in hemodynamic monitoring system 10. Flow module 38 includes methods in code for estimating a blood flow waveform from RVP_{waveform}. Flow module 38 receives RVP_{waveform} as an input. The sensed hemodynamic data to which RVP_{waveform} corresponds is passed to flow module 38. Flow module 38 can also receive the smoothed RVP_{waveform} from validation module 36. Flow module 38 outputs instructions indicating whether valid flow ("flow_valid") is true or false (i.e., whether a processed blood flow ("flow_{processed}") is valid or not valid) to CO_{AE} module 40 and can output the determination indicating whether flow_{processed} is valid to output device 102, such as display 24 (shown in FIG. 1). An instruction that flow_valid is true (e.g., "flow_valid=true") means that the estimated blood flow waveform, flow_{processed}, is valid for further processing by CO_{AE} module 40. An instruction that flow_valid is false (e.g., "flow_valid=false") means that the estimated blood flow waveform, flow_{processed}, is not valid for further processing. Flow module 38 further outputs flow_{processed} to CO_{AE} module 40. Flow module 38 can also output flow_{processed} to output device 102.

CO_{AE} module 40 is a fifth module of flow and cardiac output software code 30 in hemodynamic monitoring system 10. CO_{AE} module 40 includes methods in code for deriving cardiac output from the estimated blood flow waveform. CO_{AE} module 40 receives flow_{processed} as an input. Flow_{processed} is fed to CO_{AE} module 40 from flow module 38. CO_{AE} module 40 also receives an instruction from flow module 38 indicating whether flow_valid is true or false such that CO_{AE} module 40 can proceed or not proceed accordingly. CO_{AE} module 40 outputs an autoencoder cardiac output ("CO_{AE}") to CO_{LR} module 42 and CO_{filtered} module 44. CO_{AE} module 40 can also output CO_{AE} to output device 102.

CO_{LR} module 42 is a sixth module of flow and cardiac output software code 30 in hemodynamic monitoring system 10. CO_{LR} module 42 includes methods in code for estimating change in cardiac output and cardiac output based on RVP_{features} and PAP_{features}. COLR module 42 receives RVP_{features}, PAP_{features}, COAE, CCO, and iCO as inputs. RVP_{features} are fed to CO_{LR} module 42 from RVP features module 32 or validation module 36. Likewise, PAP_{features} are fed to CO_{LR} module 42 from PAP features module 34 or validation module 36. CO_{AE} is fed to CO_{LR} module 42 from CO_{AE} module 40. CCO and iCO correspond to cardiac output data of patient 16 received by hemodynamic monitor 12 via catheter 54 using a thermal filament and/or thermistor and a thermal dilution technique, as described above with reference to FIG. 3. CCO and iCO are passed to CO_{LR} module 42. CO_{LR} module 42 outputs a linear regression cardiac output ("CO_{LR}") and a linear regression change in cardiac output ("ΔCO_{LR}") to CO_{filtered} module 44. CO_{LR} module 42 can also output CO_{LR} and ΔCO_{LR} to output device 102.

CO_{filtered} module 44 is a seventh module of flow and cardiac output software code 30 in hemodynamic monitoring system 10. CO_{filtered} module 44 includes methods in code for filtering cardiac output or change in cardiac output estimates via a Kalman filter algorithm. CO_{filtered} module 44 receives CO_{AE}, CO_{LR}, CCO, iCO, and ΔCO_{LR} as inputs. CO_{AE} is fed to CO_{filtered} module 44 from CO_{AE} module 40. CO_{LR} and ΔCO_{LR} are fed to CO_{filtered} module 44 from CO_{LR} module 42. **In** the same manner as described above for CO_{LR} module 42, CCO and iCO are also passed to CO_{filtered} module 44. CO_{filtered} module 44 outputs a filtered cardiac output ("CO_{filtered}") and a filtered change in cardiac output ("ΔCO_{filtered}") to output device 102.

Output device 102 is a device for receiving outputs from the modules of flow and cardiac output software code 30. Output device 102 can include display 24, as shown in FIG. 1. For example, output device 102 can receive final estimates of flow, cardiac output, and/or change in cardiac output for display via display 24. Output device 102 can receive outputs from flow module 38, CO_{AE} module 40, CO_{LR} module 42, and CO_{filtered} module 44. More specifically, output device 102 receives flow_{processed}, CO_{AE}, CO_{LR}, ΔCO_{LR}, CO_{filtered}, and ΔCO_{filtered}. Each of these outputs can be displayed via display 24 as corresponding graphs representing the values over time.

Although several modules are illustrated in FIG. 6 as having multiple inputs and outputs, some of the inputs and outputs are optional, and many configurations of hemodynamic monitoring system 10 (as shown in FIG. 1) are possible. **In** some examples, PAP_{features}, PAP_{waveform}, CCO, and/or iCO may not be used. **In** some examples, an estimate of cardiac output for patient 16 (as shown in FIG. 1) can be output from any one or more of CO_{AE} module 40, CO_{LR} module 42, and CO_{filtered} module 44. These configurations can depend, for example, on the combination of hemodynamic sensors 14 (as shown in FIG. 1) used or the desired output.

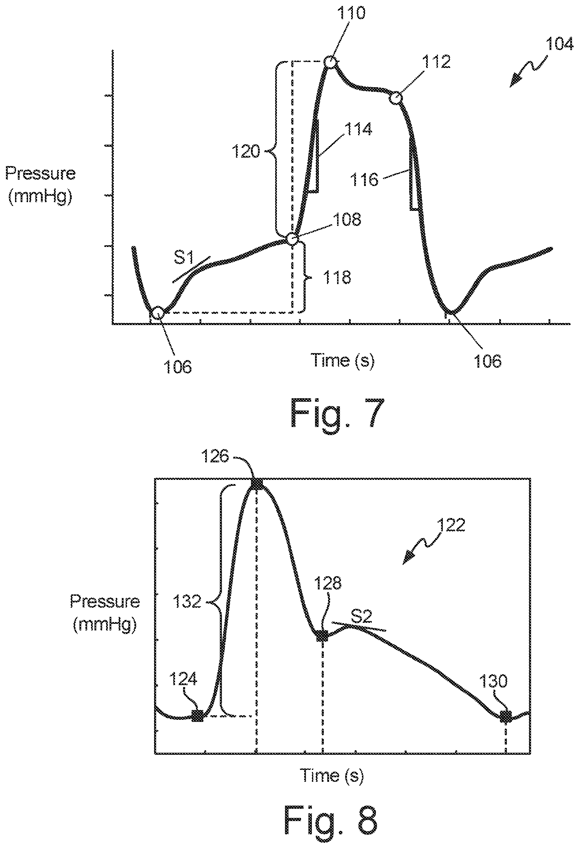

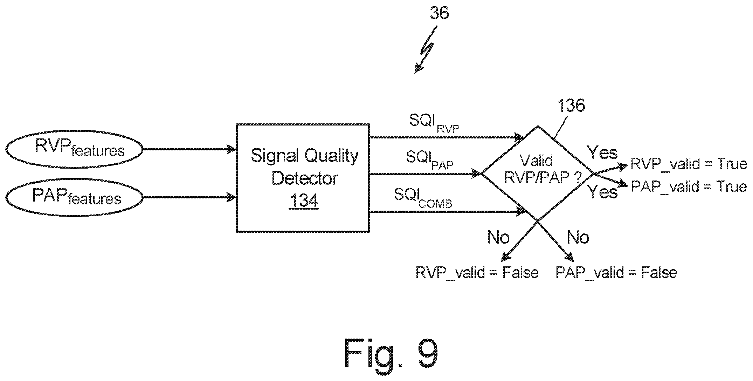

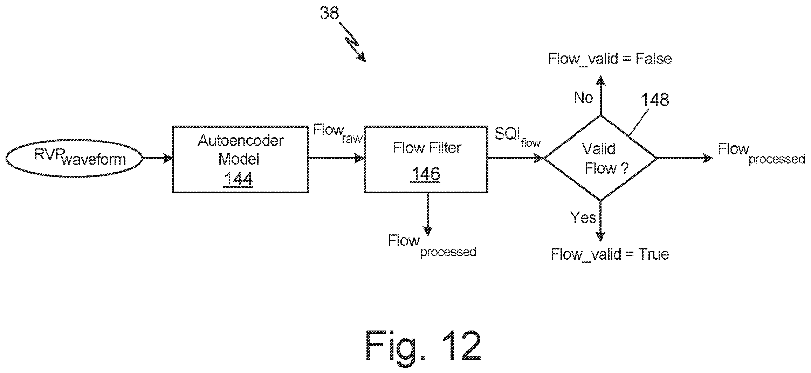

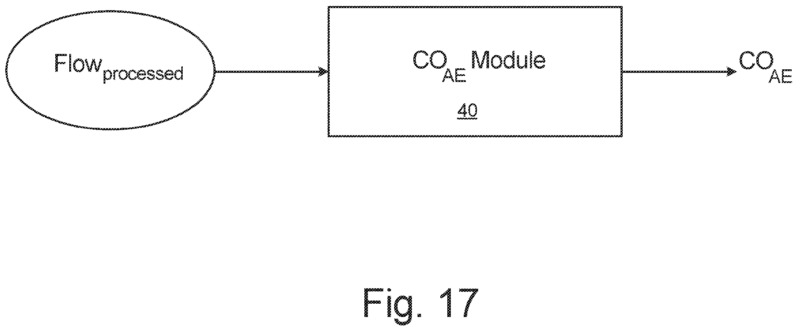

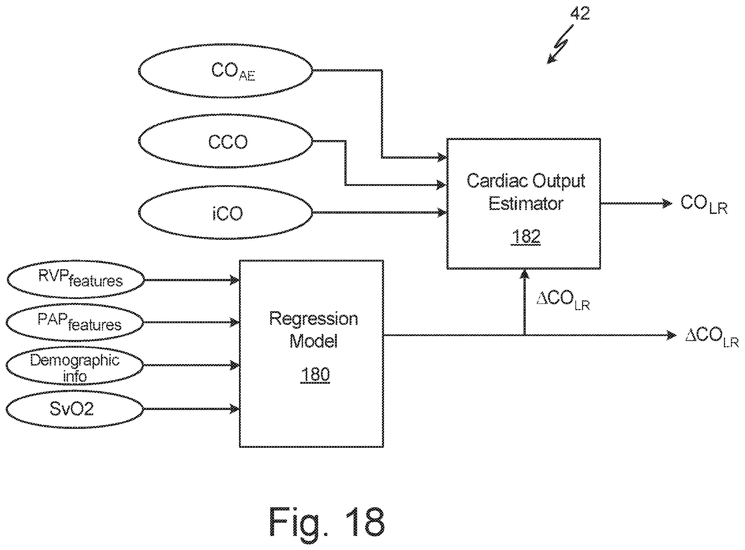

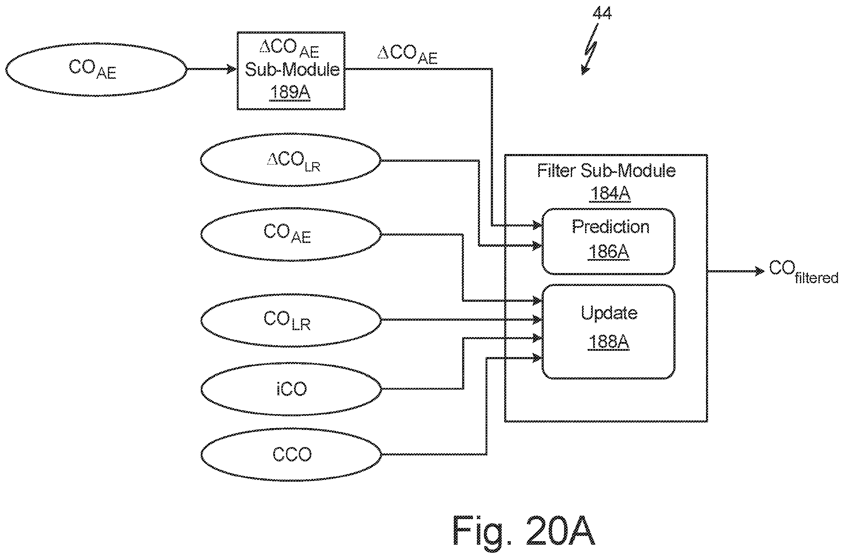

Each module of hemodynamic monitoring system 10 will be described in greater detail in turn below. RVP features module 32 will be described with reference to FIG. 7. PAP features module 34 will be described with reference to FIG. 8. Validation module 36 will be described with reference to FIGS. 9-11. Flow module 38 will be described with reference to FIGS. 12-16. CO_{AE} module 40 will be described with reference to FIG. 17. CO_{LR} module 42 will be described with reference to FIGS. 18-19. CO_{filtered} module 44 will be described with reference to FIGS. 20A-22.

FIG. 7 is a graph illustrating RVP waveform trace 104 including indicia 106, 108, 110, 112, 114, 116, 118, and 120. **In** FIG. 7, RVP waveform trace 104 is an example waveform of RVP_{waveform}. RVP_{waveform} corresponds to hemodynamic data sensed by one of hemodynamic sensors 14A and received by hemodynamic monitor 12. RVP waveform trace 104 (represented via digital hemodynamic data) can include various indicia indicative of blood flow and cardiac output for patient 16. RVP_{features} are extracted from RVP_{waveform} via RVP features module 32, as discussed above with respect to FIG. 1. Prior to extracting indicia from RVP_{waveform}, beat detector algorithms identify the start and end of individual heartbeats for each waveform. RVP beat detection algorithms identify the start of the heartbeat based on the maximum RVP, the minimum RVP, the maximum or minimum rate of change in RVP, and/or the second derivative with respect to time in the RVP. After heartbeat identification within RVP_{waveform}, various indicia of blood flow and cardiac output can be extracted from the waveform on an on-going, beat-to-beat basis.

Indicium 106 of RVP waveform trace 104 corresponds to the minimum diastolic pressure. Indicium 108 of RVP waveform trace 104 corresponds to the end diastolic pressure. Indicium 110 of RVP waveform trace 104 corresponds to the maximum systolic pressure. Indicium 112 of RVP waveform trace 104 corresponds to the end systolic pressure. Slope S1 is the slope of RVP waveform trace 104, which may also provide indicia. Slope S1 is depicted at one location but is representative of multiple slopes that may be determined at multiple locations along RVP waveform trace 104. For instance, indicium 114, corresponding to the maximum pressure rate of change with respect to time (dP/dt) during systolic rise, and indicium 116, corresponding to the minimum pressure rate of change with respect to time (dP/dt) during the relaxation period after end systole, are further example indicia. Similarly, the second time derivative of RVP waveform trace 104 may be determined at any location along RVP waveform trace 104 and used as indicia. Other exemplary indicia include RVP gradients, or the difference in pressure at different times during diastolic or systolic phase. For example, indicium 118 corresponds to the diastolic gradient, or the difference between minimum diastolic pressure (indicium 106) and end diastolic pressure (indicium 108). Indicium 120 corresponds to the right ventricular pulse pressure, or a pressure gradient equal to the difference between end diastolic pressure (indicium 108) and maximum systolic pressure (indicium 110).

Additional indicia can be extracted from RVP waveform trace 104 by flow and cardiac output software code 30 based on RVP waveform trace 104 during various intervals, for example, using RVP features module 32. For instance, systolic rise (indicia 108-110), systolic decay (indicia 110-112), isovolumetric relaxation (indicia 112-106), diastolic phase (indicia 106-108), and the heartbeat interval (between indicia 106) can be determined by flow and cardiac output software code 30. Such indicia may include the mean RVP during one of the above-referenced intervals.

FIG. 8 is a graph illustrating PAP waveform trace 122 including indicia 124, 126, 128, 130, and 132 indicative of blood flow and cardiac output. In FIG. 8, PAP waveform trace 122 is an example waveform of PAP_{waveform}. PAP_{waveform} corresponds to hemodynamic data sensed by one of hemodynamic sensors 14A and received by hemodynamic monitor 12. PAP waveform trace 122 (represented via digital hemodynamic data) can include various indicia indicative of blood flow and cardiac output for patient 16. PAP_{features} are extracted from PAP_{waveform} via PAP features module 34, as discussed above with respect to FIG. 1. Prior to extracting indicia from PAP_{waveform}, beat detector algorithms identify the start and end of individual heartbeats for each waveform. PAP beat detection algorithms identify the start of the heartbeat based on the maximum PAP, the minimum PAP, the maximum or minimum rate of change in PAP, and/or the second derivative with respect to time in the PAP. After heartbeat identification within PAP_{waveform}, various indicia of blood flow and cardiac output can be extracted from the waveform on an on-going, beat-to-beat basis.

Indicium 124 of PAP waveform trace 122 corresponds to the start of a heartbeat. Indicium 126 of PAP waveform trace 122 corresponds to the maximum systolic pressure marking the end of systolic rise. Indicium 128 of PAP waveform trace 122 corresponds to the presence and pressure of the dicrotic notch marking the end of systolic decay. Indicium 130 of PAP waveform trace 122 corresponds to the minimum diastolic pressure of the heartbeat of patient 16. The mean PAP can also be an indicium. PAP gradients, or pressure differences between points of the PAP waveform trace 122, can be indicia. For instance, indicium 132 corresponds to pulmonary pulse pressure, or the difference between minimum diastolic pressure (indicium 130) and maximum systolic pressure (indicium 126). S2 is the slope of PAP waveform trace 122, which may also provide indicia. Slope S2 is depicted at one location but is representative of multiple slopes that may be determined at multiple locations along PAP waveform trace 122. For instance, indicia may include the maximum and/or minimum time derivative of PAP waveform trace 122.

Additional indicia can be extracted from PAP waveform trace 122 by flow and cardiac output software code 30 based on PAP waveform trace 122 during various intervals, for example, using PAP features module 34. For instance, the interval from the maximum systolic pressure at indicium 126 to the diastole at indicium 128 and the interval from the start of the heartbeat at indicium 124 to the diastole at indicium 130 can be extracted from PAP waveform trace 122. Flow and cardiac output software code 30 may identify additional indicia from PAP waveform trace 122 during various intervals. For instance, systolic rise (indicia 124-126), systolic decay (indicia 126-128), systolic phase (indicia 124-128), diastolic phase (indicia 128-130), and the heartbeat interval (between indicia 124) can be determined by flow and cardiac output software code 30. Such indicia may include the mean PAP during one of the above-referenced intervals. The area under the curve of PAP waveform trace 122 and the standard deviations of PAP waveform trace 122 determined for the above-referenced intervals can also serve as additional indicia for patient 16.

### Validation Module (FIGS. 9-11)

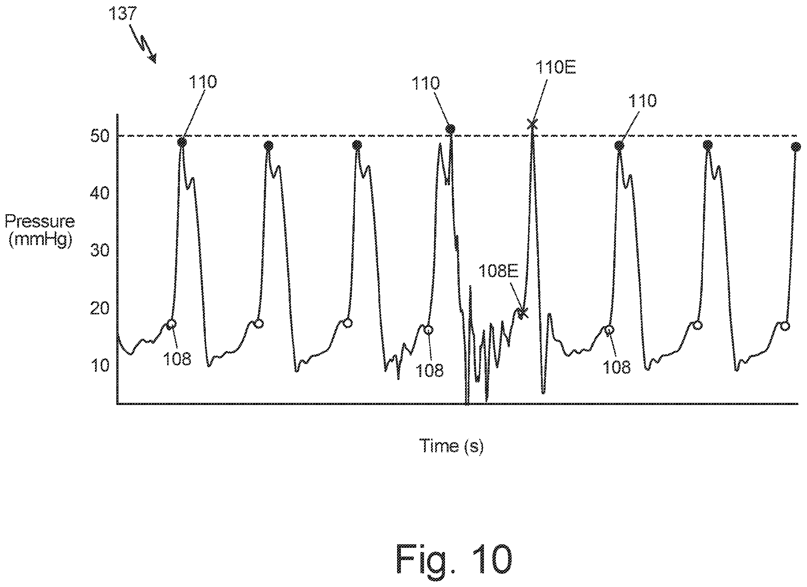

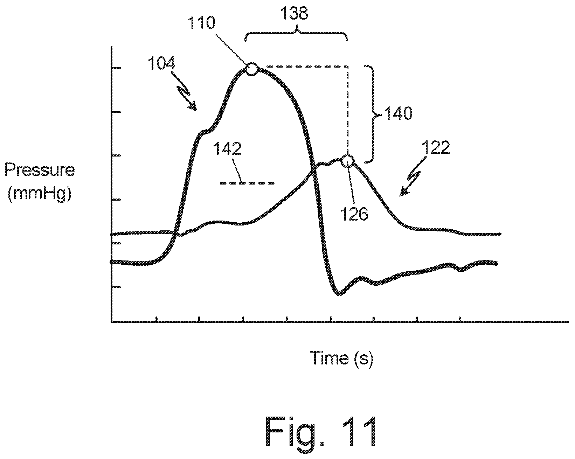

FIG. 9 is a schematic block diagram illustrating validation module 36, which includes signal quality detector 134 and decision block 136. FIG. 10 is a graph illustrating RVP waveform trace 137 including indicia 108 and 110, which are indicative of the placement of and data quality from catheter 54 (shown in FIG. 3). FIG. 11 is a graph illustrating RVP waveform trace 104 and PAP waveform trace 122 including indicia 110, 126, 138, 140, and 142, which are indicative of the placement of and data quality from catheter 54. FIGS. 9, 10, and 11 will be discussed together. Validation module 36 uses data from RVP_{waveform} and PAP_{waveform} to detect and continuously monitor catheter 54 placement issues and signal-quality issues, which affect data quality.

Validation module 36 filters, or cleans, data and ensures RVP_{waveform}, PAP_{waveform}, RVP_{features}, and PAP_{features} are valid and reliable. RVP_{features} of RVP_{waveform} and PAP_{features} of PAP_{waveform} that are derived from RVP features module 32 and PAP features module 34, respectively, are inputs for validation module 36. RVP_{waveform} and PAP_{waveform} are also input into validation module 36. Validation module 36 analyzes RVP_{waveform} and PAP_{waveform} in ten-second segments. The segments of RVP_{waveform} and PAP_{waveform} may be about ten seconds (e.g., 9.5 seconds to 10.5 seconds) or any other suitable time segment. Validation module 36 can be implemented within a patient care environment, such as an ICU, an OR, or another patient care environment. Validation module 36 refines data collected from catheter 54 and determines whether catheter 54 is placed correctly to yield data of high quality via signal quality detector 134 and decision block 136. As such, validation module 36 determines whether data from catheter 54 is usable for further analysis, such as for flow module 38 and CO_{LR} module 42.

RVP_{features} and PAP_{features} are input into signal quality detector 134. Signal quality detector 134 cleans data generated from RVP_{features} and PAP_{features} and assigns RVP signal quality index ("SQI_{RVP}") and PAP signal quality index ("SQI_{PAP}") to each ten-second segment of each of RVP_{waveform} and PAP_{waveform}, respectively. Signal quality detector 134 checks for the standard deviation of RVP_{features} and PAP_{features}, artifacts in RVP_{waveform} and PAP_{waveform}, and the standard deviation of features that represent differences between RVP_{waveform} and PAP_{waveform}.

Signal quality detector 134 consumes RVP_{features} and PAP_{features}, utilizing a signal quality algorithm to analyze data by comparing RVP_{features} and PAP_{features} against designated value ranges for RVP_{features} and PAP_{features}, respectively. When both RVP_{waveform} and PAP_{waveform} are present, features can be compared between waveforms, and features that represent differences between waveforms can be analyzed. The designated value ranges correspond to physiological limits plus system tolerance, or the standard deviation for each of RVP_{features}, PAP_{features}, and features representing differences between RVP_{waveform} and PAP_{waveform}. Signal quality detector 134 also checks data quality by comparing RVP_{features} to PAP_{features}. As such, the designated value ranges can be used to identify artifacts in RVP_{waveform} **and** PAP_{waveform}, such as excluded beats or noise, and signal quality issues, such as an underdamp or overdamp signal. Data outside of the designated value ranges, such as a negative pressure, an inordinately high pressure, or maximum systolic pressure 126 of PAP waveform trace 122 that is higher than maximum systolic pressure 110 of RVP waveform trace 104, represents data that is not possible physiologically.

Values outside of the designated value ranges represent erroneous data. Signal quality detector 134 cleans data, smoothing out RVP_{waveform} and PAP_{waveform} by removing erroneous data. Signal quality detector 134 assigns SQI_{RVP} and SQI_{PAP} to each ten-second segment of RVP_{waveform} and PAP_{waveform} based on the amount of detected erroneous data. Signal quality detector 134 outputs SQI_{RVP} and a SQI_{PAP} to decision block 136. Erroneous data (identified by features being outside the designated value ranges) in each ten-second segment of analyzed RVP_{waveform} and PAP_{waveform} is removed from the ten-second segment on a beat level, or any other suitable level. If RVP_valid is true (e.g., "RVP_valid=true") and/or PAP_valid is true (e.g., "PAP_valid=true"), the portion of the remaining ten-second segment of RVP_{waveform} and/or segment of PAP_{waveform} and the associated RVP_{features} and/or PAP_{features}, respectively, are passed on for further analysis.

FIG. 10 illustrates an example RVP waveform trace 137 that may be analyzed by signal quality detector 134. Signal quality detector 134 analyzes RVP_{features} of RVP waveform trace 137. RVP waveform trace 137 is another example waveform of RVP_{waveform}, similar to RVP waveform trace 104 shown in FIG. 7. In this example, signal quality detector 134 compares end diastolic pressures 108 and maximum systolic pressures 110 of each beat of RVP waveform trace 137 against the designated value ranges for RVP end diastolic pressure 108 and RVP maximum systolic pressure 110, respectively. Data from each beat, from RVP end diastolic pressure 108 to the subsequent RVP end diastolic pressure 108 or from RVP maximum systolic pressure 110 to the subsequent RVP maximum systolic pressure 110, is analyzed against corresponding designated value ranges. RVP waveform trace 137 includes end diastolic pressure exclusion 108E and maximum systolic pressure exclusion 110E.

FIG. 10 shows a beat of RVP waveform trace 137 extending from RVP end diastolic pressure 108 to the subsequent RVP end diastolic pressure 108 or extending from RVP maximum systolic pressure 110 to the maximum systolic pressure 110. Signal quality detector 134 detects and flags, via the signal quality algorithm, beats including data outside of the designated value ranges and removes those beats. As such, end diastolic pressure exclusion 108E and maximum systolic pressure exclusion 110E are removed, or excluded from further analysis, as they are within beats from the segment shown in RVP waveform trace 137 that contain erroneous data and are removed from the segment of RVP_{waveform}. As seen in FIG. 10, the beat from maximum systolic pressure 110 to the subsequent maximum systolic pressure exclusion 110E includes pressure values outside of the designated value ranges as RVP waveform trace 137 includes an artifact, noise, preceding end diastolic pressure exclusion 108E. As a result, signal quality detector 134 flags and excludes the beat (maximum systolic pressure 110 to the maximum systolic pressure exclusion 110E in RVP waveform trace 137), and all data from the beat is discarded from RVP_{waveform}. End diastolic pressure exclusion 108E, which is within said beat, is an end diastolic pressure measurement that is excluded from further analysis. As further seen in FIG. 10, the beat from end diastolic pressure exclusion 108E to the subsequent end diastolic pressure 108 in RVP waveform trace 137 includes a sharp drop in pressure following maximum systolic pressure exclusion 110E that is outside of the designated value range The sharp drop in pressure in RVP waveform trace 137 after maximum systolic pressure exclusion 110E is outside of the designated value range as it is physiologically unexpected for the pressure to decrease that quickly following maximum systolic pressure 110. As a result, signal quality detector 134 flags and excludes the beat, and all data from the beat (end diastolic pressure exclusion 108E to the subsequent end diastolic pressure 108 in RVP waveform trace 137) is discarded from RVP_{waveform}. Maximum systolic pressure exclusion 110E, which is within said beat, is a maximum systolic pressure measurement that is excluded from further analysis. The beats are excluded from the segment of RVP_{waveform} corresponding to RVP waveform trace 137 prior to forwarding the segment of RVP_{waveform}, including associated RVP_{features}, to subsequent modules, thereby cleaning RVP_{waveform}. Signal quality detector 134 uses the excluded beats from maximum systolic pressure 110 to the maximum systolic pressure exclusion 110E and from end diastolic pressure exclusion 108E to the subsequent end diastolic pressure 108, which contain the noise and the sharp drop in pressure, in determining SQI_{RVP}. Signal quality detector 134 can analyze PAP_{waveform} in a similar way using PAP_{features} to clean and analyze PAP_{waveform}.

Designated value ranges may also correspond to differences between RVP_{features} and PAP_{features} when both RVP_{waveform} and PAP_{waveform} are available. Further, various features can be derived from differences between RVP_{waveform} and PAP_{waveform}, the examined features depending on whether RVP_{waveform} and PAP_{waveform} are synchronous or asynchronous. Values outside of the designated value ranges represent erroneous data.

As seen in FIG. 11, signal quality detector 134 can analyze features representing differences between RVP_{features} of RVP waveform trace 104 and PAP_{features} of PAP waveform trace 122. RVP waveform trace 104 is synchronized with PAP waveform trace 122. RVP waveform trace 104 is an example waveform of RVP_{waveform}, and PAP waveform trace 122 is an example waveform of PAP_{waveform}. In this example, signal quality detector 134 analyzes pulse transit time 138, systolic gradient 140, and mean pressure 142.

Indicium 110 is determined from RVP waveform trace 104. Indicium 110 is the maximum systolic pressure, or the end of systolic rise, of RVP waveform trace 104. Indicium 126 is determined from PAP waveform trace 122. Indicium 126 is the maximum systolic pressure, or the end of systolic rise, of PAP waveform trace 122. Indicia 138, 140, and 142 are determined from differences between RVP waveform trace 104 and PAP waveform trace 122. Indicium 138 is the pulse transit time, or the time elapsed between the maximum systolic pressure (indicium 110) of RVP waveform trace 104 and the maximum systolic pressure (indicium 126) of PAP waveform trace 122. Pulse transit time 138 represents the delay between RVP_{waveform} and PAP_{waveform}, or the time difference between when beats are detected in both RVP_{waveform} and PAP_{waveform}. Indicium 140 is the systolic gradient, or the pressure gradient between maximum systolic pressure (indicium 110) of RVP waveform trace 104 and maximum systolic pressure (indicium 126) of PAP waveform trace 122. Indicium 142 is the mean pressure of PAP waveform trace 122 and RVP waveform trace 104.

Signal quality detector 134 compares pulse transit time 138, systolic gradient 140, and mean pressure 142 against the designated value ranges for pulse transit time 138, systolic gradient 140, and mean pressure 142, respectively. Signal quality detector 134 can also check for other differences between features of synchronous RVP waveform trace 104 and PAP waveform trace 122, such as a difference between maximum systolic pressure 110 of RVP waveform trace 104 and maximum systolic pressure 126 of PAP waveform trace 122 or a difference between the heart rate computed from RVP_{features} and the heart rate computed from PAP_{features}. For example, if RVP_{waveform} indicates the presence of five heartbeats and a synchronous PAP_{waveform} indicates the presence of ten heartbeats in the same time window, signal quality detector 134 would detect that the data from that segment of RVP_{waveform} and/or PAP_{waveform} is not valid. If RVP waveform trace 104 and PAP waveform trace 122 are asynchronous, signal quality detector 134 can analyze other additional RVP_{features} and PAP_{features}, such as the change in pulse transit time between RVP waveform trace 104 and PAP waveform trace 122.

Signal quality detector 134 detects and flags, via the signal quality algorithm, any errors, or values outside of the designated value ranges, for each indicium. Signal quality detector 134 excludes beats from RVP_{waveform} and PAP_{waveform} corresponding to beats from RVP waveform trace 104 and PAP waveform trace 122 that include flagged data containing errors prior to forwarding RVP_{waveform}, PAP_{waveform}, RVP_{features}, and PAP_{features} to subsequent modules, thereby cleaning RVP_{waveform} and PAP_{waveform}.

Signal quality detector 134 uses any errors in indicia identified based on differences between RVP_{features} and PAP_{features}, such as pulse transit time 138, systolic gradient 140, and mean pressure 142, in determining SQI_{RVP} and SQI_{PAP}. Indicium 138, 140, and 142, among other parameters determined or derived from RVP waveform trace 104 and PAP waveform trace 122, provide additional data by which signal quality detector 134 can analyze RVP_{waveform} and PAP_{waveform}. As such, analyzing both PAP_{waveform} and RVP_{waveform} enables a more comprehensive evaluation of data quality, resulting in a more accurate analysis.

As discussed above, signal quality detector 134 uses such information in addition to RVP_{features} and PAP_{features} from ten-second intervals of RVP_{waveform} and PAP_{waveform}, respectively, to determine SQI_{RVP}, SQI_{PAP}, and a combined RVP and PAP signal quality algorithm ("SQI_{COMB}") via the signal quality algorithm. Signal quality detector 134 assigns SQIRVP, SQI_{PAP}, and SQI_{COMB} based on RVP_{features}, PAP_{features}, and features representing differences between RVP_{waveform} and PAP_{waveform}, respectively.

SQI_{RVP} and SQI_{PAP} are values that indicate the quality of the respective segments of waveforms, or the extent of deviation in RVP_{features} and PAP_{features} computed from ten-second segments of RVP_{waveform} and PAP_{waveform}, respectively. For example, signal quality detector 134 may assign a signal quality index of zero to five (0 to 5), with zero (0) being the highest quality and five (5) being the lowest quality. SQI_{RVP} and SQI_{PAP} may be any suitable numeric range.

Signal quality detector 134 compares RVP_{features} of RVP_{waveform} and PAP_{features} of PAP_{waveform} against designated value ranges for RVP_{features} and designated value ranges for PAP_{features}, respectively, as discussed above. Signal quality detector 134 assigns SQI_{RVP} and SQI_{PAP} to each of RVP_{waveform} and PAP_{waveform} based on how close various RVP_{features} and PAP_{features}, respectively, are to the designed value ranges. RVP_{waveform} and PAP_{waveform} may be assigned SQI_{RVP} and SQI_{PAP}, respectively, having values of two to five (2 to 5) if RVP_{features} and PAP_{features}, respectively, are not within the designated value ranges. RVP_{waveform} and PAP_{waveform} may be assigned SQI_{RVP} and SQI_{PAP}, respectively, having values of zero to one (0 to 1) if RVP_{features} and PAP_{features}, respectively, are within the designated value ranges. Signal quality detector 134 outputs SQI_{RVP} and SQI_{PAP} to decision block 136.

SQI_{RVP} and SQI_{PAP} are fed into decision block 136 of validation module 36, which determines whether the ten-second segments of RVP_{waveform} and PAP_{waveform} are valid. Decision block 136 may output the determinations to output device 102 (shown in FIG. 6), which can be, for example, display 24 (shown in FIG. 1). Decision block 136 is a binary system, assigning a zero (0) to RVP_{waveform} and PAP_{waveform} with RVP_{features} and PAP_{features}, respectively, that are not within designated value ranges and assigning a one (1) to RVP_{waveform} and PAP_{waveform} with RVP_{features} and PAP_{features}, respectively, that are within designated value ranges. For example, when RVP_{features} and PAP_{features} have a signal quality index of two to five (2 to 5), decision block 136 assigns a zero (0) to RVP_{waveform} and PAP_{waveform}, respectively. When RVP_{features} and PAP_{features} have a signal quality index of zero or one (0 or 1), decision block 136 assigns a one (1) to RVP_{waveform} and PAP_{waveform}, respectively.

If SQI_{RVP} is assigned a zero (0), decision block 136 determines that RVP_valid is false (e.g., "RVP_valid=false"). This is indicated by the arrow labeled "No" in FIG. 9. The determination that RVP_valid is false can be sent from decision block 136 to a display, which may show an "INVALID RVP WAVEFORM" reading. If SQI_{RVP} is assigned a one (1), decision block 136 determines that the RVP_valid is true (e.g., "RVP_valid=true"). This is indicated by the arrow labeled "Yes" in FIG. 9. The determination that RVP_valid is true can be sent from decision block 136 to a display, which may show a "VALID RVP WAVEFORM" reading. Validation module 36 can also output instructions that indicate whether RVP_valid is true or false to the modules contained in the dashed line box shown in FIG. 6 (i.e., flow module 38, CO_{AE} module 40, CO_{LR} module 42, and CO_{filtered} module 44). Subsequent modules can proceed using data from the filtered ten-second segment of RVP_{waveform} when RVP_valid is true and cannot proceed using data from RVP_{waveform} when RVP_valid is false.

If SQI_{PAP} is assigned a zero (0), decision block 136 determines that PAP_valid is false (e.g., "PAP_valid=false"). This is indicated by the arrow labeled "No" in FIG. 9. The determination that PAP_valid is false can be sent from decision block 136 to a display, which may show an "INVALID PAP WAVEFORM" reading. If PAP signal quality index SQI_{PAP} is assigned a one (1), decision block 136 determines that the PAP_valid is true (e.g., "PAP_valid=true"). This is indicated by the arrow labeled "Yes" in FIG. 9. The determination that PAP_valid is true can be sent from decision block 136 to a display, which may show a "VALID PAP WAVEFORM" reading. Validation module 36 can also output instructions that indicate whether PAP_valid is true or false to the modules contained in the dashed line box shown in FIG. 6 (i.e., flow module 38, CO_{AE} module 40, CO_{LR} module 42, and CO_{filtered} module 44). Subsequent modules can proceed using data from the filtered ten-second segment of PAP_{waveform} when PAP_valid is true and cannot proceed using data from PAP_{waveform} when PAP_valid is false.

SQI_{COMB} is generated the same way using the features representing differences between RVP_{waveform} and PAP_{waveform}. SQI_{COMB} can be used by decision block 136 to further analyze the validity of the ten-second segment of RVP_{waveform} and/or the ten-second segment of PAP_{waveform}.

If RVP_valid and/or PAP_valid is true, catheter 54 is correctly placed, and RVP_{features} and RVP_{waveform} and PAP_{features} and PAP_{waveform} are of high quality (i.e., few artifacts and are suitable for further processing and analysis). If RVP_valid is true and PAP_valid is false, RVP_{features} and RVP_{waveform} may still be of high quality, with few artifacts, and may still be acceptable for further processing and analysis. For example, if SQI_{COMB} is assigned a one (1) and SQI_{RVP} is assigned a one (1), RVP_valid is still true, and RVP_{features} and RVP_{waveform} are of high quality and acceptable for further analysis. For example, validation module 36 can output a smoothed RVP_{waveform} to flow module 38 for determining blood flow. If RVP_valid is false, alone or in combination with PAP_valid being false, catheter 54 is incorrectly placed or signal-quality issues are occurring, and catheter 54 should be adjusted. RVP_{features} and RVP_{waveform} and/or PAP_{features} and PAP_{waveform} derived from incorrectly placed catheter 54 are of low quality and are rejected as unacceptable for further analysis.

Validation module 36 determines whether signal quality is sufficient and catheter 54 is operating within physiological limits. **In** some examples, validation module 36 continuously monitors the placement of catheter 54 via the signal quality. Validation module 36 analyzes RVP_{features} of RVP_{waveform} and PAP_{features} of PAP_{waveform} to determine whether catheter 54 is correctly placed and thus collecting accurate data to yield a valid RVP_{waveform} and PAP_{waveform} from which blood flow and cardiac output measurements can be derived. For example, if RVP_{waveform} or PAP_{waveform} is a flat line, catheter 54 may not be properly connected to hemodynamic monitor 12, and blood flow and cardiac output cannot be derived. Additionally, if values of RVP_{waveform} or PAP_{waveform} are above or below the designated value ranges, catheter 54 may be improperly placed and delivering data indicative of central venous pressure instead of RVP and/or PAP, respectively. **If** data is above or below designated value ranges, data is of poor quality and should not be used, and catheter 54 should be adjusted. If data is within designated value ranges, data is of high quality and may be used for further analysis, such as in a further module. Thus, identifying and monitoring placement issues and/or other data quality issues in real-time is important for notifying clinicians and disabling output of erroneous information.

### Flow Module (FIGS. 12-16)

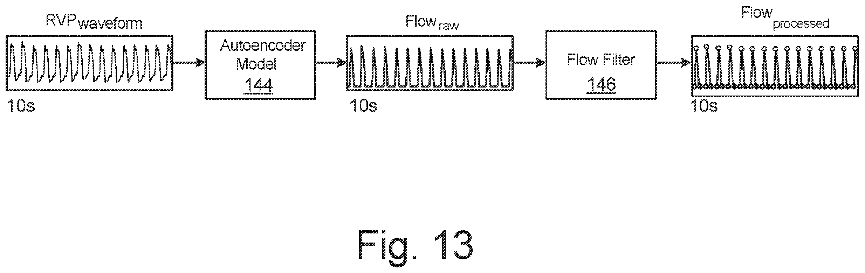

FIG. 12 is a schematic block diagram illustrating flow module 38. FIG. 13 is a schematic block diagram illustrating RVP_{waveform} transforming into flow_{processed} via flow module 38. FIGS. 12 and 13 will be discussed together. RVP_{waveform} is input into flow module 38 to predict blood flow dynamics. Flow module 38 includes autoencoder model (or "autoencoder") 144, flow filter 146, and decision block 148.

Flow module 38 receives RVP_{waveform} as an input and outputs flow_{processed}, which is an estimate of a waveform of processed blood flow for patient 16, and processed blood flow signal quality index ("SQI_{flow}"). Flow module 38 may receive a smoothed, or cleaned, RVP_{waveform} from validation module 36, as discussed above with respect to FIGS. 9-11. Additionally, RVP_{waveform} can be normalized for each patient (e.g., patient 16, as shown in FIG. 1) such that absolute values of RVP are not preserved or analyzed in flow module 38 (and CO_{AE} module 40, as shown in FIG. 17) and only relative RVP values and the shape of RVP_{waveform} are used. As seen in FIG. 13, RVP_{waveform} transforms into flow_{processed} via flow module 38. RVP_{waveform} is transformed into an estimate of a waveform of raw blood flow ("flow_{raw}") by autoencoder model 144. Flow_{raw} is subsequently transformed into flow_{processed} via flow filter 146. Flow_{raw} is processed to reflect physiological expectations of blood flow and generate SQI_{flow}. Autoencoder model 144 and flow filter 146 each analyze ten-second intervals of RVP_{waveform} and flow_{raw}, respectively, to generate beat-to-beat flow_{processed}. **In** some examples, flow module 38 continuously outputs flow_{processed}.

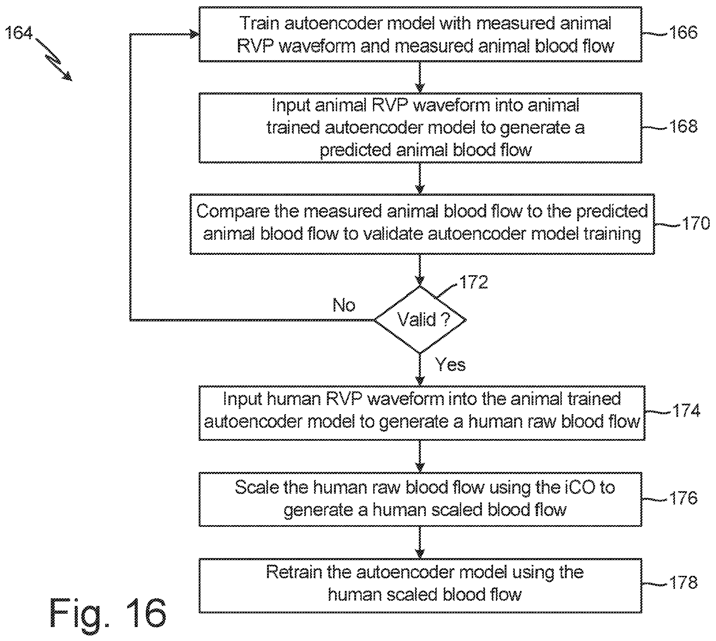

Autoencoder model 144 has an input of RVP_{waveform} and an output of flow_{raw}. Autoencoder model 144 is a deep-learning-based model, which works successfully when patient 16 has both low and high cardiac output. Autoencoder model 144 is trained to convert RVP_{waveform} into flow_{raw}, as discussed below with respect to FIG. 16. Autoencoder model 144 outputs flow_{raw} to flow filter 146.

Flow filter 146 receives flow_{raw} as an input from autoencoder model 144 and outputs flow_{processed} and SQI_{flow}. Flow filter 146 filters, or cleans, flow_{raw} by comparing features of flow_{raw} against designated value ranges for blood flow that correspond to physiological limits. As such, flow filter 146 filters flow_{raw} to remove, or exclude, from flow_{processed} artifacts or physiological inaccuracies, such as a negative flow, flow values in the thousands, or square-shaped flow, and inconsistencies, such as an inordinate, or very quick, increase in flow or inordinately dissimilar flow per beat. As a result, flow filter 146 yields a smoother, more consistent, and more accurate blood flow waveform, flow_{processed}. As such, flow_{processed} from flow filter 146 is more accurate and physiologically reasonable than flow_{raw}.

Flow filter 146 determines and assigns SQI_{flow} to flow_{processed}. SQI_{flow} is a value that indicates the quality of the waveform of flow_{processed}. Flow filter 146 compares flow_{raw} against designated value ranges for blood flow, as discussed above, to output SQI_{flow} in addition to flow_{processed}. Flow filter 146 assigns to flow_{processed} SQI_{flow} based on the amount of physiological inaccuracies and/or inconsistencies, or erroneous data, detected and removed from flow_{raw}. For example, flow_{processed} may be assigned SQI_{flow}, between zero and five (0 and 5), with zero (0) being the highest quality and five (5) being the lowest quality. SQI_{flow} may be any suitable numeric range. Flow filter 146 compares flow_{raw} to designated value ranges for blood flow. Flow filter 146 assigns SQI_{flow} to flow_{processed} based on how close flow_{raw} is to designated value ranges. Flow_{processed} may be assigned SQI_{flow} having values of two to five (2 to 5) if flow_{raw} is not within or close to all or some designated value ranges. Flow_{processed} may be assigned SQI_{flow} having values of zero to one (0 to 1) if flow_{raw} is within or close to all designated value ranges. Flow filter 146 outputs SQI_{flow} to decision block 148.

SQI_{flow} is fed into decision block 148 of flow module 38, which determines whether flow_{processed} is valid. Decision block 148 may output the determination that flow_{processed} is valid or not valid to output device 102 (shown in FIG. 6), which can be, for example, display 24 (shown in FIG. 1). Decision block 148 is a binary system, assigning a zero (0) to flow_{processed} that is not within designated value ranges and assigning a one (1) to flow_{processed} that is within designated value ranges. For example, when SQI_{flow} has a value of two to five (2 to 5), decision block 148 assigns a zero (0) to flow_{processed}. When SQI_{flow} has a value of zero or one (0 or 1), decision block 148 assigns a one (1) to flow_{processed}.

**If** SQI_{flow} is assigned a zero (0), decision block 148 determines that the flow_valid is false (e.g., "Flow_valid=false"). This is indicated by the arrow labeled "No" in FIG. 12. The determination that flow_valid is false can be sent from decision block 148 to a display, which may show an "INVALID BLOOD FLOW" reading. If SQI_{flow} is assigned a one (1), decision block 148 determines that the flow_valid is true (e.g., "Flow_valid=true"). This is indicated by the arrow labeled "Yes" in FIG. 12. The determination that the flow_valid is true can be sent from decision block 148 to a display, which may show a "VALID BLOOD FLOW" reading. Flow module 38 can also output instructions that indicate whether flow_valid is true or false to CO_{AE} module 40, which can use the valid flow_{processed} as an input.

**If** flow_valid is true, flow_{processed} is of high quality and is acceptable for further processing and analysis. Valid flow_{processed} can itself also be used to improve patient care. **If** flow_valid is false, flow_{processed} is of low quality and is rejected for further processing and analysis.

Flow module 38 transforms RVP_{waveform} into flow_{processed} and determines whether that flow_{processed} is of high quality. A high quality flow_{processed} can be used alone in patient care settings or can be used with other modules to generate cardiac output.

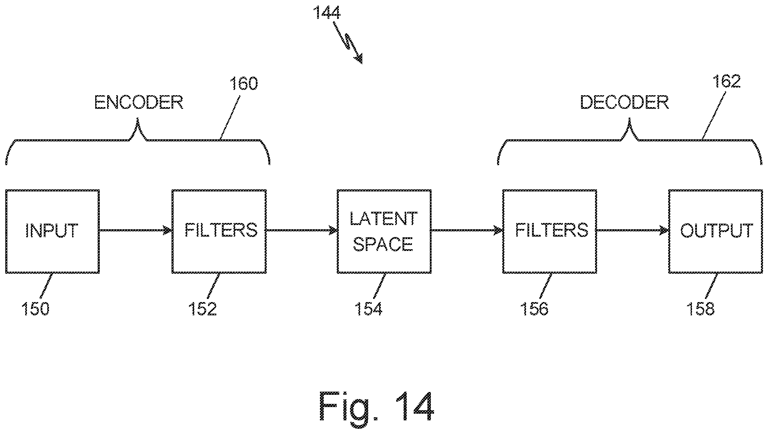

FIG. 14 is a schematic block diagram illustrating autoencoder model 144 of flow module 38. Autoencoder model 144 includes input 150, filters 152, latent space 154, filters 156, and output 158. Input 150 and filters 152 make up encoder 160, and filters 156 and output 158 make up decoder 162.

Input 150 of autoencoder model 144 is a ten-second sample of RVP_{waveform}. By utilizing a ten-second sample, the sample size is large enough that the waveform is stable and more accurate but small enough that the samples represent a continuous RVP_{waveform}. The ten-second sample of RVP_{waveform} may be about a ten-second sample (9.5 seconds to 10.5 seconds) or any other suitable time sample. Autoencoder model 144 can receive ten-second samples on a rolling basis, for example input 150 of samples are ten-second samples but may be input into autoencoder model 144 every two seconds. Input 150 is encoded via filters 152 of autoencoder model 144 into a condensed version of data from input 150 in latent space 154. Latent space 154 stores condensed data. The condensed data in latent space 154 is then decoded via filters 156 of autoencoder model 144 to become output 158. Output 158 of autoencoder model 144 is a ten-second sample of flow_{raw}. Autoencoder model 144 is trained to yield a flow_{raw} from an input of RVP_{waveform}, as described below with respect to FIG. 16. As such, encoder 160 of autoencoder model 144 is the portion of autoencoder model 144 that encodes, or compresses, input 150 of RVP_{waveform} into a smaller number of variables that include all the critical information of input 150 via filters 152. Decoder 162 of autoencoder model 144 takes such compressed encoded information and expands it via filters 156 to create output 158 of flow_{raw}.

Traditionally, blood flow measurements of patient 16 are not available to healthcare worker 18 because of the invasiveness of measuring blood flow in humans. Additionally, traditional autoencoder models are trained to learn a latent space representation of an input, the compressed input from the latent space being expanded into an output that is the same as the input. For example, a traditional autoencoder may have an input of an RVP waveform and would yield an output of an RVP waveform.

Autoencoder model 144 is a machine learning model. More specifically, autoencoder model 144 is a deep-learning-based model that is trained to use neural network architecture to convert RVP_{waveform} into flow_{raw}, as discussed below with respect to FIG. 16. Autoencoder model 144 can transform RVP_{waveform} into flow_{raw}, which can be processed via flow filter 146 to yield a robust flow_{processed}. As such, autoencoder model 144 provides a practical less invasive method for generating estimates of continuous blood flow measurements for patient 16. As a result, blood flow measurements for patient 16 are available for improved patient care. Flow_{processed} can also be used to generate a cardiac output for patient 16, as discussed below with respect to CO_{LR} module 42 and CO_{filtered} module 44.

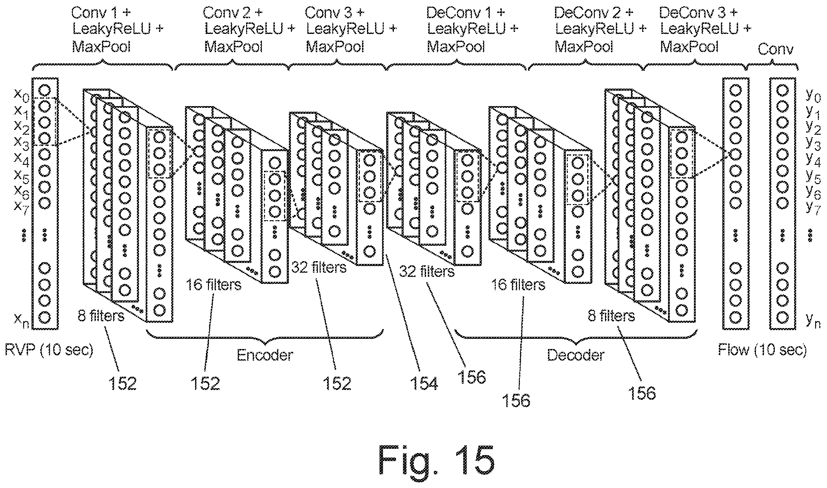

FIG. 15 is a schematic diagram illustrating filters 152 and 156 of encoder 160 and decoder 162, respectively, of autoencoder model 144. Filters 152 of encoder 160 comprise a plurality of filters 152, and filters 156 of decoder 162 comprise a plurality of filters 156.

Filters 152 are applied to input 150 of autoencoder model 144. Filters 152 are mathematical operations that transform input 150 of RVP_{waveform} into compressed data. Data samples of input 150 are reduced by a given factor by each filter 152 until fully compressed data reaches latent space 154. Filters 156 are applied to the compressed information in latent space 154 to yield output 158 of flow_{raw}. Data samples are expanded by a given factor by each filter 156 until reaching output 158. An algorithm within autoencoder model 144 autogenerates the architecture of filters 152 and filters 156, which are convolutional neural networks with auto-selected convolutions and pooling, based on input 150 and desired output 158. Complex nonlinear relationships exist among filtered data at each filtering layer 152 and 156. In the example of FIG. 15, filters 152 and 156 have an 8-16-32 architecture. A first layer of filters 152 has 8 filters, a second layer of filters 152 has 16 filters, and a third layer of filters 152 has 32 filters. A first layer of filters 156 has 32 filters, a second layer of filters 156 has 16 filters, and a third layer of filters 156 has 8 filters. In the example of FIG. 15, autoencoder model 144 has 12,000 parameters and requires 112 convolution operations. Parameters are determined as autoencoder model 144 is trained.

Filters 152 enable the compression and encoding of RVP_{waveform}. Filters 156 enable the expansion and decoding of compressed data into flow_{raw}. As a result, filters 152 and 156 of trained autoencoder model 144 can transform RVP_{waveform} into flow_{raw}.

FIG. 16 is a flow diagram illustrating process 164 for training autoencoder model 144 to predict a human flow_{raw} based on a human RVP_{waveform}. Process 164 for training autoencoder model 144 includes steps 166-178. Once autoencoder model 144 is trained via process 164, autoencoder model 144 is ready for use in flow module 38, as discussed above.

In step 166, autoencoder model 144 is trained using a measured RVP waveform of an animal and a measured blood flow of an animal. The animal RVP waveform and the animal blood flow are both known in step 166. Because directly measuring blood flow in a human is too invasive, information is derived from animal studies by directly measuring blood flow in an animal with respect to the known RVP waveform of the same animal. Autoencoder model 144 is trained using the animal RVP waveform as the input and the measured animal blood flow as the output to get an animal trained autoencoder model 144.

In step 168, the animal RVP waveform is input into animal trained autoencoder model 144 to generate a predicted animal blood flow. The known animal RVP waveform is input into animal trained autoencoder model 144, and a waveform of the predicted animal blood flow is output from autoencoder model 144.

In step 170, the measured animal blood flow is compared to the predicted animal blood flow to validate autoencoder model 144 training. Because both the animal RVP waveform and the associated waveform of measured animal blood flow are known, the predicted animal blood flow from animal trained autoencoder model 144 can be compared against the known directly measured animal blood flow with respect to various portions of the RVP waveform of the animal. Thus, the predicted animal blood flow waveform from autoencoder model 144 is compared to the directly measured animal blood flow waveform. Metrics such as mean square error (MSE), mean absolute error (MAE), correlation, and Bland-Altman analysis are used in the comparison to determine the performance of the autoencoder model 144.

In step 172, the predicted animal blood flow from animal trained autoencoder model 144 is determined to be valid or not. If the error between the predicted animal blood flow from animal trained autoencoder model 144 and the directly measured animal blood flow is below a preset threshold value, the training of autoencoder model 144 is determined to be valid. If the autoencoder model 144 training is determined to be valid, process 164 continues to step 174. If the error between the predicted animal blood flow from animal trained autoencoder model 144 and the directly measured animal blood flow is above the preset threshold value, the training of autoencoder model 144 is determined to be invalid. If autoencoder model 144 training is determined to be invalid, process 164 returns to step 166 and begins training autoencoder model 144 again. The MSE or MAE metrics are calculated iteratively during training to adjust the model parameters until convergence (e.g. the MAE or MSE in the minimum).

In step 174, a human RVP_{waveform} is input into animal trained autoencoder model 144 to generate a human raw blood flow. A known RVP_{waveform} of a human is input into validated animal trained autoencoder model 144, and a human raw blood flow waveform (i.e., a "human flow_{raw}") is output from autoencoder model 144. As such, step 174 is used to generate the shape of the human raw blood flow waveform. Because animal training of autoencoder model 144 was validated, the shape of the human raw blood flow waveform should be consistent with human blood flow (e.g., the human raw blood flow waveform is greater than zero when RVP_{waveform} indicates that valves are open and is close to zero when RVP_{waveform} indicates that valves are closed).

In step 176, the human raw blood flow waveform is scaled using iCO to generate a human scaled flow_{raw}. Because the shape of the human raw blood flow waveform is accurate, the human raw blood flow waveform is scaled at step 176 such that the average of the integral of flow_{raw} over each beat is the same as the iCO measured by catheter 54. As such, the amplitude of the waveform of flow_{raw} is adjusted. Thus, an accurate estimated flow_{raw} is generated, which is the human scaled flow_{raw}.

In step 178, autoencoder model 144 is retrained using the human scaled flow_{raw}. Autoencoder model 144 is retrained using the human RVP waveform as the input and the human scaled flow_{raw} as the output to get a human trained autoencoder model 144. Once autoencoder model 144 is retrained using the human scaled flow_{raw}, autoencoder model 144 can be used to generate a human flow_{raw} from a human RVP_{waveform} that is not known beforehand.

Animal data makes training autoencoder model 144 possible. As a result of training autoencoder model 144 using animal data, autoencoder model 144 can go from RVP_{waveform} to flow_{raw}, which cannot be measured in humans. The availability of blood flow measurements allows for further analysis of a patient's condition and improved patient care.

### CO_{AE} Module (FIG. 17)

FIG. 17 is a schematic block diagram illustrating CO_{AE} module 40, which converts flow_{processed} into autoencoder cardiac output CO_{AE}.

CO_{AE} module 40 has an input of flow_{processed} and an output of CO_{AE}. Flow_{processed} is input into CO_{AE} module 40 from flow module 38, which generates flow_{raw} via autoencoder model 144, filters flow_{raw} into flow_{processed} via flow filter 146, and determines that flow_{processed} is valid via decision block 148 prior to sending flow_{processed} on to CO_{AE} module 40. Flow module 38 also sends an instruction indicating whether flow_valid is true or false to CO_{AE} module 40 so that CO_{AE} module 40 can proceed (in response to an indication that flow_{processed} is valid) or not proceed (in response to an indication that flow_{processed} is not valid). CO_{AE} module 40 uses flow_{processed} to derive CO_{AE} for patient 16.

CO_{AE} module 40 calculates an average value of the waveform of flow_{processed} on a beat level using portions of the waveform of flow_{processed} to generate CO_{AE}. As such, the waveform of flow_{processed} is integrated to generate CO_{AE}, which is a single value that represents an estimate of cardiac output for a ten-second portion of flow_{processed}. Integration of the waveform of flow_{processed} occurs every ten seconds to yield a continuous waveform of CO_{AE}. Samples can be input into CO_{AE} module 40 more frequently than every ten seconds on a rolling basis, where samples are ten-second samples of flow_{processed} but may be input into CO_{AE} module 40 every two seconds. By utilizing a ten-second sample of flow_{processed}, the sample size is large enough that the waveform of CO_{AE} is stable and more accurate but small enough that the samples represent a continuous waveform of CO_{AE}. Having a robust continuous waveform of CO_{AE}, and not having too long of a delay in CO_{AE}, is important for frequently updating CO_{AE} to catch any changes in cardiac output for patient 16, which improves patient care.

Additionally, CO_{AE} module 40 can generally be described as "data agnostic" with respect to absolute pressure values (e.g., absolute values of RVP) because CO_{AE} can be based on relative RVP and morphology, or shape, of RVP_{waveform} and does not directly rely on absolute pressure values. This is important because, in practice, absolute pressure values may not always be accurate. For example, a healthcare worker might create some measurement error in the measurement of RVP when leveling a pressure transducer for a patient. As another example, raising or lowering a patient's bed for surgery or in the ICU can also alter absolute pressure values. CO_{AE} module 40 is able to accurately estimate CO_{AE} in these situations because it does not directly rely on absolute pressure values. Further, CO_{AE} can be accurately calculated for low cardiac outputs as well as for high cardiac outputs, which is traditionally not possible, further improving patient care. CO_{AE} module 40 can output CO_{AE} to CO_{LR} module 42 and CO_{filtered} module 44 as well as to output device 102 (shown in FIG. 6).

### CO_{LR} Module (FIGS. 18-19)

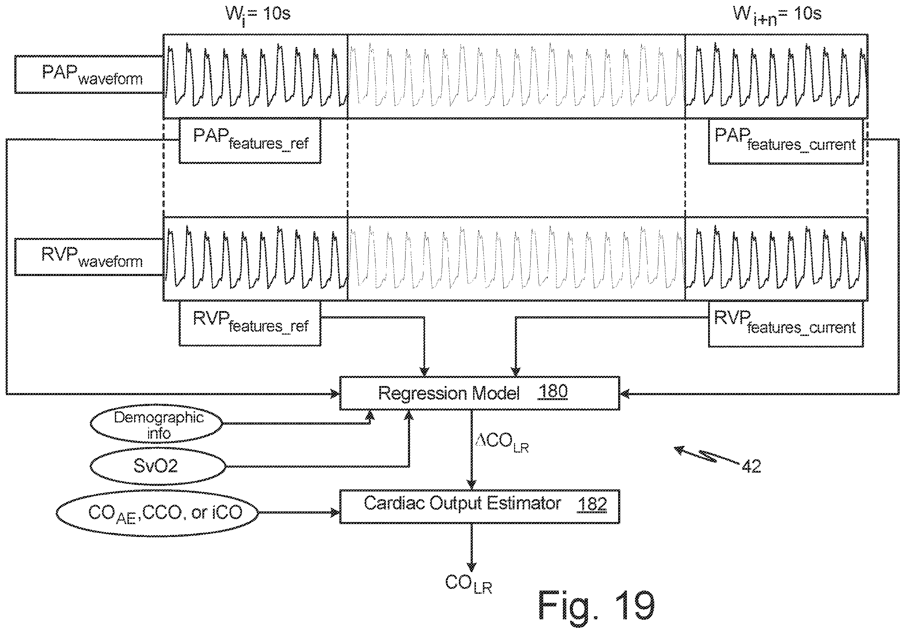

FIG. 18 is a schematic block diagram illustrating CO_{LR} module 42. FIG. 19 is a schematic block diagram illustrating reference and current features used as inputs to regression model 180 of CO_{LR} module 42. FIGS. 18-19 will be described together. RVP_{features}, PAP_{features}, demographic information, and SvO2 are input into COLR module 42 to estimate a change in cardiac output and/or a cardiac output based on features of RVP_{waveform} and PAP_{waveform}. FIGS. 18-19 show CO_{LR} module 42, including regression model 180 and cardiac output estimator 182.

Regression model 180 is a first sub-module of CO_{LR} module 42. Regression model 180 is configured to receive RVP_{features}, PAP_{features}, demographic information, and SvO2 as inputs. Regression model 180 outputs ΔCO_{LR}, which is an estimate of change in cardiac output over time for patient 16 (shown in FIG. 1). Regression model 180 is a traditional machine learning model that functions well when the available data is well-distributed rather than multi-modal. For example, patient *change* in cardiac output data- compared to patient cardiac output data-tends to be a more homogenous data set, so traditional machine learning techniques, such as regression model 180, can be used successfully to learn across the population.

Regression model 180 is trained with a training data set where a change in cardiac output and corresponding changes in features of RVP and PAP waveforms are known for a patient. **In** examples where demographic information and/or SvO2 will be used, regression model 180 can be trained with a training data set that also includes demographic information and/or SvO2 data corresponding to known change in cardiac output information.

Once regression model 180 has been trained, regression model 180 consumes RVP_{features} derived from RVP_{waveform} by RVP features module 32 (or validation module 36), as shown in FIG. 6. Regression model 180 can also consume PAP_{features} derived from PAP_{waveform} by PAP features module 34, as shown in FIG. 6. RVP_{features} and PAP_{features} are aggregated over a defined time window (or interval). For example, RVP_{features} and PAP_{features} can be aggregated over a time window of ten seconds, which includes multiple heartbeats. **In** other examples, RVP_{features} and PAP_{features} can be aggregated over longer or shorter time windows. RVP features module 32 and PAP features module 34 (shown in FIGS. 1 and 6) can extract features for each beat in the time window and then compute an average for each feature for the entire time window. The averaged features for the entire time window can be the form of RVP_{features} and PAP_{features} that are consumed by regression model 180. **In** some examples, regression model 180 only consumes averaged RVP_{features} and PAP_{features} obtained from good (i.e., valid or not discarded) beats in the time window, as determined by validation module 36 (shown in FIG. 9).

Regression model 180 can use a combined group of RVP_{features} and PAP_{features}. **In** one example, regression model 180 can use all RVP_{features} derived from RVP_{waveform} by RVP features module 32 and all PAP_{features} derived from PAP_{waveform} by PAP features module 34. **In** other examples, regression model 180 can use only some of the derived RVP_{features} and PAP_{features}. **In** yet other examples, regression model 180 uses only RVP_{features} and not PAP_{features}. Generally, regression model 180 can use any number or combination of individual RVP_{features} and PAP_{features}. Example RVP_{features} used by regression model 180 include the following: pulse rate (i.e., a pressure-based calculation of heart rate, although, in other examples, heart rate referred from an electrocardiogram or other means could also be used), maximum dP/dt, minimum dP/dt, systole time, systolic pressure, end systolic pressure, end diastolic pressure, pulse pressure, and mean pressure. Example PAP_{features} used by regression model 180 also include the following: pulse rate, maximum dP/dt, minimum dP/dt, systole time, systolic pressure, end systolic pressure, end diastolic pressure, pulse pressure, and mean pressure. Other RVP_{features} and/or PAP_{features} not specifically listed here, such as other pressure- or time-based parameters, can also be used by regression model 180. Features such as pulse rate and systole time are expected to have similar values when derived from either RVP_{waveform} or PAP_{waveform}, due to physiology. Thus, in some examples, regression model 180 can use only one source-either RVP_{features} derived from RVP_{waveform} or PAP_{features} derived from PAP_{waveform}, but not both-for features that are expected to be physiologically similar between RVP_{features} and PAP_{features}, and regression model 180 can use both RVP_{features} and PAP_{features} for other features that are expected to be physiologically different, such as maximum and minimum dP/dt.

In some examples, regression model 180 can also consume demographic information about patient 16. Such demographic information can include age, weight, height, sex (or gender), body mass index (BMI), medical condition(s), etc. Regression model 180 can receive demographic information about patient 16 either from system memory 22 (shown in FIG. 1) where it is stored (e.g., in a patient information database) or when entered by healthcare worker 18 at user interface 46 (shown in FIG. 1), for example. In some examples, regression model 180 can also consume SvO2 data received via hemodynamic sensor 14B (shown in FIG. 1 and FIG. 5). Overall, several variations of regression model 180 using different combinations of RVP_{features}, PAP_{features}, demographic information, and/or SvO2 of patient 16 (i.e., more or less complex variations of regression model 180) are possible without altering the quality of regression model 180.

As shown in FIG. 19, regression model 180 uses reference RVP features ("RVP_{features_ref}") that correspond to reference time window *Wᵢ* and current RVP features ("RVP_{features_current}") that correspond to current time window *Wᵢ₊ₙ* (where "n" represents an arbitrary integer value). RVP_{features_ref} make up a first set of individual RVP_{features} and RVP_{features_current} make up a second set of individual RVP_{features}. Similarly, regression model 180 uses reference PAP features ("PAP_{features_ref}") that correspond to reference time window *Wᵢ* and current PAP features ("PAP_{features_current}") that correspond to current time window *Wᵢ₊ₙ*. PAP_{features_ref} make up a first set of individual PAP_{features} and PAP_{features_current} make up a second set of individual PAP_{features}.

Reference time window *Wᵢ* is an initial time interval from which ΔCO_{LR} will be calculated by regression model 180. Reference time window *Wᵢ* is compared to relatively later time windows (e.g., current time window *Wᵢ₊ₙ*) to calculate ΔCO_{LR}. In some examples, reference time window *Wᵢ* can be a time interval corresponding to patient 16 first being admitted to an ICU, OR, or other patient care environment. In other examples, reference time window *Wᵢ* can be a time interval corresponding to a first known cardiac output measurement, such as a measurement of iCO, for patient 16. In yet other examples, reference time window *Wᵢ* can be any suitable time interval over which RVP_{waveform} and/or PAP_{waveform} was obtained.

CO_{LR} module 42 can be configured such that reference time window *Wᵢ* can be updated to a new time interval. For example, reference time window *Wᵢ* can be updated periodically. In some examples, reference time window *Wᵢ* can be updated upon passage of a set amount of time after which RVP_{features_ref} and PAP_{features_ref} are considered too old. In some examples, reference time window *Wᵢ* can be continuously updated at each time interval for which RVP_{waveform} and/or PAP_{waveform} are measured. That is, reference time window *Wᵢ* could also be designated reference time window *W₍ᵢ₊ₙ₎₋₁* in such examples because it would be the time interval immediately preceding current time window *Wᵢ₊ₙ*. In other examples, reference time window *Wᵢ* can be updated at any time, either manually by healthcare worker 18 via user interface 46 or automatically according to instructions in flow and cardiac output software code 30. In yet other examples, reference time window *Wᵢ* is not updated.

As shown in FIG. 19, reference time window *Wᵢ* is a ten-second (10s) interval and represents a ten-second portion of RVP_{waveform}. The length (in units of time) of reference time window *Wᵢ* can be determined based on minimum requirements for proper functioning of CO_{AE} module 40 or other modules represented in flow and cardiac output software code 30. As such, other lengths of time are possible for reference time window *Wᵢ*, such as longer or shorter than ten-second intervals. Additionally, the length of current time window *W*_{*i*+*n*} will be the same as the length of reference time window *Wᵢ* so that reference time window *Wᵢ* and current time window *Wᵢ₊ₙ* can be directly compared. Accordingly, current time window *Wᵢ₊ₙ* can also be a ten-second interval and represent a different ten-second portion of RVP_{waveform}.

Current time window *Wᵢ₊ₙ* is a current time interval to which ΔCO_{LR} will be calculated by regression model 180. Current time window *Wᵢ₊ₙ* can be any time interval after reference time window *Wᵢ.* Current time window *Wᵢ₊ₙ* can correspond to real-time or nearly real-time measurements from patient 16 and, thus, allow regression model 180 to produce a real-time or nearly real-time estimate of change in cardiac output for patient 16.

**Each individual** one of RVP_{features_ref} and PAP_{features_ref} corresponds, respectively, to an individual one of RVP_{features_current} or PAP_{features_current}. That is, RVP_{features_ref} and PAP_{features_ref} include the same individual features as RVP_{features_current} and PAP_{features_current}, respectively, measured from the corresponding time windows. For example, one of RVP_{features} used by regression model 180 can be end diastolic pressure 108 derived from RVP_{waveform}, as shown in FIG. 7. **In** such an example, regression model 180 uses an end diastolic pressure measurement derived from RVP_{waveform} during reference time window *Wᵢ* (i.e., end diastolic pressure_{w_i}) and an end diastolic pressure measurement derived from RVP_{waveform} during current time window *Wᵢ₊ₙ* (i.e., end diastolic pressure_{w_i+n}). **In** the same example, end diastolic pressure_{w_i} is one of RVP_{features_ref} and end diastolic pressure_{w_i+n} is one of RVP_{features_current}.

Regression model 180 is a linear model configured to include variables based on RVP_{features}, PAP_{features}, demographic information, and/or SvO2 for patient 16 multiplied by coefficients to estimate ΔCO_{LR}. More specifically, regression model 180 uses a change between individual features of RVP_{features_ref} and PAP_{features_ref} for reference time window *Wᵢ* and corresponding individual features of RVP_{features_current} and PAP_{features_current} for current time window *Wᵢ₊ₙ* to determine ΔCO_{LR} from reference time window *Wᵢ* to current time window *Wᵢ₊ₙ*.

The variables used in regression model 180 can take several different forms. The variables used in regression model 180 can include measured or received values of one or more of RVP_{features_ref}, PAP_{features_ref}, RVP_{features_current}, PAP_{features_current}, demographic information, and/or SvO2. The variables used in regression model 180 can also include differential variables that represent a difference between one of RVP_{features_current} or PAP_{features_current} and a corresponding one of RVP_{features_ref} or PAP_{features_ref}. The differential variables can also be represented as a ratio of the difference between a current feature and a reference feature over the reference feature to result in a normalized variable. For example, one differential variable in regression model 180 could be expressed as: (*HeartRate*_{*w*_*i+n*} - *HeartRate*_{*w*_*i*})/*HeartRate*_{*w*_*i*}.

The example differential variable shown above includes the difference between a measured value of heart rate for current time window *W*_{*i*+*n*} and for reference time window *Wᵢ* over the measured value of heart rate for reference time window *Wᵢ.* Similar differential variables can be generated for any pair of one of RVP_{features_current} or PAP_{features_current} and the corresponding one of RVP_{features_ref} or PAP_{features_ref}. The variables used in regression model 180 can further include combinatorial variables that represent a combination of one or more measured or received values of RVP_{features_ref}, PAP_{features_ref}, RVP_{features_current}, PAP_{features_current}, demographic information, and SvO2, and/or one or more differential variables. For example, one combinatorial variable in regression model 180 could be expressed as: $\left(\frac{\left({PulsePressure}_{w _ i + n}-{PulsePressure}_{w _ i}\right)}{{PulsePressure}_{w _ i}}\right) \times \left({HeartRate}_{w _ i + n}\times {SystoleTime}_{w _ i + n}\right) .$

The example combinatorial variable shown above includes a differential variable that uses a reference and current pulse pressure, a measured value of heart rate for current time window *Wᵢ₊ₙ*, and a measured value of systole time for current time window *Wᵢ₊ₙ*. Similar combinatorial variables can be generated for any combination of one or more measured or received values of RVP_{features_ref}, PAP_{features_ref}, RVP_{features_current}, PAP_{features_current}, demographic information, and SvO2, and/or one or more differential variables.

Terms in regression model 180 include, or are formed of, the variables as described above multiplied by predetermined coefficients. For example, the coefficients can be determined during the training process for regression model 180. Regression model 180 can include any number of terms from 1 to n. Regression model 180 calculates ΔCO_{LR} as the sum of all the terms. The general form of regression model 180 is as follows: ${ΔCO}_{LR} = \left({a}_{1}\times del_{feat}_{1}\right) + \left({a}_{2}\times del_{feat}_{2}\right) + \left({a}_{3}\times del_{feat}_{3}\right) + ⋯ + \left(\begin{matrix}{a}_{n} \times \\ del _ {feat}_{n}\end{matrix}\right)$ (Equation 1) Where, $del _ {feat}_{1} = \frac{\left({EndSystolicPressure}_{w _ i + n}-{EndSystolicPressure}_{w _ i}\right)}{{EndSystolicPressure}_{w _ i}} ;$ $del _ {feat}_{2} = \frac{\left({HeartRate}_{w _ i + n}-{HeartRate}_{w _ i}\right)}{{HeartRate}_{w _ i}} ;$ $del _ {feat}_{3} = \frac{\left({MaxdPdt}_{w _ i + n}-{MaxdPdt}_{w _ i}\right)}{{MaxdPdt}_{w _ i}} ;$ $del _ {feat}_{4} = \frac{\left({PulsePressure}_{w _ i + n}-{PulsePressure}_{w _ i}\right)}{{PulsePressure}_{w _ i}} ;$ $del _ {feat}_{5} = \frac{\left({SystolicPressure}_{w _ i + n}-{SystolicPressure}_{w _ i}\right)}{{SystolicPressure}_{w _ i}} ;$ $del _ {feat}_{6} = \left(\frac{\left({HeartRate}_{w _ i + n}-{HeartRate}_{w _ i}\right)}{{HeartRate}_{w _ i}}\right) \times \left({HeartRate}_{w _ i + n}\times {SystoleTime}_{w _ i + n}\right) ;$ $del _ {feat}_{7} = \left(\frac{\left({MaxdPdt}_{w _ i + n}-{MaxdPdt}_{w _ i}\right)}{{MaxdPdt}_{w _ i}}\right) \times \left({HeartRate}_{w _ i + n}\times {SystoleTime}_{w _ i + n}\right) ;$ $del _ {feat}_{8} = \left(\frac{\left({PulsePressure}_{w _ i + n}-{PulsePressure}_{w _ i}\right)}{{PulsePressure}_{w _ i}}\right) \times \left(\begin{matrix}{HeartRate}_{w _ i + n} \times \\ {SystoleTime}_{w _ i + n}\end{matrix}\right) ;$ $del _ {feat}_{9} = \left(\frac{\left({SystolicPressure}_{w _ i + n}-{SystolicPressure}_{w _ i}\right)}{{SystolicPressure}_{w _ i}}\right) \times \left(\begin{matrix}{HeartRate}_{w _ i + n} \times \\ {SystoleTime}_{w _ i + n}\end{matrix}\right) ;$ $del _ {feat}_{10} = \left(\frac{\left({EndSystolicPressure}_{w _ i + n}-{EndSystolicPressure}_{w _ i}\right)}{{EndSystolicPressure}_{w _ i}}\right) \times \left(\begin{matrix}{HeartRate}_{w _ i + n} \times \\ {SystoleTime}_{w _ i + n}\end{matrix}\right) ; and$ $del _ {feat}_{11} = \left(\frac{\left({SystoleTime}_{w _ i + n}-{SystoleTime}_{w _ i}\right)}{{SystoleTime}_{w _ i}}\right) \times \left(\begin{matrix}{HeartRate}_{w _ i + n} \times \\ {SystoleTime}_{w _ i + n}\end{matrix}\right) .$

In Equation 1 above, variables *del*_*feat₁* through *del*_*feat₁₁* are based on ones of RVP_{features}. In other examples, the variables in Equation 1 can be based on ones of PAP_{features} or can be based on a combination of RVP_{features}, PAP_{features}, and/or demographic information.

ΔCO_{LR}, as expressed in Equation 1 above, is a value representing percent change in cardiac output from an initial cardiac output corresponding to reference time window *Wᵢ* to current time window *Wᵢ₊ₙ*. ΔCO_{LR} represents a percent change rather than a magnitude of the change because variables *del*_*feat₁* through *del*_*feat₁₁* are normalized variables. ΔCO_{LR} may be particularly useful to healthcare worker 18 in this form, for example, if a percent change is more relevant compared to a magnitude of the change for a particular application or if the magnitude of the change in cardiac output can vary widely within a patient population but the percent change is more consistent. ΔCO_{LR} can be converted to a magnitude of the change by multiplying ΔCO_{LR} by a known cardiac output value, such as from a measurement of iCO or a CO_{AE} value from CO_{AE} module 40, that corresponds to reference time window *Wᵢ.*

Alternatively, regression model 180 can be configured to compute ΔCO_{LR} as a magnitude of the change by including variables that are not normalized to RVP_{features_ref} or PAP_{features_ref}. For example, one non-normalized differential variable in regression model 180 could be expressed as: $\left({EndSystolicPressure}_{w _ i + n}-{EndSystolicPressure}_{w _ i}\right) ,$ and one non-normalized combinatorial variable in regression model 180 could be expressed as: $\left({HeartRate}_{w _ i + n}-{HeartRate}_{w _ i}\right) \times \left({HeartRate}_{w _ i + n}\times {SystoleTime}_{w _ i + n}\right) .$

In yet other examples, regression model 180 can use variables that are a combination of normalized and non-normalized to compute ΔCO_{LR}. For example, one such combination could be expressed as: $\left(\frac{\left({HeartRate}_{w _ i + n}-{HeartRate}_{w _ i}\right)}{{HeartRate}_{w _ i}}\right) \times \left(\begin{matrix}{EndSystolicPressure}_{w _ i + n} - \\ {EndSystolicPressure}_{w _ i}\end{matrix}\right) .$

ΔCO_{LR} is the output of regression model 180. ΔCO_{LR} indicates how much cardiac output has changed between reference time window *Wᵢ* and current time window *Wᵢ₊ₙ* for patient 16. ΔCO_{LR} is independently important information pertaining to a hemodynamic condition of patient 16. Healthcare worker 18 can use ΔCO_{LR} from CO_{LR} module 42 to ascertain whether cardiac output for patient 16 is increasing or decreasing. For example, healthcare worker 18 could be performing an intervention for patient 16, such as administering an inotrope, and want to know how cardiac output is changing but not need to know the actual value of cardiac output at that time.

In some examples, CO_{LR} module 42 can output ΔCO_{LR} from regression model 180 to output device 102, as shown in FIG. 6. For example, output device 102 could display a graph showing values of ΔCO_{LR} over time for patient 16. In some examples, CO_{LR} module 42 continuously outputs ΔCO_{LR} to output device 102 for monitoring the hemodynamic condition of patient 16. In some examples, ΔCO_{LR} can be passed from regression model 180 to cardiac output estimator 182 within CO_{LR} module 42.

Cardiac output estimator 182 is a second sub-module of CO_{LR} module 42. Cardiac output estimator 182 receives ΔCO_{LR}, CO_{AE}, CCO, and iCO as inputs. Cardiac output estimator 182 outputs CO_{LR}, which is an estimate of cardiac output for patient 16. Cardiac output estimator 182 computes CO_{LR} from ΔCO_{LR} by using a cardiac output value that corresponds to reference time window *Wᵢ* (i.e., a "reference cardiac output").

Cardiac output estimator 182 receives ΔCO_{LR} from regression model 180. As described above, ΔCO_{LR} represents a change in cardiac output from reference time window *Wᵢ* to current time window *Wᵢ₊ₙ*. Cardiac output estimator 182 also receives CO_{AE} from CO_{AE} module 40. Specifically, cardiac output estimator 182 uses a value of CO_{AE} that corresponds to reference time window *Wᵢ.* Cardiac output estimator 182 is also configured to receive CCO and iCO measurements. Specifically, cardiac output estimator 182 uses a measurement of CCO or iCO that corresponds to reference time window *Wᵢ.* The value of CO_{AE}, the measurement of CCO, and the measurement of iCO corresponding to reference time window *Wᵢ* are each a reference cardiac output. One of the reference cardiac outputs is used to initialize or calibrate cardiac output estimator 182 (i.e., to provide a starting point from which to compute CO_{LR}).

Cardiac output estimator 182 computes CO_{LR} according to the following general form: ${CO}_{LR} = {RefCO}_{w _ i} + \left({ΔCO}_{LR}\times {RefCO}_{w _ i}\right)$ (Equation 2) Where,
*RefCO*_{*w*_*i*} is a measurement of CCO, a measurement of iCO, or a value of CO_{AE} that corresponds to reference time window *Wᵢ* (i.e., a reference cardiac output); and
*ΔCO_{LR}* is a value of ΔCO_{LR} that is output from regression model 180.

In Equation 2 above, *RefCO*_{*w*_*i*} can be a value of CO_{AE} or a measurement of CCO or iCO that corresponds to reference time window *Wᵢ*-all are not needed. For example, if a measurement of iCO corresponding to reference time window *Wᵢ* is available, CO_{LR} module 42 can be configured to use the measurement of iCO. In other examples, CO_{LR} module 42 can be configured to use CO_{AE} instead of the CCO or iCO measurement.

As shown in Equation 2, cardiac output estimator 182 adds the magnitude of the change in cardiac output to the reference cardiac output to calculate CO_{LR}. CO_{LR} is the output of cardiac output estimator 182. CO_{LR} is a value of cardiac output for patient 16 that corresponds to current time window *Wᵢ₊ₙ*. In some examples, CO_{LR} module 42 can output CO_{LR} from cardiac output estimator 182 to output device 102, as shown in FIG. 6. For example, output device 102 could display a graph showing values of CO_{LR} over time for patient 16. In some examples, CO_{LR} module 42 continuously outputs CO_{LR} to output device 102 for monitoring the hemodynamic condition of patient 16. In some examples, CO_{LR} can be passed from cardiac output estimator 182 to CO_{filtered} module 44, as will be described in greater detail below.

CO_{LR} module 42 estimates cardiac output from features of RVP_{waveform} (and optionally features of PAP_{waveform}), which is a different method of estimating a cardiac output for patient 16 compared to the method described above using autoencoder model 144, as shown in FIGS. 12-15, and CO_{AE} module 40, as shown in FIG. 17. This adds a level of redundancy within hemodynamic monitoring system 10 for estimating cardiac output. CO_{LR} module 42 allows for a second calculation of cardiac output (CO_{LR}), which can be used in addition to CO_{AE} to result in a more accurate cardiac output estimate, as will be described in greater detail below with reference to CO_{filtered} module 44 in FIGS. 20A-22. Like with CO_{AE} module 40 described above, another advantage here is that CO_{LR} module 42 allows for determining a continuous cardiac output. CO_{LR} is a continuous cardiac output estimate because it can be updated as quickly as every ten seconds or even more frequently on a rolling basis, such as every two seconds. Accordingly, CO_{LR} is a more continuous estimate compared to iCO, which is only measured intermittently. A continuous cardiac output estimate, such as CO_{LR}, can capture fleeting or rapid changes in cardiac output for patient 16, which improves patient care.

Additionally, like CO_{AE} module 40 described above, CO_{LR} module 42 can be described as "data agnostic" with respect to absolute pressure values (e.g., absolute values of RVP) because CO_{LR} that is calculated using CO_{AE} can therefore also be based on relative RVP and morphology, or shape, of RVP_{waveform} and not directly rely on absolute pressure values. Additionally, both time-based (e.g., heart rate, systole time, etc.) variables and normalized pressure-based (e.g., systolic pressure, pulse pressure, etc.) variables used in CO_{LR} module 42 are unaffected by errors or bias in absolute pressure values. This is important because, in practice, absolute pressure values may not always be accurate. For example, a healthcare worker might create some measurement error in the measurement of RVP when leveling a pressure transducer for a patient. As another example, raising or lowering a patient's bed for surgery or in the ICU can also alter absolute pressure values. CO_{LR} module 42 is able to accurately estimate CO_{LR} in these situations because it can be configured such that it does not directly rely on absolute pressure values. Moreover, CO_{LR} module 42 is also able to compute ΔCO_{LR}, which can be independently useful information about patient 16.

### CO_{filtered} Module (FIGS. 20A-22)

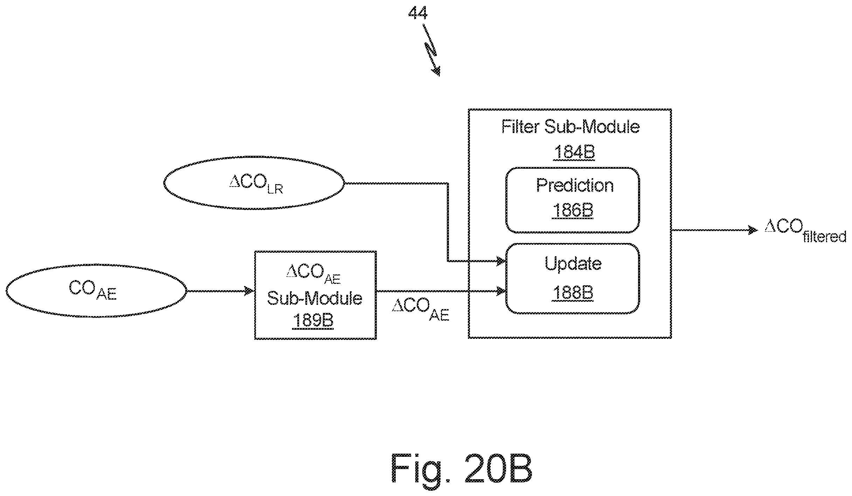

FIGS. 20A and 20B each show a respective variation of a Kalman filter algorithm of CO_{filtered} module 44. A first variation of the Kalman filter algorithm, as shown in FIG. 20A, is configured to output a filtered estimate of cardiac output. A second variation of the Kalman filter algorithm, as shown in FIG. 20B, is configured to output a filtered estimate of change in cardiac output, rather than cardiac output itself.

FIG. 20A is a schematic block diagram illustrating CO_{filtered} module 44, including filter sub-module 184A. As illustrated in FIG. 20A, CO_{filtered} module 44 includes filter sub-module 184A, including prediction block 186A and update block 188A, and ΔCO_{AE} sub-module 189A. CO_{AE}, CO_{LR}, CCO, iCO, and ΔCO_{LR} are input into CO_{filtered} module 44 to estimate CO_{filtered}. More specifically, CO_{AE} is input into ΔCO_{AE} sub-module 189A, which outputs autoencoder change in cardiac output ("ΔCO_{AE}"). Linear regression change in cardiac output ΔCO_{LR} and/or ΔCO_{AE} are input into prediction block 186A of filter sub-module 184A. CO_{AE}, CO_{LR}, CCO, and iCO are input into update block 188A of filter sub-module 184A. Filtered cardiac output CO_{filtered} is output from filter sub-module 184A.

Filter sub-module 184A is a first variation of a Kalman filter algorithm of CO_{filtered} module 44. (A second variation of a Kalman filter algorithm of CO_{filtered} module 44 is described below with reference to FIG. 20B.) Filter sub-module 184A includes instructions in code for implementing a Kalman filter algorithm to combine (or filter) one or more estimates of cardiac output into a single, more robust, filtered estimate of cardiac output.

In general, a Kalman filter algorithm predicts the current state of an input variable and then can update that prediction with additional information, such as from measured inputs, to output a filtered estimate. Filter sub-module 184A is configured to predict the current state of cardiac output for patient 16. Additionally, filter sub-module 184A consumes measured inputs over time. As shown in FIG. 20A, measured inputs that can be consumed by filter sub-module 184A include CO_{AE} and CO_{LR}, which are estimates of cardiac output estimated by flow and cardiac output software code 30, and CCO and iCO, which are measured values obtained, e.g., via catheter 54. Each of the measured inputs can include statistical noise and other inaccuracies, which can be represented in the Kalman filter algorithm of filter sub-module 184A as a corresponding uncertainty. Filter sub-module 184A outputs CO_{filtered}.

ΔCO_{AE} sub-module 189A is an additional sub-module of CO_{filtered} module 44 associated with filter sub-module 184A. ΔCO_{AE} sub-module 189A computes ΔCO_{AE} for input into filter sub-module 184A. ΔCO_{AE} sub-module 189A receives CO_{AE} as an input from CO_{AE} module 40. In turn, ΔCO_{AE} sub-module 189A outputs ΔCO_{AE}, which is consumed by filter sub-module 184A in prediction block 186A. For example, ΔCO_{AE} sub-module 189A can receive CO_{AE} values for at least two time points. ΔCO_{AE} sub-module 189A can then calculate a slope between the CO_{AE} values to determine ΔCO_{AE}. Accordingly, ΔCO_{AE} is computed in a different way and can be different than ΔCO_{LR} from CO_{LR} module 42.

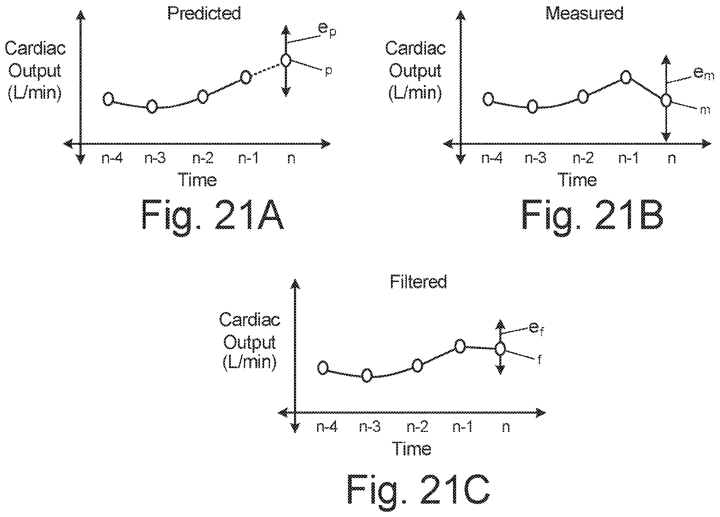

The Kalman filter algorithm of filter sub-module 184A can be conceptualized as two distinct phases or steps, including a prediction phase and an update phase. Prediction block 186A represents the prediction phase of filter sub-module 184A. As shown in FIG. 20A, ΔCO_{LR} and/or ΔCO_{AE} are input into prediction block 186A. The prediction phase of filter sub-module 184A will also be described in greater detail below with reference to FIG. 21A. Update block 188A represents the update phase of filter sub-module 184A. As shown in FIG. 20A, the measured inputs, including CO_{AE}, CO_{LR}, CCO, and iCO, are input into update block 188A. The update phase of filter sub-module 184A will also be described in greater detail below with reference to FIG. 21B.

At prediction block 186A (i.e., in the prediction phase), filter sub-module 184A predicts cardiac output for the current time step based on past filtered estimates of cardiac output from the Kalman filter algorithm (e.g., past values of CO_{filtered}, as will be described in greater detail below) and ΔCO_{LR} and/or ΔCO_{AE} (or another prediction model, as will be described below), along with a corresponding prediction uncertainty, resulting in a predicted estimate of cardiac output. The predicted estimate of cardiac output generally does not directly include information from current measured inputs. However, the prediction phase of the Kalman filter algorithm of filter sub-module 184A can be initialized with one of the measured inputs for the first iteration of the algorithm. For example, the prediction phase can be initialized with an iCO measurement. After initialization, the prediction phase will run using previous filtered estimates, as will be described in greater detail below. The Kalman filter algorithm can also be re-initialized based on predefined rules and triggers, e.g., time elapsed since the last initialization, time elapsed between the last two successive measurements, etc.

At update block 188A (i.e., in the update phase), filter sub-module 184A receives and consumes one or more of the measured inputs (e.g., one or more of CO_{AE}, CO_{LR}, CCO, and iCO) and its corresponding measurement (or estimate) uncertainty for the current time step. Once filter sub-module 184A receives a measured input, the predicted estimate of cardiac output is updated using a weighted average of the prediction and the measurement. More weight is given to estimates with greater certainty and lower uncertainty. The weighted average results in a filtered estimate of cardiac output that lies between the predicted estimate of cardiac output and the measured inputs and has a better estimated uncertainty than either alone. This process is repeated at every time step (i.e., every iteration of the Kalman filter algorithm of filter sub-module 184A), with the filtered estimate of cardiac output and its uncertainty from the previous time step informing the prediction phase in the following iteration (i.e., the current time step).

Typically, the prediction phase and the update phase alternate, with the predicted estimate of cardiac output advancing the state at each time step until the next measured input is received and the update phase subsequently incorporating the measured inputs. That is, the Kalman filter algorithm of filter sub-module 184A is configured such that each iteration of the Kalman filter algorithm can include the prediction phase and the update phase. However, if a measured input is unavailable at a particular time step, the corresponding update phase can be skipped, and multiple prediction phases can be carried out in a row. Likewise, if multiple measured inputs are received for the same time step (e.g., cardiac output estimates and/or measurements from multiple sources, such as more than one of CO_{AE}, CO_{LR}, CCO, and iCO), then multiple update phases can be carried out in a row to further refine the filtered estimate of cardiac output. Filter sub-module 184A can include an update phase for any one source or any combination of sources of measured inputs. In other words, filter sub-module 184A is configured such that the total number of update phases (which, in some cases, may be zero) per iteration of the Kalman filter algorithm depends on which of the measured inputs are available during that iteration. As will be described in greater detail below with reference to FIG. 21C, filter sub-module 184A produces a filtered estimate of cardiac output that is generally more accurate than any single one of the measured inputs.

The Kalman filter algorithm of filter sub-module 184A is recursive. That is, filter sub-module 184A uses only the filtered estimate of cardiac output from the previous time step, uncertainty associated with that previous filtered estimate of cardiac output, and the current measured input to compute the filtered estimate of cardiac output for the current time step-no other past information is needed. For each time step, the previous filtered estimate of cardiac output is fed back into prediction block 186A to inform the upcoming prediction of filter sub-module 184A. In this way, information about previous measured inputs and their respective uncertainties is still carried into the next prediction because the previous filtered estimate of cardiac output will have been refined based on the measured inputs from that time step.

The final filtered estimate of cardiac output produced by filter sub-module 184A at each time step is a value of CO_{filtered}. CO_{filtered} is a filtered representation of a right ventricular cardiac output ("RVCO"). CO_{filtered} is the output of filter sub-module 184A and, therefore, is one possible output of CO_{filtered} module 44. In some examples, CO_{filtered} module 44 can output CO_{filtered} from filter sub-module 184A to output device 102, as shown in FIG. 6. For example, output device 102 could display a graph showing values of CO_{filtered} over time for patient 16. In some examples, CO_{filtered} module 44 continuously outputs CO_{filtered} to output device 102 for monitoring the hemodynamic condition of patient 16.

FIG. 20B is a schematic block diagram illustrating CO_{filtered} module 44, including filter sub-module 184B. As illustrated in FIG. 20B, CO_{filtered} module 44 includes filter sub-module 184B, including prediction block 186B and update block 188B, and ΔCO_{AE} sub-module 189B. CO_{AE} and ΔCO_{LR} are input into CO_{filtered} module 44 to estimate ΔCO_{filtered}. More specifically, CO_{AE} is input into ΔCO_{AE} sub-module 189B, which outputs ΔCO_{AE}. ΔCO_{LR} and ΔCO_{AE} are input into update block 188B of filter sub-module 184B. Filtered change in cardiac output ΔCO_{filtered} is output from filter sub-module 184B.

Filter sub-module 184B is a second variation of a Kalman filter algorithm of CO_{filtered} module 44. Filter sub-module 184B includes instructions in code for implementing a Kalman filter algorithm to combine (or filter) one or more estimates of change in cardiac output into a single, more robust, filtered estimate of change in cardiac output.

Filter sub-module 184B is generally similar in function to filter sub-module 184A described above with reference to FIG. 20A, except filter sub-module 184B is configured to filter multiple change in cardiac output values instead of multiple cardiac output values. For simplicity, features and functionality of filter sub-module 184B that are generally the same as for filter sub-module 184A (as shown in FIG. 20A) will not be fully repeated in this section, except to the extent needed to describe any differences.

Filter sub-module 184B is configured to predict the current state of change in cardiac output for patient 16. Additionally, filter sub-module 184B consumes measured inputs over time. As shown in FIG. 20B, measured inputs that can be consumed by filter sub-module 184B include ΔCO_{AE} and ΔCO_{LR}, which are each estimates of change in cardiac output estimated by flow and cardiac output software code 30. Each of the measured inputs can include statistical noise and other inaccuracies, which can be represented in the Kalman filter algorithm of filter sub-module 184B as a corresponding uncertainty. Filter sub-module 184B outputs ΔCO_{filtered}.

ΔCO_{AE} sub-module 189B is an additional sub-module of CO_{filtered} module 44 associated with filter sub-module 184B. Similar to ΔCO_{AE} sub-module 189A described above with reference to FIG. 20A, ΔCO_{AE} sub-module 189B computes ΔCO_{AE} for input into filter sub-module 184B. ΔCO_{AE} sub-module 189B receives CO_{AE} as an input from CO_{AE} module 40. In turn, ΔCO_{AE} sub-module 189B outputs ΔCO_{AE}, which is consumed by filter sub-module 184B in update block 188B. For example, ΔCO_{AE} sub-module 189B can receive CO_{AE} values for at least two time points. ΔCO_{AF} sub-module 189B can then calculate a slope between the CO_{AE} values to determine ΔCO_{AE}. Accordingly, ΔCO_{AE} is computed in a different way and can be different than ΔCO_{LR} from CO_{LR} module 42.

Prediction block 186B represents the prediction phase of filter sub-module 184B. At prediction block 186B (i.e., in the prediction phase), filter sub-module 184B predicts change in cardiac output for the current time step based on past filtered estimates of change in cardiac output from the Kalman filter algorithm (e.g., past values of ΔCO_{filtered}, as will be described in greater detail below), along with a corresponding prediction uncertainty, resulting in a predicted estimate of change in cardiac output. The predicted estimate of change in cardiac output generally does not directly include information from current measured inputs. However, the prediction phase of the Kalman filter algorithm of filter sub-module 184B can be initialized with one of the measured inputs for the first iteration of the algorithm. For example, the prediction phase can be initialized with a value of either ΔCO_{AE} or ΔCO_{LR}. After initialization, the prediction phase will run using previous filtered estimates, as will be described in greater detail below. The Kalman filter algorithm can also be re-initialized based on predefined rules and triggers, e.g., time elapsed since the last initialization, time elapsed between the last two successive measurements, etc.

Update block 188B represents the update phase of filter sub-module 184B. As shown in FIG. 20B, the measured inputs, including ΔCO_{AE} and ΔCO_{LR}, are input into update block 188B. At update block 188B (i.e., in the update phase), filter sub-module 184B receives and consumes one or more of the measured inputs (e.g., one or more of ΔCO_{AE} and ΔCO_{LR}) and its corresponding measurement (or estimate) uncertainty for the current time step. Once filter sub-module 184B receives a measured input, the predicted estimate of change in cardiac output is updated using a weighted average of the prediction and the measurement. More weight is given to estimates with greater certainty and lower uncertainty. The weighted average results in a filtered estimate of change in cardiac output that lies between the predicted estimate of change in cardiac output and the measured inputs and has a better estimated uncertainty than either alone. This process is repeated at every time step (i.e., every iteration of the Kalman filter algorithm of filter sub-module 184B), with the filtered estimate of change in cardiac output and its uncertainty from the previous time step informing the prediction phase in the following iteration (i.e., the current time step). Similar to filter sub-module 184A described above with reference to FIG. 20A, the prediction phase and update phase of filter sub-module 184B can alternate or, in other examples, the update phase can be skipped or repeated, depending on the availability of the measured inputs. Filter sub-module 184B produces a filtered estimate of change in cardiac output that is generally more accurate than any single one of the measured inputs.

The Kalman filter algorithm of filter sub-module 184B is recursive. That is, filter sub-module 184B uses only the filtered estimate of change in cardiac output from the previous time step, uncertainty associated with that previous filtered estimate of change in cardiac output, and the current measured input to compute the filtered estimate of change in cardiac output for the current time step. For each time step, the previous filtered estimate of change in cardiac output is fed back into prediction block 186B to inform the upcoming prediction of filter sub-module 184B. In this way, information about previous measured inputs and their respective uncertainties is still carried into the next prediction because the previous filtered estimate of change in cardiac output will have been refined based on the measured inputs from that time step.

The final filtered estimate of change in cardiac output produced by filter sub-module 184B at each time step is a value of ΔCO_{filtered}. Filtered change in cardiac output ΔCO_{filtered} is the output of filter sub-module 184B and, therefore, is another possible output of CO_{filtered} module 44. In some examples, CO_{filtered} module 44 can output ΔCO_{filtered} from filter sub-module 184B to output device 102, as shown in FIG. 6. For example, output device 102 could display a graph showing values of ΔCO_{filtered} over time for patient 16. In some examples, CO_{filtered} module 44 continuously outputs ΔCO_{filtered} to output device 102 for monitoring the hemodynamic condition of patient 16.

Although illustrated as separate examples in FIGS. 20A-20B, CO_{filtered} module 44 can be configured to include either or both filter sub-module 184A and filter sub-module 184B. In one example, CO_{filtered} module 44 only includes filter sub-module 184A and, as such, is configured to filter cardiac output values. In another example, CO_{filtered} module 44 only includes filter sub-module 184B and, as such, is configured to filter change in cardiac output values. In yet other examples, CO_{filtered} module 44 includes filter sub-module 184A and filter sub-module 184B and, as such, is configured to filter both cardiac output and change in cardiac output values.

Referring to FIGS. 20A and 20B together, the Kalman filter algorithms of filter sub-module 184A and filter sub-module 184B are powerful estimators when there are multiple noisy estimates and/or measurements for a variable, such as cardiac output or change in cardiac output. Due to the nature of the Kalman filter algorithm, including the prediction phase and the update phase, filter sub-module 184A and filter sub-module 184B can effectively handle uncertainty due to noisy measured inputs and produce a more accurate CO_{filtered} and ΔCO_{filtered}. Moreover, filter sub-module 184A and filter sub-module 184B can operate on cardiac output and change in cardiac output estimates and/or measurements received from different sources to merge these into the more accurate CO_{filtered} and ΔCO_{filtered}.

Filter sub-module 184A and filter sub-module 184B can also tolerate receiving asynchronous or sporadic cardiac output and change in cardiac output estimates and/or measurements from the different sources. That is, filter sub-module 184A and filter sub-module 184B do not require measured inputs from all the possible sources at each time step and can even tolerate not receiving any measured input at all for a particular time step. For example, filter sub-module 184A could be receiving measured inputs from both CO_{AE} module 40 and CO_{LR} module 42, and then at some time step, flow module 38 could determine that autoencoder model 144 (as shown in FIG. 12) is no longer producing good values, so CO_{AE} would not be computed for that time step and not fed to filter sub-module 184A. In such an example, the Kalman filter algorithm of filter sub-module 184A would automatically adapt to this change and could continue generating CO_{filtered} using only the measured input from CO_{LR} module 42. The Kalman filter algorithm could also automatically re-adapt to using both measured inputs from CO_{AE} module 40 and CO_{LR} module 42 at a future time. Likewise, if additional sources of cardiac output or change in cardiac output estimates and/or measurements were added to hemodynamic monitoring system 10 (e.g., measurements from additional or different devices, such as an echocardiogram, etc., or estimates from other models), these could be readily integrated into the Kalman filter algorithm as additional update phases.

Using a Kalman filter algorithm in filter sub-module 184A and filter sub-module 184B is very computationally efficient because the Kalman filter algorithm is recursive. Likewise, the recursive nature of the Kalman filter algorithm means it does not merge information in isolation. Instead, the Kalman filter algorithm allows filter sub-module 184A and filter sub-module 184B to intelligently combine information using the context of what the filtered estimate of cardiac output or change in cardiac output was at the previous time step. In this way, CO_{filtered} and ΔCO_{filtered} can be more accurate estimates of cardiac output for patient 16. Moreover, like with CO_{AE} module 40 and CO_{LR} module 42 described above, another advantage here is that CO_{filtered} module 44 using filter sub-module 184A allows for determining a continuous cardiac output. CO_{filtered} is a continuous cardiac output estimate because it can be updated as quickly as every ten seconds or even more frequently on a rolling basis, such as every two seconds. Accordingly, CO_{filtered} is a more continuous estimate compared to iCO, which is only measured intermittently. A continuous cardiac output estimate, such as CO_{filtered}, can capture fleeting or rapid changes in cardiac output for patient 16, which improves patient care.

Additionally, like CO_{AE} module 40 and CO_{LR} module 42 described above, CO_{filtered} module 44 can be described as "data agnostic" with respect to absolute pressure values (e.g., absolute values of RVP) because CO_{filtered} that is based on CO_{AE} can therefore also be based on relative RVP and morphology, or shape, of RVP_{waveform}, and CO_{filtered} that is based on CO_{LR} can likewise be unaffected by absolute pressure values. This is important because, in practice, absolute pressure values may not always be accurate. For example, a healthcare worker might create some measurement error in the measurement of RVP when leveling a pressure transducer for a patient and, as another example, raising or lowering a patient's bed for surgery or in the ICU can also alter absolute pressure values. CO_{filtered} module 44 using filter sub-module 184A is able to accurately generate CO_{filtered} in these situations because it can be configured such that it does not directly rely on absolute pressure values.

FIG. 21A is a graph illustrating predicted value *p* of CO_{filtered} module 44. FIG. 21B is a graph illustrating measured value *m* of CO_{filtered} module 44 over time. FIG. 21C is a graph illustrating filtered value *f* from CO_{filtered} module 44 over time. FIGS. 21A-21C will be discussed together. Taken together, FIGS. 21A-21C are graphs corresponding to the functioning of filter sub-module 184A (as shown in FIG. 20A) over time, and each of the graphs in FIGS. 21A-21C show cardiac output values over time. However, it should be understood that the concepts described with respect to FIGS. 21A-21C are also generally applicable to the functioning of filter sub-module 184B (as shown in FIG. 20B), and the graphs in FIGS. 21A-21C could instead represent change in cardiac output values over time.

Each of the graphs in FIGS. 21A-21C shows the same arbitrary time steps *n, n*-1*, n*-2*, n*-3*,* and *n*-4 along the x-axis. Each of time steps *n, n*-1*, n*-2, *n-*3, and *n*-4 corresponds to a respective iteration of the Kalman filter algorithm of filter sub-module 184A. That is, time step *n* represents the current iteration of filter sub-module 184A. Time steps *n*-1, *n-*2, *n-*3, and *n*-4 represent time steps preceding time step *n.* For example, time step *n*-1 is the time step representing the iteration immediately before time step *n*, time step *n*-2 is the time step representing the iteration immediately before time step *n*-1 (and two before time step *n*), etc. As will be described in greater detail below, each of the graphs in FIGS. 21A-21C also shows cardiac output values corresponding to the time steps *n, n*-1, *n-*2, *n*-3, and *n*-4. The cardiac output values graphed in FIGS. 21A-21C are shown with units of liters per minute (L/min) but, in other examples, could be represented with other rate units.

FIG. 21A is a graph that corresponds to the functioning of prediction block 186A, as shown in FIG. 20A. In FIG. 21A, a cardiac output value is shown for each of time steps *n, n*-1, *n*-2, *n*-3, and *n*-4. Predicted value *p* is the cardiac output value corresponding to current time step *n.* Predicted value *p* is an example value of a predicted estimate of cardiac output predicted at prediction block 186A. The cardiac output values corresponding to time steps *n*-1, *n*-2, *n*-3, and *n*-4 can be previous values of CO_{filtered} that can be used in current time step *n* (or that were used in previous time steps) by prediction block 186A to produce predicted value *p* that corresponds to current time step *n.* If any of time steps *n*-1, *n*-2, *n*-3, and *n*-4 represent the first time step for which predicted value *p* was calculated, then the cardiac output values at the previous time steps with respect to that first time step can be measured input values used for initialization instead. Predicted value *p* at current time step *n* is illustrated in FIG. 21A as connected to the previous value with a dashed line because predicted value *p* is a predicted estimate of cardiac output used internally within filter sub-module 184A. Unlike the other cardiac output values shown in FIG. 21A for previous time steps, predicted value *p* is generally not the final filtered output of filter sub-module 184A.

As shown in FIG. 21A, predicted value *p* has corresponding uncertainty *eₚ.* Uncertainty *eₚ* represents the uncertainty associated with predicted value *p* as an estimate of the standard deviation. The uncertainty in predicted value *p* can be configurable or predetermined for each application of filter sub-module 184A and can depend on the type of prediction that is used. The arrows extending from predicted value *p* are proportional to the amount of uncertainty in predicted value *p*. In one, non-limiting example, predicted value *p* could have an associated uncertainty *eₚ* of ±0.4 L/min, so if predicted value *p* is 4.0 L/min, the range illustrated by the arrows for uncertainty *eₚ* would be 3.6-4.4 L/min, signifying that there is an approximately 68% chance that the true value falls within this range.

The prediction phase of the Kalman filter algorithm of filter sub-module 184A can be configured as one of several different prediction models to produce predicted value *p*. In one example, the prediction phase can use extrapolation to compute predicted value *p*. For example, the prediction phase can use linear extrapolation based on the cardiac output values for time steps *n*-1 and *n*-2 to determine the value of predicted value *p* at current time step *n*. Using linear extrapolation in the prediction phase means that the change in cardiac output (i.e., the slope) between the previous two time steps will be carried over as the same up to current time step *n*. That is, the slope between the cardiac output values at time steps *n*-2 and *n*-1 is taken to be the same between time steps *n*-1 and *n*. In other examples, the prediction phase can use extrapolation techniques with other types of fit functions, such as cubic fit, etc., and by incorporating additional past cardiac output values (e.g., for time steps *n*-3, *n*-4, etc.). For example, prediction block 186A could take the last four values of CO_{filtered} and fit a cubic function onto those points to determine predicted value *p* at current time step *n.* In yet other examples, such as examples where the system is stable, prediction block 186A could use the value of CO_{filtered} for time step *n*-1 and extrapolate predicted value *p* at current time *n* as unchanged from (i.e., the same as) the value of CO_{filtered} for time step *n*-1*.*

Alternatively, the prediction phase can use ΔCO_{LR} and/or ΔCO_{AE} to compute predicted value *p*. For example, prediction block 186A can start with the value of CO_{filtered} at time step *n*-1 and add the value of ΔCO_{LR} or ΔCO_{AE} for current time step *n* to determine predicted value *p* at current time step *n.* In other examples, predication block 186A could add the average (e.g., a weighted average based on designated or predetermined weights, such as 0.5 and 0.5 for ΔCO_{LR} and ΔCO_{AE}, respectively) of ΔCO_{LR} and ΔCO_{AE} for current time step *n* to the value of CO_{filtered} at time step *n*-1 to determine predicted value *p* at current time step *n*. In yet other examples, prediction block 186A could determine a first predicted value by adding the value of ΔCO_{LR} for current time step *n* to the value of CO_{filtered} at time step *n*-1, determine a second predicted value by adding the value of ΔCO_{AE} for current time step *n* to the value of CO_{filtered} at time step *n*-1, and then determine predicted value *p* as the average (e.g., a weighted average) of the first and second predicted values. Further details and examples of weighting inputs will be described below with reference to FIG. 21B but are also applicable here. In one, non-limiting example, the designated or predetermined weights associated with ΔCO_{LR} and ΔCO_{AE} could be based on the relative accuracy (or uncertainty) associated with the two models (i.e., regression model 180, as shown in FIGS. 18-19, and autoencoder model 144, as shown in FIGS. 12-15). In yet another example, the weights associated with ΔCO_{LR} and ΔCO_{AE} may change over time depending on the relative signal quality index (or indices) associated with such methods. Compared to extrapolation, these prediction models only require the value of CO_{filtered} for the immediately previous time step (e.g., time step *n*-1) rather than for one or more previous time steps. The tradeoff with using ΔCO_{LR} and/or ΔCO_{AE} instead of extrapolation is that the quality of the prediction will depend to some extent on the functioning of regression model 180 and autoencoder model 144. For example, as the reference features used in regression model 180 become older, the quality of ΔCO_{LR} can diminish, so a prediction using ΔCO_{LR} may be improved if RVP_{features_ref} and PAP_{feature_ref} have been updated more recently. It should be noted that the prediction model using ΔCO_{LR} and/or ΔCO_{AE} is generally only used in prediction block 186A of filter sub-module 184A (shown in FIG. 20A) but not in prediction block 186B of filter sub-module 184B (shown in FIG. 20B) because prediction block 186B does not receive any change in cardiac output values as inputs. That is, prediction block 186B makes predictions using extrapolation, whereas prediction block 186A can make predictions using either extrapolation or using ΔCO_{LR} and/or ΔCO_{AE}.

Each of the prediction models described above uses the rate of change of cardiac output, either by extrapolation or using ΔCO_{LR}, to make a prediction about the current value of cardiac output for patient 16. Moreover, each of the prediction models is based on the underlying assumption that the change in cardiac output within the measurement time window (e.g., a ten-second interval) can be represented by a single aggregate or average value.

FIG. 21B is a graph that corresponds to the functioning of update block 188A, as shown in FIG. 20A. In FIG. 21B, a cardiac output value is shown for each of time steps *n, n*-1*, n*-2*, n-3,* and *n*-4*.* Measured value *m* is the cardiac output value corresponding to current time step *n*. Measured value *m* is an example value of a measured input received by filter sub-module 184A at update block 188A. As described above, the measured input can be any one (or more, although only one is shown in FIG. 21B for simplicity) of CO_{AE}, CO_{LR}, CCO, or iCO. Measured value *m* is used by update block 188A to update predicted value *p* for current time step *n*. The cardiac output values corresponding to time steps *n*-1, *n*-2, *n*-3, and *n*-4 are previous measured input values that were used in previous time steps by update block 188A to update previous predicated estimates of cardiac output for the respective time steps.

FIG. 21B shows cardiac output values from a single source, such as one of CO_{AE} module 40, CO_{LR} module 42, CCO, or iCO. That is, measured value *m* is one of CO_{AE}, CO_{LR}, CCO, or iCO. In this example, the Kalman filter algorithm of filter sub-module 184A would only have one update phase using measured value *m*. In other examples where cardiac output values are received from multiple sources, there would be multiple measured values *m* at current time step *n.*

As shown in FIG. 21B, measured value *m* has corresponding uncertainty *eₘ.* Uncertainty *eₘ* represents the uncertainty in measured value *m* as an estimate of the standard deviation. The uncertainty in measured value *m* can be configurable or predetermined for each application of filter sub-module 184A and can depend on the type of measured input that is used. The arrows extending from measured value *m* are proportional to the amount of uncertainty in measured value *m.* In one, non-limiting example, CO_{AE} from CO_{AE} module 40 could have an associated uncertainty *eₘ* of 0.5 L/min, so if measured value *m* (of CO_{AE}) is 5.0 L/min, the range illustrated by the arrows for uncertainty *eₘ* would be 4.5-5.5 L/min, signifying that there is an approximately 68% chance that the true value falls within this range. Uncertainty *eₘ* can be the same or different from uncertainty *eₚ*, depending on how the respective uncertainties are determined.

In the update phase or phases of the Kalman filter algorithm of filter sub-module 184A, predicted value *p* and measured value *m* are dynamically or adaptively weighted to generate filtered value *f*, as shown in FIG. 21C. This weighting can result in an uncertainty associated with filtered value *f* at current time step *n* that is expected to be the same or less than either of uncertainty *eₚ* or uncertainty *eₘ.*

When the Kalman filter algorithm of filter sub-module 184A is initialized, uncertainty *eₚ* and uncertainty *eₘ* are specified. If there are multiple sources of measured inputs (e.g., CO_{AE} module 40, CO_{LR} module 42, CCO, and/or iCO), each source can have a different associated uncertainty *eₘ.* Uncertainty *eₚ* of predicted value *p* includes uncertainty associated with the prediction model and any uncertainty associated with past values of CO_{filtered} that were used to determine predicted value *p*. Accordingly, once filter sub-module 184A has determined predicted value *p* and received measured value *m* for current time step *n*, filter sub-module 184A also has information about corresponding uncertainty *eₚ* and uncertainty *eₘ.* Predicted value *p* and measured value *m* are weighted based on their associated uncertainties *eₚ* and *eₘ*, respectively. The one of predicted value *p* and measured value *m* that has a greater uncertainty will be given less weight, and the one of predicted value *p* and measured value *m* that has a lower uncertainty will be given more weight. Using the examples from above to illustrate, if uncertainty *eₚ* was 0.4 L/min and uncertainty *eₘ* was 0.5 L/min, predicted value *p* would be given more weight and measured value *m* would be given less weight.

Each of uncertainty *eₚ* and uncertainty *eₘ* can be fixed or modulated over time. For example, uncertainty *eₘ* could be scaled based on SQI_{RVP} or SQI_{PAP}, as described above with reference to FIG. 9. To illustrate, if the value of SQI_{RVP} is zero to one (0 to 1, indicating a signal quality within the designated range), then flow and cardiac output software code 30 (shown in FIG. 1) can proceed to estimate CO_{AE} and CO_{LR} using the corresponding RVP_{waveform} data. However, there may be a different uncertainty for CO_{AE} and CO_{LR} associated with a signal quality index of zero versus a signal quality index of one (SQI_{RVP}=0 vs. SQI_{RVP}=1), even though both are valid. For example, uncertainty in measurements might be lower when SQI_{RVP}=0, so uncertainty *eₘ* associated with measured value *m* could be multiplied by one (or unchanged). In the same example, uncertainty in measurements might be higher when SQI_{RVP}=1, so uncertainty *eₘ* associated with measured value *m* could be multiplied by some factor, such as two or more, to increase the uncertainty. In other examples, uncertainty *eₚ* and/or uncertainty *eₘ* could be scaled based on factors other than SQI_{RVP} and SQI_{PAP}.

FIG. 21C is a graph that corresponds to the functioning of filter sub-module 184A using information from prediction block 186A and update block 188A, as shown in FIG. 20A and described with reference to FIGS. 21A-21B above. In FIG. 21C, a cardiac output value is shown for each of time steps *n, n*-1, *n*-2, *n*-3, and *n*-4. Filtered value *f is* the cardiac output value corresponding to current time step *n*. Filtered value *f* is an example value of CO_{filtered} that is output by filter sub-module 184A for current time step *n*. Filtered value *f* is also used by prediction block 186A in the prediction for the next time step. The cardiac output values corresponding to time steps *n*-1, *n*-2, *n*-3, and *n*-4 are previous CO_{filtered} values that were output by filter sub-module 184A in previous time steps.

Filtered value *f*, and its associated uncertainty *e_{f},* are computed according to the following general forms using predicted value *p*, measured value *m*, and associated uncertainties *eₚ* and *eₘ*: $f = \left(\frac{{e}_{m}^{2}}{\left({e}_{p}^{2}+{e}_{m}^{2}\right)}\times p\right) + \left(\frac{{e}_{p}^{2}}{\left({e}_{p}^{2}+{e}_{m}^{2}\right)}\times m\right)$ (Equation 3) Where, ${e}_{f} \leq {e}_{m} ;$ ${e}_{f} \leq {e}_{p} ;$ and $\frac{1}{{{e}_{f}}^{2}} = \left(\frac{1}{{{e}_{p}}^{2}}\right) + \left(\frac{1}{{{e}_{m}}^{2}}\right) .$ (Equation 4) Moreover, Equation 3 can be simplified and re-written to clearly show the update of prediction value *p* using measured value *m* (i.e., the measured inputs in the Kalman filter algorithm): $f = p + \left(\frac{{{e}_{p}}^{2}}{\left({{e}_{p}}^{2}+{{e}_{m}}^{2}\right)}\times \left(m-p\right)\right)$ (Equation 5) Equation 5 can also be expressed as: $f = p + \left(K\times \left(m-p\right)\right)$ (Equation 6)

**Where,**
*K* is the Kalman Gain $= \frac{{{e}_{p}}^{2}}{\left({{e}_{p}}^{2}+{{e}_{m}}^{2}\right)}$; ${e}_{f} \leq {e}_{m} ;$ and ${e}_{f} \leq {e}_{p} .$

To extend Equation 3 to show two simultaneous measured values *m* from two different sources of measured inputs at a time (e.g., CO_{LR} and CO_{AE}), Equation 3 can be re-written as: $\begin{matrix}f = \left(\frac{\frac{1}{{{e}_{p}}^{2}}}{\left(\frac{1}{{{e}_{p}}^{2}}\right) + \left(\frac{1}{{{e}_{m _ 1}}^{2}}\right) + \left(\frac{1}{{{e}_{m _ 2}}^{2}}\right)}\times p\right) + \left(\frac{\frac{1}{{{e}_{m _ 1}}^{2}}}{\left(\frac{1}{{{e}_{p}}^{2}}\right) + \left(\frac{1}{{{e}_{m _ 1}}^{2}}\right) + \left(\frac{1}{{{e}_{m _ 2}}^{2}}\right)}\times m_1\right) + \\ \left(\frac{\frac{1}{{{e}_{m _ 2}}^{2}}}{\left(\frac{1}{{{e}_{p}}^{2}}\right) + \left(\frac{1}{{{e}_{m _ 1}}^{2}}\right) + \left(\frac{1}{{{e}_{m _ 2}}^{2}}\right)}\times m_2\right)\end{matrix}$ (Equation 7)

Where,
*m*_1 is a first measured value from a first source of the measured inputs, such as one of CO_{AE}, CO_{LR}, CCO, or iCO;
*m*_2 is a second measured value from a second source of the measured inputs, such as a different one of CO_{AE}, CO_{LR}, CCO, or iCO; ${e}_{f} \leq {e}_{m _ 1} ;$ ${e}_{f} \leq {e}_{m _ 2} ;$ ${e}_{f} \leq {e}_{p} ;$ and $\frac{1}{{{e}_{f}}^{2}} = \left(\frac{1}{{{e}_{p}}^{2}}\right) + \left(\frac{1}{{{e}_{m _ 1}}^{2}}\right) + \left(\frac{1}{{{e}_{m _ 2}}^{2}}\right) .$ (Equation 8)

As shown in Equation 3, predicted value *p* is weighted by the respective contribution of measured value *m* to the overall noise, and measured value *m* is weighted by the respective contribution of predicted value *p* to the overall noise. In other words, whichever value of predicted value *p* and measured value *m* has a greater uncertainty will cause the other value to be weighted more. To illustrate, if predicted value *p* had uncertainty *eₚ*=0 (no uncertainty), then Equation 3 simplifies so that predicted value *p* is weighted by one (*eₘ*/*eₘ*) and measured value *m* is weighted by zero (0/*eₘ*). Generally, filtered value *f* is different than both predicted value *p* and measured value *m.* However, if measured value *m* is the same as predicted value *p,* then filtered value *f* will also be the same regardless of the weighting in Equation 3.

Equation 7 shows how filtered value *f* is calculated when there are two simultaneous measured values *m,* such as from two different sources of measured inputs. Equation 7 can be further expanded to include any number of measured values *m* (e.g., *m_1, m_2,* ... *m_n*). For example, filtered value *f* could be computed using a measured value *m* from each of the sources of measured inputs that is available in the system, including CO_{AE}, CO_{LR}, CCO, and iCO.

As shown in FIG. 21C, filtered value *f* has corresponding uncertainty *e_{f}.* Uncertainty *e_{f}* represents the uncertainty in filtered value *f.* The arrows extending from filtered value *f* are proportional to the amount of uncertainty in filtered value *f*. In an example that includes a single measured value *m,* uncertainty *e_{f}* can be determined using Equation 4 above. In an example that includes multiple measured values *m,* uncertainty *e_{f}* can be determined using Equation 8 above. As described above, uncertainty *e_{f}* is ideally less than both uncertainty *eₚ* and uncertainty *eₘ* individually. Accordingly, the arrows indicating uncertainty *e_{f}* are shorter than the arrows indicating uncertainty *eₚ* and uncertainty *eₘ.* Because uncertainty *e_{f}* is less than uncertainty *eₚ* and uncertainty *eₘ*, filtered value *f* is more accurate than predicted value *p* or measured value *m* alone. Thus, CO_{filtered} module 44 is able to output CO_{filtered}, which can be more accurate than either of the computed CO_{AE} or CO_{LR} alone for current time step *n*.

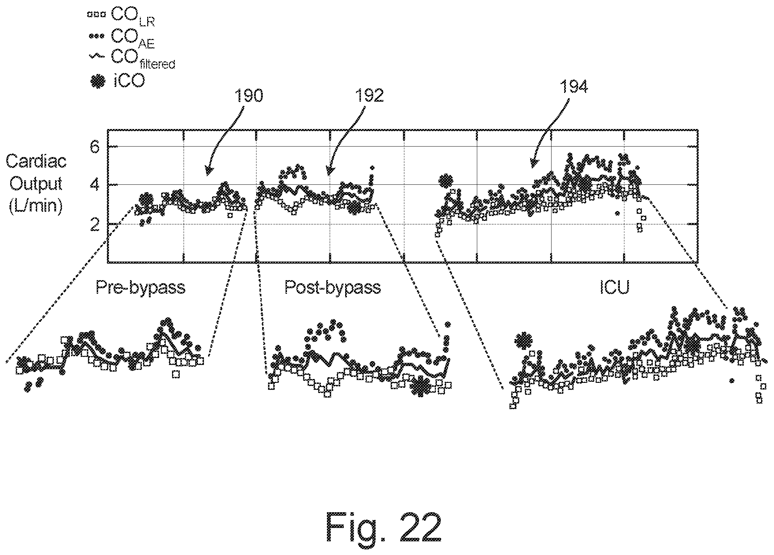

FIG. 22 is a graph comparing example cardiac output values from CO_{AE} module 40, CO_{LR} module 42, and CO_{filtered} module 44 and example iCO values over time for patient 16 in a pre-bypass state, a post-bypass state, and an ICU state. CO_{AE} module 40, CO_{LR} module 42, CO_{filtered} module 44, and iCO are each different sources of cardiac output within hemodynamic monitoring system 10, as shown in FIG. 1. In FIG. 22, CO_{LR} values from CO_{LR} module 42 (illustrated as open squares), CO_{AE} values from CO_{AE} module 40 (illustrated as solid circles), CO_{filtered} values from CO_{filtered} module 44 (illustrated as solid lines), and iCO values (illustrated as solid asterisks) are shown over time.

As illustrated in FIG. 22, the graph of the example cardiac output values is separated into three portions, including first portion 190, second portion 192, and third portion 194. First, second, and third portions 190, 192, and 194 are not connected to each other because they are separated by some arbitrary amount of time, which is represented by the space along the *x*-axis between first portion 190 and second portion 192 and between second portion 192 and third portion 194. First portion 190 corresponds to a pre-bypass state of patient 16. First portion 190 includes example cardiac output values measured or obtained for patient 16 prior to a bypass procedure. Second portion 192 corresponds to a post-bypass state of patient 16. Second portion 192 includes example cardiac output values measured or obtained for patient 16 after a bypass procedure. Third portion 194 corresponds to an ICU state of patient 16. Third portion 194 includes example cardiac output values measured or obtained for patient 16 during a period when patient 16 was admitted to an ICU.

FIG. 22 shows that CO_{filtered} values from CO_{filtered} module 44 are more continuous and less noisy than CO_{LR} values from CO_{LR} module 42, CO_{AE} values from CO_{AE} module 40, and iCO values. That is, the values of CO_{LR}, CO_{AE}, and iCO can be synchronous, asynchronous, trend in the same direction, and/or trend in different directions at each time point.

For example, in one region slightly before midway along second portion 192 (as viewed from left to right), the values for CO_{LR} and CO_{AE} diverge or trend in opposite directions. Over the rest of second portion 192 (and most of first portion 190 and third portion 194 as well), CO_{LR} and CO_{AE} generally tend to track in the same direction, though this is not necessarily the case. As shown in FIG. 22, the values of CO_{filtered} fall between the diverging values of CO_{LR} and CO_{AE} in the region near midway along second portion 192. This is because CO_{filtered} module 44 uses a Kalman filter algorithm to intelligently weight and merge the estimates of cardiac output into CO_{filtered}. Accordingly, CO_{filtered} values are less noisy than the other cardiac output values shown in FIG. 22.

As another example, there are several time points where values of CO_{LR}, CO_{AE}, and iCO are not received. Time points on the graph in FIG. 22 where one or more of CO_{LR}, CO_{AE}, and iCO are missing represent asynchronous cardiac output estimation activity from the various sources of cardiac output in hemodynamic monitoring system 10. However, CO_{filtered} is still available from CO_{filtered} module 44 at these time points. CO_{filtered} module 44 is generally always able to produce CO_{filtered} values, so the graph of CO_{filtered} is more continuous than for the other cardiac output sources shown in FIG. 22.

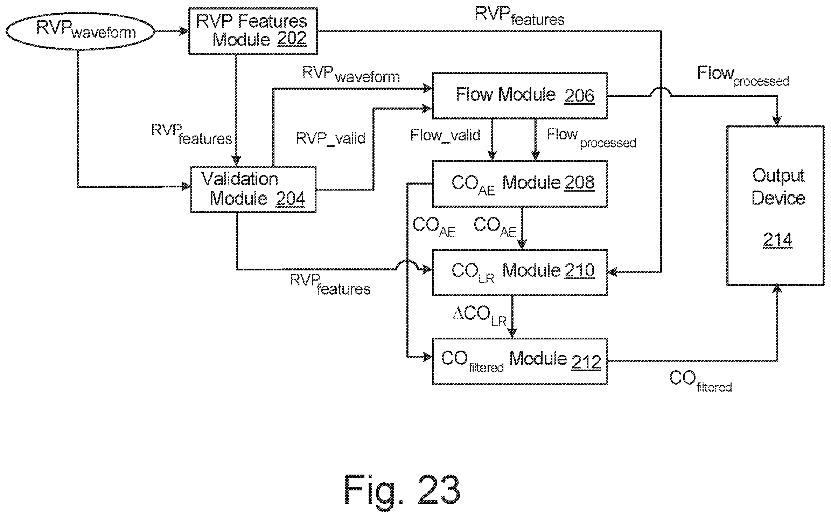

### Example Implementation and Alternatives (FIG. 23)

Many different configurations of hemodynamic monitoring system 10 are possible using combinations of the components and modules described above with reference to FIGS. 1-22. That is, hemodynamic monitoring system 10, according to the techniques described herein, can be implemented in many different ways, including using different combinations of hemodynamic sensors 14 to obtain hemodynamic data about patient 16 (as shown in FIG. 1). Accordingly, some of the inputs and outputs shown in FIG. 6 are optional. Depending on the hemodynamic data that is available from patient 16, or depending on the desired output, different combinations of RVP features module 32, PAP features module 34, validation module 36, flow module 38, CO_{AE} module 40, CO_{LR} module 42, and CO_{filtered} module 44, as shown in FIG. 1, can be implemented together to estimate blood flow, cardiac output, and/or change in cardiac output for patient 16.

For example, one or more of PAP_{waveform}, CCO, and iCO may not be available from patient 16 or may be ignored for other reasons. In an example where PAP_{waveform} is not available or not used, PAP features module 34 is also not used and PAP_{features} are not passed to validation module 36 or CO_{LR} module 42. Instead, only RVP features module 32 is used and validation module 36 and CO_{LR} module 42 only receive RVP_{features}. In an example where CCO and iCO are not available or not used, CCO and iCO measurements are not passed to CO_{LR} module 42 or CO_{filtered} module 44. Instead, CO_{LR} module 42 uses only CO_{AE} from CO_{AE} module 40, and CO_{filtered} module 44 uses some combination of CO_{AE}, CO_{LR}, and ΔCO_{LR}.

Moreover, an estimate of cardiac output for patient 16 can be output from any one or more of CO_{AE} module 40, CO_{LR} module 42, and CO_{filtered} module 44. Likewise, an estimated flow_{processed} can be output from flow module 38, in addition or alternatively to being passed to CO_{AE} module 40 to estimate CO_{AE}. Similarly, an estimated change in cardiac output can be output from CO_{LR} module 42, in addition or alternatively to being passed to CO_{filtered} module 44 to estimate ΔCO_{filtered}.

FIG. 23 is a schematic block diagram illustrating inputs and outputs for each module of an example implementation of hemodynamic monitoring system 10. FIG. 23 shows RVP features module 202, validation module 204, flow module 206, CO_{AE} module 208, CO_{LR} module 210, CO_{filtered} module 212, and output device 214. Each of RVP features module 202, validation module 204, flow module 206, CO_{AE} module 208, CO_{LR} module 210, CO_{filtered} module 212, and output device 214 is generally similar to the component or module with the same name as described above with reference to FIGS. 1-22, and one possible configuration of these components and modules is described here.

As shown in FIG. 23, RVP features module 202 receives RVP_{waveform} as an input. RVP features module 202 extracts RVP_{features} from RVP_{waveform}. RVP features module 202 outputs RVP_{features} to validation module 204. RVP features module 202 also outputs RVP_{features} to COLR module 210.

Validation module 204 receives RVP_{waveform} and RVP_{features} as inputs. RVP_{features} are fed to validation module 204 from RVP features module 202. Validation module 204 outputs instructions indicating if RVP_valid is true or false (i.e., whether RVP_{waveform} is valid or not valid) to flow module 206. An instruction that RVP_valid is true (e.g., "RVP_valid=true") means that hemodynamic data corresponding to RVP_{waveform} is valid for further processing by flow module 206. An instruction that RVP_valid is false (e.g., "RVP_valid=false") means that hemodynamic data corresponding to RVP_{waveform} is not valid for further processing. Validation module 204 also outputs a smoothed RVP_{waveform} to flow module 206.

Flow module 206 receives the smoothed RVP_{waveform} from validation module 204 as an input. Flow module 206 outputs instructions indicating whether flow_valid is true or false (i.e., whether flow_{processed} is valid or not valid) to CO_{AE} module 208. An instruction that flow_valid is true (e.g., "flow_valid=true") means that the estimated blood flow waveform, flow_{processed}, is valid for further processing by CO_{AE} module 208. An instruction that flow_valid is false (e.g., "flow_valid=false") means that the estimated blood flow waveform, flow_{processed}, is not valid for further processing. Flow module 206 further outputs flow_{processed} to CO_{AE} module 208. Flow module 206 also outputs flow_{processed} to output device 214.

CO_{AE} module 208 receives flow_{processed} as an input. Flow_{processed} is fed to CO_{AE} module 208 from flow module 206. CO_{AE} module 208 also receives an instruction from flow module 206 indicating whether flow _valid is true or false such that CO_{AE} module 208 can proceed or not proceed accordingly. CO_{AE} module 208 outputs CO_{AE} to CO_{LR} module 210 and CO_{filtered} module 212.

COLR module 210 receives RVP_{features} and COAE as inputs. RVP_{features} are fed to CO_{LR} module 210 from RVP features module 202. CO_{AE} is fed to CO_{LR} module 210 from CO_{AE} module 208. CO_{LR} module 210 outputs CO_{LR} and ΔCO_{LR} to CO_{filtered} module 212.

CO_{filtered} module 212 receives CO_{AE}, CO_{LR}, and ΔCO_{LR} as inputs. CO_{AE} is fed to CO_{filtered} module 212 from CO_{AE} module 208. CO_{LR} and ΔCO_{LR} are fed to CO_{filtered} module 212 from CO_{LR} module 210. CO_{filtered} module 212 outputs CO_{filtered} to output device 214.

As shown in FIG. 23, output device 214 receives outputs from flow module 206 and CO_{filtered} module 212. More specifically, output device 214 receives flow_{processed} and CO_{filtered}. Each of these outputs can be displayed (e.g., via display 24, as shown in FIG. 1) as corresponding graphs representing the values over time.

Any of the various systems, devices, apparatuses, etc. in this disclosure can be sterilized (e.g., with heat, radiation, ethylene oxide, hydrogen peroxide, etc.) to ensure they are safe for use with patients, and the methods herein can comprise sterilization of the associated system, device, apparatus, etc. (e.g., with heat, radiation, ethylene oxide, hydrogen peroxide, etc.).

The treatment techniques, methods, steps, etc. described or suggested herein or in references incorporated herein can be performed on a living animal or on a non-living simulation, such as on a cadaver, cadaver heart, anthropomorphic ghost, simulator (e.g., with the body parts, tissue, etc. being simulated), etc.

### Discussion of Possible Examples

The following are non-exclusive descriptions of possible examples of the present disclosure.

A system for determining a hemodynamic condition of a patient includes a first hemodynamic sensor and a display. The first hemodynamic sensor produces, on an ongoing basis, a first hemodynamic sensor signal representative of a right ventricular pressure waveform of the patient. The system further includes one or more processors and computer-readable memory encoded with instructions that, when executed by the one or more processors, cause the system to receive the first hemodynamic sensor signal representative of the right ventricular pressure waveform of the patient. The instructions further cause the system to estimate a filtered cardiac output by filtering one or more cardiac output estimates using a Kalman filter algorithm and output the filtered cardiac output to the display. At least one of the one or more cardiac output estimates is determined using the right ventricular pressure waveform of the patient or features extracted from the right ventricular pressure waveform of the patient.

The system of the preceding paragraph can optionally include, additionally and/or alternatively, any one or more of the following features, configurations, and/or additional components:

The Kalman filter algorithm can be configured such that each iteration of the Kalman filter algorithm can include a prediction phase and one or more update phases, and the prediction phase can alternate with the one or more update phases.

The prediction phase can predict a predicted estimate of cardiac output that corresponds to a current time step, each of the one or more update phases can consume a measured input that corresponds to the current time step, such that one or more measured inputs are consumed, and the predicted estimate of cardiac output can be updated using a weighted average of the predicted estimate of cardiac output and each of the one or more measured inputs.

The one or more measured inputs can include one or more of an autoencoder cardiac output estimated by an autoencoder model, a linear regression cardiac output estimated using a regression model, a continuous cardiac output obtained via a catheter-based thermal filament, and an intermittent cardiac output obtained via a catheter-based thermistor after administration of a fluid bolus.

The prediction phase can predict the predicted estimate of cardiac output based on one or more previous filtered estimates of cardiac output corresponding to previous time steps of the Kalman filter algorithm.

The prediction phase can further predict the predicted estimate of cardiac output by extrapolation using at least two of the one or more previous filtered estimates of cardiac output.

The prediction phase can further predict the predicted estimate of cardiac output by extrapolation using three or more of the one or more previous filtered estimates of cardiac output.

The prediction phase can further predict the predicted estimate of cardiac output by adding a magnitude of an estimated change in cardiac output to a most recent one of the one or more previous filtered estimates of cardiac output.

The predicted estimate of cardiac output can include a corresponding prediction uncertainty, each of the one or more measured inputs can include a corresponding measurement uncertainty, and the corresponding prediction uncertainty and the corresponding measurement uncertainties can be predetermined.

The predicted estimate of cardiac output and each of the one or more measured inputs can be weighted by the corresponding prediction uncertainty and the corresponding measurement uncertainties, respectively, such that greater uncertainty is given less weight and lower uncertainty is given more weight.

The corresponding prediction uncertainty and the corresponding measurement uncertainties can be fixed over time.

The corresponding prediction uncertainty and the corresponding measurement uncertainties can be modulated over time.

The corresponding measurement uncertainties can be scaled based on a signal quality index of the right ventricular pressure waveform of the patient.

The corresponding measurement uncertainties can be unchanged when the signal quality index of the right ventricular pressure waveform of the patient is a first value within a designated range, and the corresponding measurement uncertainties can be multiplied by a factor to increase the corresponding measurement uncertainties when the signal quality index of the right ventricular pressure waveform of the patient is a second value within the designated range.

A corresponding uncertainty of the filtered cardiac output can be less than the corresponding prediction uncertainty and the corresponding measurement uncertainties.

The Kalman filter algorithm can be configured such that a number of update phases per iteration of the Kalman filter algorithm depends on which of the one or more measured inputs are available for a corresponding iteration, and the Kalman filter algorithm can be configured to tolerate asynchronous receipt of the one or more measured inputs and/or additional sources of measured inputs.

The system of can further include a second hemodynamic sensor that produces, on an ongoing basis, a second hemodynamic sensor signal representative of a pulmonary artery pressure waveform of the patient; the instructions, when executed by the one or more processors, can further cause the system to receive the second hemodynamic sensor signal representative of the pulmonary artery pressure waveform of the patient, and at least one of the one or more cardiac output estimates can be determined using the pulmonary artery pressure waveform of the patient or features extracted from the pulmonary artery pressure waveform of the patient.

The Kalman filter algorithm can be recursive and use one or more previous filtered estimates of cardiac output corresponding to previous time steps of the Kalman filter algorithm and one or more measured inputs that correspond to a current time step to produce the filtered cardiac output for the current time step.

The display can display a graph of filtered cardiac output values over time.

The filtered cardiac output estimated using the Kalman filter algorithm can be a continuous estimate of cardiac output for the patient.

The first hemodynamic sensor can be connected to a hemodynamic monitor that includes the display, the one or more processors, and the computer-readable memory.

The system can continuously output the filtered cardiac output to the display for monitoring the hemodynamic condition of the patient.

A system for determining a hemodynamic condition of a patient includes a first hemodynamic sensor and a display. The first hemodynamic sensor produces, on an ongoing basis, a first hemodynamic sensor signal representative of a right ventricular pressure waveform of the patient. The system further includes one or more processors and computer-readable memory encoded with instructions that, when executed by the one or more processors, cause the system to receive the first hemodynamic sensor signal representative of the right ventricular pressure waveform of the patient. The instructions further cause the system to estimate a filtered change in cardiac output by filtering one or more change in cardiac output estimates using a Kalman filter algorithm and output the filtered change in cardiac output to the display. At least one of the one or more change in cardiac output estimates is determined using the right ventricular pressure waveform of the patient or features extracted from the right ventricular pressure waveform of the patient.

The system of the preceding paragraph can optionally include, additionally and/or alternatively, any one or more of the following features, configurations, and/or additional components:
The Kalman filter algorithm can be configured such that each iteration of the Kalman filter algorithm can include a prediction phase and one or more update phases, and the prediction phase can alternate with the one or more update phases.

The prediction phase can predict a predicted estimate of change in cardiac output that corresponds to a current time step, each of the one or more update phases can consume a measured input that corresponds to the current time step, such that one or more measured inputs are consumed, and the predicted estimate of change in cardiac output can be updated using a weighted average of the predicted estimate of change in cardiac output and each of the one or more measured inputs.

The one or more measured inputs can include one or more of an autoencoder change in cardiac output estimated using an autoencoder model and a linear regression change in cardiac output estimated by a regression model.

The prediction phase can predict the predicted estimate of change in cardiac output based on one or more previous filtered estimates of change in cardiac output corresponding to previous time steps of the Kalman filter algorithm.

The prediction phase can further predict the predicted estimate of change in cardiac output by extrapolation using at least two of the one or more previous filtered estimates of change in cardiac output.

The prediction phase can further predict the predicted estimate of change in cardiac output by extrapolation using three or more of the one or more previous filtered estimates of change in cardiac output.

The predicted estimate of change in cardiac output can include a corresponding prediction uncertainty, each of the one or more measured inputs can include a corresponding measurement uncertainty, and the corresponding prediction uncertainty and the corresponding measurement uncertainties can be predetermined.

The predicted estimate of change in cardiac output and each of the one or more measured inputs can be weighted by the corresponding prediction uncertainty and the corresponding measurement uncertainties, respectively, such that greater uncertainty is given less weight and lower uncertainty is given more weight.

The corresponding prediction uncertainty and the corresponding measurement uncertainties can be fixed over time.

The corresponding prediction uncertainty and the corresponding measurement uncertainties can be modulated over time.

The corresponding measurement uncertainties can be scaled based on a signal quality index of the right ventricular pressure waveform of the patient.

The corresponding measurement uncertainties can be unchanged when the signal quality index of the right ventricular pressure waveform of the patient is a first value within a designated range, and the corresponding measurement uncertainties can be multiplied by a factor to increase the corresponding measurement uncertainties when the signal quality index of the right ventricular pressure waveform of the patient is a second value within the designated range.

A corresponding uncertainty of the filtered change in cardiac output can be less than the corresponding prediction uncertainty and the corresponding measurement uncertainties.

The Kalman filter algorithm can be configured such that a number of update phases per iteration of the Kalman filter algorithm depends on which, if any, of the one or more measured inputs are available for a corresponding iteration, and the Kalman filter algorithm can be configured to tolerate asynchronous receipt of the one or more measured inputs and/or additional sources of measured inputs.

The system can further include a second hemodynamic sensor that produces, on an ongoing basis, a second hemodynamic sensor signal representative of a pulmonary artery pressure waveform of the patient; the instructions, when executed by the one or more processors, can further cause the system to receive the second hemodynamic sensor signal representative of the pulmonary artery pressure waveform of the patient, and at least one of the one or more change in cardiac output estimates can be determined using the pulmonary artery pressure waveform of the patient or features extracted from the pulmonary artery pressure waveform of the patient.

The Kalman filter algorithm can be recursive and use one or more previous filtered estimates of change in cardiac output corresponding to previous time steps of the Kalman filter algorithm and one or more measured inputs that correspond to a current time step to produce the filtered change in cardiac output for the current time step.

The display can display a graph of filtered change in cardiac output values over time.

The filtered change in cardiac output estimated using the Kalman filter algorithm can be a continuous estimate of change in cardiac output for the patient.

The first hemodynamic sensor can be connected to a hemodynamic monitor that includes the display, the one or more processors, and the computer-readable memory.

The system can continuously output the filtered change in cardiac output to the display for monitoring the hemodynamic condition of the patient.

A system for determining a hemodynamic condition of a patient includes a first hemodynamic sensor and a display. The first hemodynamic sensor produces, on an ongoing basis, a first hemodynamic sensor signal representative of a right ventricular pressure waveform of the patient. The system further includes one or more processors and computer-readable memory encoded with instructions that, when executed by the one or more processors, cause the system to receive the first hemodynamic sensor signal representative of the right ventricular pressure waveform of the patient. The instructions further cause the system to estimate a filtered cardiac output by filtering one or more cardiac output estimates using a first Kalman filter algorithm, estimate a filtered change in cardiac output by filtering one or more change in cardiac output estimates using a second Kalman filter algorithm, and output the filtered cardiac output and the filtered change in cardiac output to the display. At least one of the one or more cardiac output estimates is determined using the right ventricular pressure waveform of the patient or features extracted from the right ventricular pressure waveform of the patient. At least one of the one or more change in cardiac output estimates is determined using the right ventricular pressure waveform of the patient or the features extracted from the right ventricular pressure waveform of the patient.

A method of determining a hemodynamic condition of a patient includes receiving, by a hemodynamic monitoring system, sensed hemodynamic data representative of a right ventricular pressure waveform of the patient. The method further includes estimating, by the hemodynamic monitoring system, a filtered cardiac output by filtering one or more cardiac output estimates using a first Kalman filter algorithm; estimating, by the hemodynamic monitoring system, a filtered change in cardiac output by filtering one or more change in cardiac output estimates using a second Kalman filter algorithm; and outputting, by the hemodynamic monitoring system, the filtered cardiac output and the filtered change in cardiac output to a display. At least one of the one or more cardiac output estimates is determined using the right ventricular pressure waveform of the patient or features extracted from the right ventricular pressure waveform of the patient. At least one of the one or more change in cardiac output estimates is determined using the right ventricular pressure waveform of the patient or the features extracted from the right ventricular pressure waveform of the patient.

While the dislosure has been described with reference to an exemplary example(s), it will be understood by those skilled in the art that various changes may be made and equivalents may be substituted for elements thereof without departing from the scope of the disclosure.

In addition, many modifications may be made to adapt a particular situation or material to the teachings of the invention without departing from the essential scope thereof. Therefore, it is intended that the invention not be limited to the particular example(s) disclosed, but that the invention will include all examples falling within the scope of the appended claims.

## Claims

1. A system for determining a hemodynamic condition of a patient, the system comprising:
a first hemodynamic sensor that produces, on an ongoing basis, a first hemodynamic sensor signal representative of a right ventricular pressure waveform of the patient;
a display;
one or more processors; and
computer-readable memory encoded with instructions that, when executed by the one or more processors, cause the system to:
receive the first hemodynamic sensor signal representative of the right ventricular pressure waveform of the patient;
estimate a filtered cardiac output by filtering one or more cardiac output estimates using a filter algorithm, wherein at least one of the one or more cardiac output estimates is determined using the right ventricular pressure waveform of the patient or features extracted from the right ventricular pressure waveform of the patient; and
output the filtered cardiac output to the display,
**characterised in that** the filter algorithm is a Kalman filter algorithm.

2. The system of claim 1, wherein the Kalman filter algorithm is configured such that each iteration of the Kalman filter algorithm can include a prediction phase and one or more update phases;
wherein the prediction phase alternates with the one or more update phases;
wherein the prediction phase predicts a predicted estimate of cardiac output that corresponds to a current time step;
wherein each of the one or more update phases consumes a measured input that corresponds to the current time step, such that one or more measured inputs are consumed; and
wherein the predicted estimate of cardiac output is updated using a weighted average of the predicted estimate of cardiac output and each of the one or more measured inputs.

3. The system of claim 2, wherein the one or more measured inputs include one or more of:
an autoencoder cardiac output estimated by an autoencoder model;
a linear regression cardiac output estimated using a regression model;
a continuous cardiac output obtained via a catheter-based thermal filament; and
an intermittent cardiac output obtained via a catheter-based thermistor after administration of a fluid bolus.

4. The system of claim 2 or claim 3, wherein the prediction phase predicts the predicted estimate of cardiac output based on one or more previous filtered estimates of cardiac output corresponding to previous time steps of the Kalman filter algorithm,
optionally wherein the prediction phase further predicts:
a) the predicted estimate of cardiac output by extrapolation using at least two of the one or more previous filtered estimates of cardiac output;
b) the predicted estimate of cardiac output by extrapolation using three or more of the one or more previous filtered estimates of cardiac output; or
c) the predicted estimate of cardiac output by adding a magnitude of an estimated change in cardiac output to a most recent one of the one or more previous filtered estimates of cardiac output.

5. The system of claim 2,
wherein the predicted estimate of cardiac output includes a corresponding prediction uncertainty;
wherein each of the one or more measured inputs includes a corresponding measurement uncertainty; and
wherein the corresponding prediction uncertainty and the corresponding measurement uncertainties are predetermined.

6. The system of claim 5, wherein:
a) the predicted estimate of cardiac output and each of the one or more measured inputs are weighted by the corresponding prediction uncertainty and the corresponding measurement uncertainties, respectively, such that greater uncertainty is given less weight and lower uncertainty is given more weight; or
b) the corresponding prediction uncertainty and the corresponding measurement uncertainties are fixed over time or modulated over time.

7. The system of claim 6, wherein the corresponding fixed or modulated measurement
uncertainties are scaled based on a signal quality index of the right ventricular pressure waveform of the patient, optionally wherein the corresponding fixed or modulated measurement uncertainties are unchanged when the signal quality index of the right ventricular pressure waveform of the patient is a first value within a designated range, and the corresponding measurement uncertainties are multiplied by a factor to increase the corresponding measurement uncertainties when the signal quality index of the right ventricular pressure waveform of the patient is a second value within the designated range.

8. The system of claim 5, wherein a corresponding uncertainty of the filtered cardiac output is less than the corresponding prediction uncertainty and the corresponding measurement uncertainties.

9. The system of any preceding claim and further comprising:
a second hemodynamic sensor that produces, on an ongoing basis, a second hemodynamic sensor signal representative of a pulmonary artery pressure waveform of the patient;
wherein the instructions, when executed by the one or more processors, further cause the system to receive the second hemodynamic sensor signal representative of the pulmonary artery pressure waveform of the patient; and
wherein at least one of the one or more cardiac output estimates is determined using the pulmonary artery pressure waveform of the patient or features extracted from the pulmonary artery pressure waveform of the patient.

10. The system of any preceding claim, wherein the display displays a graph of filtered cardiac output values over time.

11. The system of any preceding claim, wherein the filtered cardiac output estimated using the Kalman filter algorithm is a continuous estimate of cardiac output for the patient.

12. The system of any preceding claim, wherein the first hemodynamic sensor is connected to a hemodynamic monitor that includes the display, the one or more processors, and the computer-readable memory.

13. The system of any preceding claim, wherein the system continuously outputs the filtered cardiac output to the display for monitoring the hemodynamic condition of the patient.

14. The system of any preceding claim, wherein the Kalman filter algorithm is a first Kalman filter algorithm and wherein the computer-readable memory is further encoded with instructions that, when executed by the one or more processors, cause the system to:
estimate a filtered change in cardiac output by filtering one or more change in cardiac output estimates using a second Kalman filter algorithm, wherein at least one of the one or more change in cardiac output estimates is determined using the right ventricular pressure waveform of the patient or the features extracted from the right ventricular pressure waveform of the patient; and
output the filtered change in cardiac output to the display.

15. A method of determining a hemodynamic condition of a patient, the method comprising:
receiving, by a hemodynamic monitoring system, sensed hemodynamic data representative of a right ventricular pressure waveform of the patient;
estimating, by the hemodynamic monitoring system, a filtered cardiac output by filtering one or more cardiac output estimates using a first Kalman filter algorithm, wherein at least one of the one or more cardiac output estimates is determined using the right ventricular pressure waveform of the patient or features extracted from the right ventricular pressure waveform of the patient;
estimating, by the hemodynamic monitoring system, a filtered change in cardiac output by filtering one or more change in cardiac output estimates using a second Kalman filter algorithm, wherein at least one of the one or more change in cardiac output estimates is determined using the right ventricular pressure waveform of the patient or the features extracted from the right ventricular pressure waveform of the patient; and
outputting, by the hemodynamic monitoring system, the filtered cardiac output and the filtered change in cardiac output to a display.

## Patentansprüche

1. System zum Bestimmen eines hämodynamischen Zustands eines Patienten, wobei das System Folgendes umfasst:
einen ersten hämodynamischen Sensor, der fortlaufend ein erstes hämodynamisches Sensorsignal, das repräsentativ für eine rechtsventrikuläre Druckkurve des Patienten ist, erzeugt;
eine Anzeige;
einen oder mehrere Prozessoren; und
einen computerlesbaren Speicher, der mit Anweisungen codiert ist, die, wenn sie durch den einen oder die mehreren Prozessoren ausgeführt werden, das System zu Folgendem veranlassen:
Empfangen des ersten hämodynamischen Sensorsignals, das repräsentativ für die rechtsventrikuläre Druckkurve des Patienten ist;
Schätzen eines gefilterten Herzzeitvolumens durch Filtern eines oder mehrerer Herzzeitvolumenschätzwerte unter Verwendung eines Filteralgorithmus, wobei mindestens einer des einen oder der mehreren Herzzeitvolumenschätzwerte unter Verwendung der rechtsventrikulären Druckkurve des Patienten oder von aus der rechtsventrikulären Druckkurve des Patienten extrahierten Merkmalen bestimmt wird; und
Ausgeben des gefilterten Herzzeitvolumens an die Anzeige, **dadurch gekennzeichnet, dass** der Filteralgorithmus ein Kalman-Filteralgorithmus ist.

2. System nach Anspruch 1, wobei der Kalman-Filteralgorithmus so konfiguriert ist, dass jede Iteration des Kalman-Filteralgorithmus eine Vorhersagephase und eine oder mehrere Aktualisierungsphasen beinhalten kann;
wobei die Vorhersagephase mit der einen oder den mehreren Aktualisierungsphasen alterniert;
wobei die Vorhersagephase einen vorhergesagten Schätzwert des Herzzeitvolumens, der einem aktuellen Zeitschritt entspricht, vorhersagt;
wobei jede der einen oder der mehreren Aktualisierungsphasen eine gemessene Eingabe, die dem aktuellen Zeitschritt entspricht, verbraucht, so dass eine oder mehrere gemessene Eingaben verbraucht werden; und
wobei der vorhergesagte Schätzwert des Herzzeitvolumens unter Verwendung eines gewichteten Mittelwerts des vorhergesagten Schätzwerts des Herzzeitvolumens und jeder der einen oder der mehreren gemessenen Eingaben aktualisiert wird.

3. System nach Anspruch 2, wobei die eine oder die mehreren gemessenen Eingaben eines oder mehrere von Folgendem beinhalten:
einem durch ein Autoencoder-Modell geschätzten Autoencoder-Herzzeitvolumen;
einem unter Verwendung eines Regressionsmodells geschätzten Herzzeitvolumen einer linearen Regression;
einem über ein katheterbasiertes Thermofilament erhaltenen kontinuierlichen Herzzeitvolumen; und
einem über einen katheterbasierten Thermistor nach einer Verabreichung eines Flüssigkeitsbolus erhaltenen intermittierenden Herzzeitvolumen.

4. System nach Anspruch 2 oder Anspruch 3, wobei die Vorhersagephase den vorhergesagten Schätzwert des Herzzeitvolumens basierend auf einem oder mehreren vorherigen gefilterten Schätzwerten des Herzzeitvolumens, die vorherigen Zeitschritten des Kalman-Filteralgorithmus entsprechen, vorhersagt,
wobei die Vorhersagephase optional ferner Folgendes vorhersagt:
a) den vorhergesagten Schätzwert des Herzzeitvolumens durch Extrapolation unter Verwendung von mindestens zwei des einen oder der mehreren vorherigen gefilterten Schätzwerte des Herzzeitvolumens;
b) den vorhergesagten Schätzwert des Herzzeitvolumens durch Extrapolation unter Verwendung von drei oder mehr des einen oder der mehreren vorherigen gefilterten Schätzwerte des Herzzeitvolumens; oder
c) den vorhergesagten Schätzwert des Herzzeitvolumens durch Addieren eines Betrags einer geschätzten Änderung des Herzzeitvolumens zu einem jüngsten des einen oder der mehreren vorherigen gefilterten Schätzwerte des Herzzeitvolumens.

5. System nach Anspruch 2, wobei der vorhergesagte Schätzwert des Herzzeitvolumens eine entsprechende Vorhersageunsicherheit beinhaltet;
wobei jede der einen oder der mehreren gemessenen Eingaben eine entsprechende Messunsicherheit beinhaltet; und
wobei die entsprechende Vorhersageunsicherheit und die entsprechenden Messunsicherheiten vorgegeben sind.

6. System nach Anspruch 5, wobei:
a) der vorhergesagte Schätzwert des Herzzeitvolumens und jede der einen oder der mehreren gemessenen Eingaben durch die entsprechende Vorhersageunsicherheit bzw. die entsprechenden Messunsicherheiten gewichtet werden, so dass eine größere Unsicherheit geringer gewichtet und eine geringere Unsicherheit stärker gewichtet wird; oder
b) die entsprechende Vorhersageunsicherheit und die entsprechenden Messunsicherheiten über die Zeit fest oder über die Zeit moduliert sind.

7. System nach Anspruch 6, wobei die entsprechenden festen oder modulierten Messunsicherheiten basierend auf einem Signalqualitätsindex der rechtsventrikulären Druckkurve des Patienten skaliert sind,
wobei optional die entsprechenden festen oder modulierten Messunsicherheiten unverändert sind, wenn der Signalqualitätsindex der rechtsventrikulären Druckkurve des Patienten ein erster Wert innerhalb eines festgelegten Bereichs ist, und die entsprechenden Messunsicherheiten mit einem Faktor multipliziert werden, um die entsprechenden Messunsicherheiten zu erhöhen, wenn der Signalqualitätsindex der rechtsventrikulären Druckkurve des Patienten ein zweiter Wert innerhalb des festgelegten Bereichs ist.

8. System nach Anspruch 5, wobei eine entsprechende Unsicherheit des gefilterten Herzzeitvolumens kleiner als die entsprechende Vorhersageunsicherheit und die entsprechenden Messunsicherheiten ist.

9. System nach einem der vorhergehenden Ansprüche, das ferner Folgendes umfasst:
einen zweiten hämodynamischen Sensor, der fortlaufend ein zweites hämodynamisches Sensorsignal, das repräsentativ für eine pulmonalarterielle Druckkurve des Patienten ist, erzeugt;
wobei die Anweisungen, wenn sie durch den einen oder die mehreren Prozessoren ausgeführt werden, ferner das System dazu veranlassen, das zweite hämodynamische Sensorsignal, das repräsentativ für die pulmonalarterielle Druckkurve des Patienten ist, zu empfangen; und
wobei mindestens einer des einen oder der mehreren Herzzeitvolumenschätzwerte unter Verwendung der pulmonalarteriellen Druckkurve des Patienten oder von aus der pulmonalarteriellen Druckkurve des Patienten extrahierten Merkmalen bestimmt wird.

10. System nach einem der vorhergehenden Ansprüche, wobei die Anzeige ein Diagramm gefilterter Herzzeitvolumenwerte über die Zeit anzeigt.

11. System nach einem der vorhergehenden Ansprüche, wobei das gefilterte Herzzeitvolumen, das unter Verwendung des Kalman-Filteralgorithmus geschätzt wird, eine fortlaufende Schätzung des Herzzeitvolumens für den Patienten ist.

12. System nach einem der vorhergehenden Ansprüche, wobei der erste hämodynamische Sensor mit einem hämodynamischen Monitor, der die Anzeige, den einen oder die mehreren Prozessoren und den computerlesbaren Speicher aufweist, verbunden ist.

13. System nach einem der vorhergehenden Ansprüche, wobei das System das gefilterte Herzzeitvolumen fortlaufend an die Anzeige zum Überwachen des hämodynamischen Zustands des Patienten ausgibt.

14. System nach einem der vorhergehenden Ansprüche, wobei der Kalman-Filteralgorithmus ein erster Kalman-Filteralgorithmus ist und wobei der computerlesbare Speicher ferner mit Anweisungen codiert ist, die, wenn sie durch den einen oder die mehreren Prozessoren ausgeführt werden, das System zu Folgendem veranlassen:
Schätzen einer gefilterten Änderung des Herzzeitvolumens durch Filtern einer oder mehrerer Änderungen der Herzzeitvolumenschätzwerte unter Verwendung eines zweiten Kalman-Filteralgorithmus, wobei mindestens eine der einen oder der mehreren Änderungen der Herzzeitvolumenschätzwerte unter Verwendung der rechtsventrikulären Druckkurve des Patienten oder der aus der rechtsventrikulären Druckkurve des Patienten extrahierten Merkmale bestimmt wird; und
Ausgeben der gefilterten Änderung des Herzzeitvolumens an die Anzeige.

15. Verfahren zum Bestimmen eines hämodynamischen Zustands eines Patienten, wobei das Verfahren Folgendes umfasst:
Empfangen, durch ein hämodynamisches Überwachungssystem, erfasster hämodynamischer Daten, die repräsentativ für eine rechtsventrikuläre Druckkurve des Patienten sind;
Schätzen, durch das hämodynamische Überwachungssystem, eines gefilterten Herzzeitvolumens durch Filtern eines oder mehrerer Herzzeitvolumenschätzwerte unter Verwendung eines ersten Kalman-Filteralgorithmus, wobei mindestens einer des einen oder der mehreren Herzzeitvolumenschätzwerte unter Verwendung der rechtsventrikulären Druckkurve des Patienten oder von aus der rechtsventrikulären Druckkurve des Patienten extrahierten Merkmalen bestimmt wird;
Schätzen, durch das hämodynamische Überwachungssystem, einer gefilterten Änderung des Herzzeitvolumens durch Filtern einer oder mehrerer Änderungen der Herzzeitvolumenschätzwerte unter Verwendung eines zweiten Kalman-Filteralgorithmus, wobei mindestens eine der einen oder der mehreren Änderungen der Herzzeitvolumenschätzwerte unter Verwendung der rechtsventrikulären Druckkurve des Patienten oder der aus der rechtsventrikulären Druckkurve des Patienten extrahierten Merkmale bestimmt wird; und
Ausgeben, durch das hämodynamische Überwachungssystem, des gefilterten Herzzeitvolumens und der gefilterten Änderung des Herzzeitvolumens an eine Anzeige.

## Revendications

1. Système permettant de déterminer un état hémodynamique d'un patient, le système comprenant :
un premier capteur hémodynamique qui produit, en continu,
un premier signal de capteur hémodynamique représentatif d'une forme d'onde de pression ventriculaire droite du patient ;
un écran ;
un ou plusieurs processeurs ; et
une mémoire lisible par ordinateur codée avec des instructions qui, lorsqu'elles sont exécutées par les un ou plusieurs processeurs, amènent le système à :
recevoir le premier signal signal de capteur hémodynamique représentatif d'une forme d'onde de pression ventriculaire droite du patient ;
estimer un débit cardiaque filtré en filtrant une ou plusieurs estimations de débit cardiaque à l'aide d'un algorithme de filtrage, au moins l'une des une ou plusieurs estimations de débit cardiaque étant déterminée à l'aide de la forme d'onde de pression ventriculaire droite du patient ou de caractéristiques extraites de la forme d'onde de pression ventriculaire droite du patient ; et
envoyer le débit cardiaque filtré à l'écran, **caractérisé en ce que** l'algorithme de filtrage est un algorithme de filtrage de Kalman.

2. Système selon la revendication 1, dans lequel l'algorithme de filtre de Kalman est configuré de telle sorte que chaque itération de l'algorithme de filtre de Kalman puisse inclure une phase de prédiction et une ou plusieurs phases de mise à jour ;
la phase de prédiction alternant avec les une ou plusieurs phases de mise à jour ;
la phase de prédiction prédisant une estimation prédite du débit cardiaque qui correspond à un pas de temps courant ;
chacune des une ou plusieurs phases de mise à jour consommant une entrée mesurée qui correspond au pas de temps courant, de sorte qu'une ou plusieurs entrées mesurées sont consommées ; et
l'estimation prédite du débit cardiaque étant mise à jour au moyen d'une moyenne pondérée de l'estimation prédite du débit cardiaque et de chacune des une ou plusieurs entrées mesurées.

3. Système de la revendication 2, dans lequel les une ou plusieurs entrées mesurées incluent un ou plusieurs des débits suivants :
un débit cardiaque d'auto-codeur estimé par un modèle d'auto-codeur ;
un débit cardiaque de régression linéaire estimé à l'aide d'un modèle de régression ;
un débit cardiaque continu obtenu à l'aide d'un filament thermique à base de cathéter ; et
un débit cardiaque intermittent obtenu à l'aide d'une thermistance à base de cathéter après administration d'un bolus de fluide.

4. Système selon la revendication 2 ou la revendication 3, dans lequel la phase de prédiction prédit l'estimation prédite du débit cardiaque sur la base d'une ou plusieurs estimations filtrées précédentes du débit cardiaque correspondant à des pas de temps précédents de l'algorithme de filtre de Kalman,
dans lequel, éventuellement, la phase de prédiction prédit en outre :
a) l'estimation prédite du débit cardiaque par extrapolation en utilisant au moins deux des une ou plusieurs estimations filtrées précédentes du débit cardiaque ;
b) l'estimation prédite du débit cardiaque par extrapolation en utilisant trois ou plus des estimations filtrées précédentes du débit cardiaque ; ou
c) l'estimation prévue du débit cardiaque par ajout d'une amplitude d'un changement estimé du débit cardiaque à la plus récente des une ou plusieurs estimations filtrées précédentes du débit cardiaque.

5. Système selon la revendication 2, dans lequel l'estimation prédite du débit cardiaque comprend une incertitude de prédiction correspondante ;
dans lequel chacune des une ou plusieurs entrées mesurées inclut une incertitude de mesure correspondante ; et dans lequel l'incertitude de prédiction correspondante et les incertitudes de mesures correspondantes sont prédéterminées.

6. Système selon la revendication 5, dans lequel :
a) l'estimation prédite du débit cardiaque et chacune des une ou plusieurs entrées mesurées sont pondérées, respectivement, par l'incertitude de prédiction correspondante et les incertitudes de mesure correspondantes, de telle sorte qu'une incertitude plus grande reçoivent un poids inférieur et une incertitude plus faible reçoive un poids plus élevé ; ou
b) l'incertitude de prédiction correspondante et les incertitudes de mesure correspondantes sont fixes dans le temps ou modulées dans le temps.

7. Système selon la revendication 6, dans lequel les incertitudes de mesure fixes ou modulées correspondantes sont mises à l'échelle sur la base d'un indice de qualité de signal de la forme d'onde de pression ventriculaire droite du patient,
où, éventuellement, les incertitudes de mesure fixes ou modulées correspondantes sont inchangées lorsque l'indice de qualité du signal de la forme d'onde de pression ventriculaire droite du patient est une première valeur dans une plage désignée, et les incertitudes de mesure correspondantes sont multipliées par un facteur pour augmenter les incertitudes de mesure correspondantes lorsque l'indice de qualité du signal de la forme d'onde de pression ventriculaire droite du patient est une seconde valeur dans la plage désignée.

8. Système selon la revendication 5, dans lequel une incertitude correspondante du débit cardiaque filtré est inférieure à l'incertitude de prédiction correspondante et aux incertitudes de mesure correspondantes.

9. Système selon l'une quelconque des revendications précédentes, comprenant en outre :
un second capteur hémodynamique produisant, en continu,
un second signal de capteur hémodynamique représentatif d'une forme d'onde de pression artérielle pulmonaire du patient ;
dans lequel les instructions, lorsqu'elles sont exécutées par les un ou plusieurs processeurs, amènent en outre le système à recevoir le second signal de capteur hémodynamique représentatif de la forme d'onde de pression artérielle pulmonaire du patient ; et
dans lequel au moins l'une des une ou plusieurs estimations de débit cardiaque est déterminée à l'aide de la forme d'onde de pression artérielle pulmonaire du patient ou de caractéristiques extraites de la forme d'onde de pression artérielle pulmonaire du patient.

10. Système selon l'une quelconque des revendications précédentes, dans lequel l'écran affiche un graphique de valeurs de débit cardiaque filtrées dans le temps.

11. Système selon l'une quelconque des revendications précédentes, dans lequel le débit cardiaque filtré estimé à l'aide de l'algorithme de filtre de Kalman est une estimation continue du débit cardiaque pour le patient.

12. Système selon l'une quelconque des revendications précédentes, dans lequel le premier capteur hémodynamique est connecté à un moniteur hémodynamique qui comprend l'écran, le ou les processeurs, et la mémoire lisible par ordinateur.

13. Système selon l'une quelconque des revendications précédentes, dans lequel le système délivre en continu le débit cardiaque filtré à l'écran pour permettre la surveillance de l'état hémodynamique du patient.

14. Système selon l'une quelconque des revendications précédentes, dans lequel l'algorithme de filtre de Kalman est un premier algorithme de filtre de Kalman et dans lequel la mémoire lisible par ordinateur est en outre codée avec des instructions qui, lorsqu'elles sont exécutées par les un ou plusieurs processeurs, amènent le système à :
estimer un changement filtré du débit cardiaque par filtrage d'un ou plusieurs changements des estimations du débit cardiaque à l'aide d'un second algorithme de filtre de Kalman, au moins l'un des un ou plusieurs changements des estimations du débit cardiaque étant déterminé à l'aide de la forme d'onde de pression ventriculaire droite du patient ou des caractéristiques extraites de la forme d'onde de pression ventriculaire droite du patient ; et
envoyer le changement filtré du débit cardiaque à l'écran.

15. Procédé de détermination d'un état hémodynamique d'un patient, le procédé comprenant :
la réception, par un système de surveillance hémodynamique, de données hémodynamiques détectées représentatives d'une forme d'onde de pression ventriculaire droite du patient ;
l'estimation, par le système de surveillance hémodynamique, d'un débit cardiaque filtré par filtrage d'une ou plusieurs estimations de débit cardiaque à l'aide d'un premier algorithme de filtre de Kalman, au moins l'une des une ou plusieurs estimations de débit cardiaque étant déterminée à l'aide de la forme d'onde de pression ventriculaire droite du patient ou de caractéristiques extraites de la forme d'onde de pression ventriculaire droite du patient ;
l'estimation, par le système de surveillance hémodynamique, d'un changement filtré du débit cardiaque par filtrage d'un ou plusieurs changements des estimations du débit cardiaque à l'aide d'un second algorithme de filtre de Kalman, au moins l'un des un ou
plusieurs changements des estimations du débit cardiaque étant déterminé à l'aide de la forme d'onde de pression ventriculaire droite du patient ou des caractéristiques extraites de la forme d'onde de pression ventriculaire droite du patient ; et
l'envoi, par le système de surveillance hémodynamique, du débit cardiaque filtré et du changement filtré du débit cardiaque à un écran.
